(19) **Europäisches Patentamt**
**European Patent Office**
**Office européen des brevets**

(11) **EP 4 772 613 A1**

(12) **EUROPEAN PATENT APPLICATION**
published in accordance with Art. 153(4) EPC

(43) Date of publication:
**08.07.2026  Bulletin 2026/28**

(21) Application number: **24860532.1**

(22) Date of filing: **29.08.2024**

(51) International Patent Classification (IPC):
*C12N 5/0783* (2010.01)    *C07K 14/55* (2006.01)
*C07K 14/54* (2006.01)    *C07K 14/715* (2006.01)
*A61K 35/17* (2025.01)    *A61K 39/00* (2006.01)
*A61P 35/00* (2006.01)

(52) Cooperative Patent Classification (CPC):
A61K 35/17; A61K 38/00; A61K 39/00;
A61P 31/00; A61P 35/00; A61P 37/06;
C07K 14/54; C07K 14/55; C07K 14/715; C12N 5/06

(86) International application number:
**PCT/KR2024/096098**

(87) International publication number:
**WO 2025/048616 (06.03.2025 Gazette 2025/10)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **31.08.2023  KR 20230115121
15.12.2023  KR 20230182873**

(71) Applicant: **GI Cell, Inc.
Seongnam-si, Gyeonggi-do 13201 (KR)**

(72) Inventors:
• **JANG, Myung Ho
Seoul 05849 (KR)**
• **HONG, Chun-Pyo
Seongnam-si Gyeonggi-do 13643 (KR)**
• **PARK, Jae Chan
Seoul 05853 (KR)**

• **KIM, Gil Jung
Sejong 30151 (KR)**
• **JANG, Hyehyeon
Seoul 01132 (KR)**
• **KIM, Kyong Hoon
Suwon-si Gyeonggi-do 16418 (KR)**
• **PARK, Young Hyun
Seongnam-si Gyeonggi-do 13256 (KR)**
• **KIM, Hyun Ju
Seongnam-si Gyeonggi-do 13584 (KR)**

(74) Representative: **Mewburn Ellis LLP
Aurora Building
Counterslip
Bristol BS1 6BX (GB)**

Remarks:
The complete document including Reference
Table(s) and the Sequence Listing(s) can be
downloaded from the EPO website

(54) **IL-2 PROTEIN AND IL-15 PROTEIN CHIMERIC PROTEIN OR VARIANT, FUSION PROTEIN COMPRISING SAME, AND GENETICALLY-ENGINEERED CELL EXPRESSING SAME**

(57)    Disclosed are chimeric proteins including IL-2 and IL-15 proteins, variants thereof, fusion proteins containing the same and/or genetically engineered cells modified to express at least one thereof. The natural killer cells modified to express the chimeric protein including IL-2 and IL-15 proteins, variant thereof, or the fusion protein containing the same reduce binding affinity for IL-2Rβ, suppress side effects caused by excessive immune cell activity of IL-2, and prevent rapid exhaustion of natural killer cells, thereby maintaining an appropriate level of cell activity and cell survival ability for a long time. Therefore, the chimeric proteins of IL-2 and IL-15 proteins, variants thereof, fusion proteins containing the same and/or genetically engineered cells are useful for regulating the immune system and treating immune-related diseases such as cancer, infectious diseases, and autoimmune diseases.

EP 4 772 613 A1

Fig. 1a

**Description**

BACKGROUND OF THE INVENTION

Field of the Invention

The present invention relates to chimeric proteins of IL-2 and IL-15 proteins, variants thereof, fusion proteins containing the same and/or genetically modified cells to express at least one thereof.

Description of the Related Art

Interleukin 2 (IL-2), which is also called "T-cell growth factor (TCGF)", is a 15.5 to 16 kDa globular glycoprotein that plays a pivotal role in lymphocyte production, survival, and homeostasis. IL-2 has a length of 133 amino acids and forms a quaternary structure consisting of four antiparallel, amphipathic $\alpha$-helices (Smith, Science 240, 1169-76 (1988); Bazan, Science 257, 410-413 (1992)).

IL-2 mediates various immune effects by binding to the IL-2 receptor including three subunits (IL-2R), namely, IL-2R$\alpha$ (CD25), $\beta$ (CD122), and $\gamma$ (CD132), and has different binding affinities for IL-2 proteins depending on the different binding of each chain of the IL-2 receptor. Specifically, the trimeric IL-2 receptor composed of $\alpha$, $\beta$, and $\gamma$ chains has high affinity for IL-2 proteins, and the dimeric IL-2 receptor composed of $\beta$ and $\gamma$ chains has an intermediate (about 100-fold lower) binding affinity for IL-2 proteins compared to the trimeric IL-2 receptor, but the monomeric IL-2 receptor composed only of the $\alpha$ chain has a low binding affinity for IL-2 proteins. Both the IL-2 receptor trimers ($\alpha$, $\beta$, $\gamma$) and the IL-2 receptor dimers ($\beta$, $\gamma$) are essential for cell signaling activation based on IL-2 binding (Minami et al., Annu Rev Immunol 11, 245-268 (1993)), but the IL-2 receptor monomer ($\alpha$, CD25) is not essential for cell signaling (Krieg et al., Proc Natl Acad Sci 107, 11906-11 (2010)).

IL-15 is a 14 kDa to 15 kDa glycoprotein that exists in a membrane-bound form attached to the cell surface based on binding to the IL-15 receptor alpha (IL-15R$\alpha$) present on the cell surface. The IL-15/IL-15R$\alpha$ complex binds to IL-2R$\beta$ (CD122) and $\gamma$ (CD132), and shares a cell signaling mechanism with IL-2. (Marek Jakobisiak et al., Cytokine & Growth Factor Reviews 22, 99-108(2011)).

IL-2 receptor trimer ($\alpha$, $\beta$, $\gamma$) containing IL-2 receptor $\alpha$-chain (CD25) is consistently expressed at high levels in FoxP3+ CD+4 regulatory T cells. On the other hand, normal *in vivo* other immune effector cells such as CD8 T cells and NK cells are known to express IL-2 receptor dimer ($\beta$, $\gamma$) in the resting state and transiently express IL-2 receptor trimer ($\alpha$, $\beta$, $\gamma$) when the cells are activated (Fontenot et al., Nature Immunol 6, 1142-51 (2005); H. Asao, Encyclopedia of Endocrine Disease, 60-63 (2004)).

IL-2 is mainly synthesized by activated T cells, especially CD4+ helper T cells, stimulates the proliferation and differentiation of T cells and induces the production of cytotoxic T lymphocytes (CTLs). IL-2 also induces the differentiation of peripheral blood lymphocytes into cytotoxic cells and lymphokine-activated killer (LAK) cells, facilitates the expression of cytokines and cytolytic molecules by T cells, facilitates the proliferation and differentiation of B cells and the synthesis of immunoglobulins by B cells, and stimulates the production, proliferation, and activation of natural killer (NK) cells (reviewed e.g. in Waldmann, Nat Rev Immunol 6, 595-601 (2009); Olejniczak and Kasprzak, Med Sci Monit 14, RA179-89 (2008); Malek, Annu Rev Immunol 26, 453-79 (2008)).

In addition, IL-2 is involved in the maintenance of CD4+CD25+ regulatory T cells (Treg, regulatory T cells), known as suppressive T cells, and inhibits the activity of effector functions of T cells and natural killer cells through cell-cell contact and the release of immunosuppressive cytokines such as IL-10 or TGF-$\beta$ (Fontenot et al., Nature Immunol 6, 1142-51 (2005); D'Cruz and Klein, Nature Immunol 6, 1152-59 (2005)).

IL-2 or IL-15 has a dual function in the immune response in that it increases the lymphocyte populations *in vivo,* enhances the function of these immune cells, and thus mediates the increase and activation of anticancer or immune cells, but suppresses antitumor immunity mediated by CD8+ T cells and natural killer cells by strongly expanding regulatory T (Treg) cells expressing the high-affinity IL-2 receptor. (Brandenburg, S., et al., Eur J Immunol, 2008. 38(6): p. 1643-53; Facciabene, A., et al., Cancer Res, 2012. 72(9): p. 2162-71).

Patients who underwent immunotherapy based on IL-2 have serious cardiovascular, pulmonary, renal, hepatic, gastrointestinal, neurological, skin, hematological, and systemic adverse effects. Therefore, various IL-2 mutations have been studied to improve the therapeutic efficacy of IL-2 and minimize adverse effects (US 5,229,109 B).

An approach to solve the drawbacks of therapy based on IL-2 or IL-15 is to extend the *in vivo* half-life of IL-2 or IL-15 while selectively activating CD8 + T cells and natural killer cells expressing low-affinity IL-2 receptors. Although many attempts have been made toward this goal, there have been no significant results (Arenas-Ramirez, N., et al., Sci Transl Med, 2016. 8(367): p. 367ra166). It has been studied that continuous stimulation of lymphocytes, especially natural killer cells, by IL-15 protein actually inhibits immune enhancement and therapeutic efficacy for cancer (Felices et al., JCI Insight, 2018).

Repeated administration of IL-2 or IL-15 proteins induces hyporesponsiveness of natural killer cells *in vivo,* which reduces anticancer ability due to deteriorated proliferation ability and imbalanced activation mechanism (Frutoso et al, Int J Mol Sci 20(18), 4514(2019)). Therefore, there is demand for continuous improvement of IL-2 variants that can minimize hyporesponsiveness of natural killer cells.

Natural killer cells that were modified to express IL-15 protein in the form of a membrane protein have been found to have higher cell proliferation and survival abilities than natural killer cells that express naïve soluble IL-15 (Masaru Imamura et al, Blood, 2014, 124(7), 1081-1088). However, natural killer cells that were modified to express IL-15 protein in the form of a membrane protein, were unstable in proliferation ability thereof the number of cells depending on the donor, and in most cases, only maintenance or a slight increase in the number of cells was observed.

Under these backgrounds, the present inventors have made efforts to develop a chimeric protein including IL-2 and IL-15 sequences with low affinity and low irritation while lengthening the half-life of IL-2. As a result, the present inventors have found that a chimeric protein in which a part of the domain of IL-2 is replaced with a domain of IL-15, or a variant of a chimeric protein in which a part of the amino acid sequence of the IL-2Rβ binding domain of IL-2 is replaced, has low binding affinity for IL-2Rβ, low activity intensity of STAT5, and excellent inhibitory activity against cancer cell proliferation, and is free from side effects such as pulmonary edema after administration.

Furthermore, the present inventors have found that, when the chimeric protein including IL-2 and IL-15 sequences is expressed in natural killer cells, it exhibits superior proliferation, survival, and activation sustainment abilities than natural killer cells expressing the conventional IL-15 cell membrane protein, and exhibits a potent tumor cell killing ability against a variety of cancers. Based on these findings, the present invention has been completed.

**Prior Art Document**

**Non-patent Document**

(Non-patent Document 1) Smith, Science 240, 1169-76 (1988)
(Non-patent Document 2) Bazan, Science 257, 410-413 (1992)
(Non-patent Document 3) Minami et al., Annu Rev Immunol 11, 245-268 (1993)
(Non-patent Document 4) Krieg et al., Proc Natl Acad Sci 107, 11906-11 (2010)
(Non-patent Document 5) Marek Jakobisiak et al., Cytokine & Growth Factor Reviews 22, 99-108(2011)
(Non-patent Document 6) Fontenot et al., Nature Immunol 6, 1142-51 (2005)
(Non-patent Document 7) H. Asao, Encyclopedia of Endocrine Disease, 60-63 (2004)
(Non-patent Document 8) reviewed e.g. in Waldmann, Nat Rev Immunol 6, 595-601 (2009)
(Non-patent Document 9) Olejniczak and Kasprzak, Med Sci Monit 14, RA179-89 (2008)
(Non-patent Document 10) Malek, Annu Rev Immunol 26, 453-79 (2008)
(Non-patent Document 11) Fontenot et al., Nature Immunol 6, 1142-51 (2005);
(Non-patent Document 12) D'Cruz and Klein, Nature Immunol 6, 1152-59 (2005)
(Non-patent Document 13) Brandenburg, S., et al., Eur J Immunol, 2008. 38(6): p. 1643-53
(Non-patent Document 14) Arenas-Ramirez, N., et al., Sci Transl Med, 2016. 8(367): p. 367ra166
(Non-patent Document 15) Felices et al., JCI Insight 3(3):e96219 (2018)
(Non-patent Document 16) Frutoso et al, Int J Mol Sci 20(18), 4514(2019)
(Non-patent Document 17) Masaru Imamura et al, Blood, 2014, 124(7), 1081-1088

SUMMARY OF THE INVENTION

Therefore, it is one object of the present invention to provide a chimeric protein or variant thereof that reduces the excessive activity of natural killer cells, delays cell exhaustion and thus maintains the activity of immune cells for a long time.

It is another object of the present invention to provide a fusion protein including the chimeric protein or variant thereof.

It is another object of the present invention to provide a nucleic acid encoding the fusion protein including the chimeric protein or variant thereof.

It is another object of the present invention to provide a recombinant vector including the nucleic acid encoding the fusion protein including the chimeric protein or variant thereof.

It is another object of the present invention to provide a genetically modified cells expressing the chimeric protein or the variant thereof, and/or the fusion protein containing the same.

It is another object of the present invention to provide a genetically modified immune cells expressing the chimeric protein or the variant thereof, and/or the fusion protein containing the same.

It is another object of the present invention to provide a method of producing the chimeric protein or a variant thereof, and/or a fusion protein containing the same, the method including: culturing genetically modified cells to express the chimeric protein or a variant thereof, and/or a fusion protein containing the same; and recovering the expressed chimeric protein or variant thereof, and/or fusion protein containing the same.

It is another object of the present invention to provide a pharmaceutical composition for preventing or treating cancer containing the chimeric protein or variant thereof, the fusion protein, and/or the genetically modified cells.

It is another object of the present invention to provide a pharmaceutical composition for preventing or treating infectious diseases containing the chimeric protein or variant thereof, the fusion protein, and/or the genetically modified cells.

It is another object of the present invention to provide a pharmaceutical composition for preventing or treating autoimmune diseases containing the chimeric protein or variant thereof, the fusion protein, and/or the genetically modified cells.

It is another object of the present invention to provide a method of preventing or treating cancer using the chimeric protein or variant thereof, the fusion protein and/or the genetically modified cells, the use thereof for prevention or treatment of cancer, and the use thereof for preparation of a drug for preventing or treating cancer.

It is another object of the present invention to provide a method of preventing or treating infectious diseases using the chimeric protein or the variant thereof, the fusion protein and/or the genetically modified cells, the use thereof for prevention or treatment of infectious diseases, and the use thereof for preparation of a drug for preventing or treating infectious diseases.

It is another object of the present invention to provide a method of preventing or treating autoimmune diseases using the chimeric protein or variant thereof, the fusion protein and/or the genetically modified cells, the use thereof for prevention or treatment of autoimmune diseases, and the use thereof for preparation of a drug for preventing or treating autoimmune diseases.

The present invention provides a chimeric protein or a variant thereof containing IL-2 and IL-15 proteins.

The present invention also provides a fusion protein containing the chimeric protein or the variant thereof.

The present invention also provides genetically modified cells expressing the chimeric protein or the variant thereof, or the fusion protein containing the same.

The present invention also provides a pharmaceutical composition for preventing or treating cancer containing the modified natural killer cells.

The present invention also provides a pharmaceutical composition for preventing or treating an infectious disease containing the chimeric protein or variant thereof, the fusion protein and/or the genetically modified cells.

The present invention also provides a pharmaceutical composition for preventing or treating an autoimmune disease containing the chimeric protein or variant thereof, the fusion protein and/or the genetically modified cells.

The present invention also provides a method of preventing or treating cancer including administering the chimeric protein or variant thereof, the fusion protein and/or the genetically modified cell, the use thereof for prevention or treatment of cancer, and the use thereof for preparation of a drug for preventing or treating cancer.

The present invention also provides a method of preventing or treating an infectious disease including administering the chimeric protein or variant thereof, the fusion protein and/or the genetically modified cell, the use thereof for prevention or treatment of an infectious disease, and the use thereof for preparation of a drug for preventing or treating an infectious disease.

The present invention also provides a method of preventing or treating an autoimmune disease including administering the chimeric protein or variant thereof, the fusion protein and/or the genetically modified cell, the use thereof for prevention or treatment of an autoimmune disease, and the use thereof for preparation of a drug for preventing or treating an autoimmune disease.

BRIEF DESCRIPTION OF THE DRAWINGS

FIG. 1A is a schematic diagram illustrating IL-2 and/or IL-15 chimeric proteins according to the present invention.
FIG. 1B is a schematic diagram illustrating a fusion protein comprising IL-15Ra (sushi domain) and an Fc domain.
FIGS. 1C to 1F are SDS-PAGE gel images showing prepared and purified fusion proteins GIC-982C1 to GIC-982C15.
FIG. 2 is a diagram illustrating the results of Octet binding assay for Fc-IL2, GIC-982C1 to GIC-982C6, and GIC-982C12 to GIC-982C15.
FIG. 3 is a diagram illustrating the results of analysis of STAT-5 cell signaling pathway activity of Fc-IL2, GIC-982C1 to GIC-982C6, and GIC-982C13 to GIC-982C15.
FIG. 4A shows the structure of the insert gene and vector for genetically modifying natural killer cells to express a chimeric protein including IL-2 and IL-15 proteins (control group: mbIL-15, experimental group: mbIL-2/IL-15).

FIG. 4B is a schematic diagram illustrating natural killer cells expressing the produced chimeric protein.

FIG. 5 shows FACS data of genetically modified natural killer cells.

FIG. 6 shows the proliferation ability of natural killer cells depending on the culture period.

FIG. 7 shows the killing ability against tumor cells *in vitro* after treatment with genetically modified natural killer cells.

## DETAILED DESCRIPTION OF THE INVENTION

Unless defined otherwise, all technical and scientific terms used herein have the same meanings as appreciated by those skilled in the field to which the present invention pertains. In general, the nomenclature used herein is well-known in the art and is ordinarily used.

The present inventors made an effort to develop recombinant cytokine with low affinity and low irritation that can maintain the killing ability against cancer cells while suppressing the side effects of the potent cytotoxicity of IL-2 and developed a novel chimeric protein that exhibits excellent inhibitory activity against cancer cell proliferation without causing side effects of conventional IL-2 such as pulmonary edema by modifying non-specific and potent toxicity of IL-2 so that a chimeric protein in which some domains of IL-2 are substituted with the domain of IL-15 has weak binding affinity for IL-2Rβ and low intensity of STAT5 activity compared to wild-type IL-2.

In one aspect, the present invention is related to a chimeric protein including IL-2 and IL-15 proteins.

**Chimeric protein including IL-2 and IL-15 proteins**

Interleukin 2 (IL-2) is a protein encoded by the IL-2 gene in humans, is also called "T-cell growth factor (TCGF)", and is a 15.5 to 16 kDa globular glycoprotein that plays a pivotal role in lymphocyte production, survival, and homeostasis. IL-2 has a length of 133 amino acids and forms a quaternary structure consisting of four antiparallel, amphipathic α-helices.

It was reported that human IL-2 may have a structure in which a "Helix A" domain, an "A-B loop" domain, a "Helix B" domain, a "B-C loop" domain, a "Helix C" domain, a "C-D loop" domain, and a "Helix D" domain are sequentially linked, the "A-B loop" domain, the "Helix B" domain, and the "C-D loop" domain may be involved in IL-2Rα binding, and the "Helix C" domain may be involved in IL-2Rβ binding (Cassell, Current Pharmaceutical Design, 2002, 8, 2171-2183).

The amino acid or nucleic acid sequences of human IL-2 are shown in Table 1 below:

[Table 1]

|  | Amino acid or nucleic acid sequence | SEQ ID NO: |
|---|---|---|
| IL-2 | APTSSSTKKTQLQLEHLLLDLQMILNGINNYKNPKLTRMLTFKFY MPKKATELKHLQCLEEELKPLEEVLNLAQSKNFHLRPRDLISNIN VIVLELKGSETTFMCEYADETATIVEFLNRWITFCQSIISTLT | 1 |
| | GCCCCCACCAGTAGCAGCACCAAGAAGACCCAGCTTCAGCTTGAG CATTTGCTTCTTGATCTCCAGATGATACTGAATGGGATCAACAAT TATAAAAATCCTAAATTGACACGTATGCTGACATTCAAGTTCTAC ATGCCTAAGAAAGCAACCGAGCTTAAACATTTGCAGTGCTTGGAA GAAGAGTTGAAGCCTCTCGAGGAAGTGTTGAACTTGGCTCAATCC AAGAACTTTCATCTTCGGCCCAGAGATTTGATTTCAAATATTAAC GTCATCGTCCTCGAGCTCAAGGGGAGCGAAACCACATTCATGTGT GAGTATGCAGACGAAACCGCCACTATTGTAGAATTTCTTAATCGG TGGATCACATTCTGCCAAAGCATCATTTCAACTCTTACA | 2 |
| IL-2 helix A domain | SSTKKTQLQLEHLLLDLQMILNG | 3 |
| | AGCTCCACCAAGAAGACACAATTGCAACTCGAACACCTTTTGCTC GATCTGCAAATGATCCTCAATGGG | 4 |
| IL-2 A-B loop domain | INNYKNPKLTRMLTFKFYMPKKATE | 5 |
| | ATTAATAATTACAAAAATCCTAAACTTACTCGCATGTTGACATTC AAGTTCTATATGCCAAAAAAAGCTACCGAG | 6 |

(continued)

| | Amino acid or nucleic acid sequence | SEQ ID NO: |
|---|---|---|
| IL-2 helix B domain | LKHLQCLEEELKPLEEVLNLAQ | 7 |
| | CTGAAGCACCTGCAATGCCTTGAGGAAGAACTCAAACCATTGGAA GAGGTTCTGAATTTGGCCCAG | 8 |
| IL-2 B-C loop domain | SKNFHLR | 9 |
| | TCCAAAAACTTTCATCTCCGA | 10 |
| IL-2 helix C domain | PRDLISNINVIVLELKGS | 11 |
| | CCACGAGACCTCATCTCTAATATTAACGTCATTGTACTTGAGCTC AAAGGGAGC | 12 |
| IL-2 C-D loop domain | ETTFMCEYADETAT | 13 |
| | GAGACCACCTTTATGTGCGAGTACGCCGATGAGACAGCTACT | 14 |
| IL-2 helix D domain | IVEFLNRWITFCQSIISTLT | 15 |
| | ATTGTTGAATTCTTGAATAGATGGATTACATTTTGTCAATCTATA ATCTCAACCCTGACC | 16 |

Interleukin 15 (IL-15) is a protein encoded by the IL-15 gene in humans and is a 14 to 15 kDa glycoprotein that exists in a membrane-bound form attached to the cell surface based on binding to the IL-15 receptor alpha (IL-15Rα) present on the cell surface. The IL-15/IL-15Rα complex binds to IL-2Rβ (CD122) and γ (CD132), and shares a cell signaling mechanism with IL-2.

It was reported that human IL-15 may have a structure in which a "Helix A" domain, an "A-B loop" domain, a "Helix B" domain, a "B-C loop" domain, a "Helix C" domain, a "C-D loop" domain, and a "Helix D" domain are sequentially linked, the "A-B loop" domain, the "Helix B" domain, and the "C-D loop" domain may be involved in IL-15Rα binding (Lowe, Journal of Molecular Biology, 2011, 406, 160-175).

The sequences of human IL-15 are shown in Table 2 below:

[Table 2]

| | Amino acid or nucleic acid sequence | SEQ ID NO: |
|---|---|---|
| IL-15 | GIHVFILGCFSAGLPKTEANWVNVISDLKKIEDLIQSMHIDATL YTESDVHPSCKVTAMKCFLLELQVISLESGDASIHDTVENLIIL ANNSLSSNGNVTESGCKECEELEEKNIKEFLQSFVHIVQMFINT S | 17 |
| | GGCATCCACGTGTTCATTCTGGGATGCTTCAGCGCAGGCCTGCC TAAAACCGAGGCGAACTGGGTTAACGTGATCAGCGATTTGAAAA AGATTGAGGATCTGATCCAGTCCATGCATATTGACGCAACCTTG TACACAGAGTCAGATGTGCACCCTTCCTGCAAAGTTACGGCTAT GAAGTGTTTTTTGCTTGAATTGCAAGTAATAAGTTTGGAGTCTG GCGATGCATCTATACATGATACCGTAGAAAATTTGATCATCCTG GCTAATAATAGTTTGTCCAGTAATGGTAACGTAACAGAGAGCGG TTGTAAAGAGTGTGAAGAGCTCGAGGAAAAGAATATAAAAGAAT TCTTGCAATCCTTTGTACATATCGTGCAAATGTTCATTAATACA AGC | 18 |
| IL-15 Helix A domain | ANWVNVISDLKKIEDLIQ | 19 |
| | GCAAATTGGGTGAACGTCATATCTGACCTTAAAAAGATAGAAGA CTTGATCCAA | 20 |

(continued)

| | | Amino acid or nucleic acid sequence | SEQ ID NO: |
|---|---|---|---|
| | | SMHIDATLYTESDVH | 21 |
| | IL-15 A-B loop domain | AGCATGCATATAGACGCAACACTCTACACCGAATCCGATGTCCAT | 22 |
| | IL-15 Helix B domain | PSCKVTAMKCFLLELQVISLE | 23 |
| | | CCCTCCTGCAAGGTCACCGCCATGAAATGCTTTCTGCTCGAGTTGCAGGTTATTAGCCTCGAA | 24 |
| | IL-15 B-C loop domain | SGD | 25 |
| | | AGTGGTGAT | 26 |
| | IL-15 Helix C domain | ASIHDTVENLIILANNSLSS | 27 |
| | | GCAAGTATCCACGATACTGTAGAAAATCTGATCATACTTGCTAACAACTCTCTTAGCAGT | 28 |
| | IL-15 C-D loop domain | NGNVTESGCKECEELEEKN | 29 |
| | | AATGGCAATGTCACTGAGTCTGGCTGTAAAGAGTGTGAGGAACTCGAGGAAAAGAAC | 30 |
| | IL-15 Helix D domain | IKEFLQSFVHIVQMFINTS | 31 |
| | | ATAAAGGAATTTCTTCAGTCCTTCGTTCATATCGTTCAGATGTTTATCAACACCAGC | 32 |

IL-2 or IL-15 protein can increase the lymphocyte population *in vivo* and enhance the function of the immune cells, so that IL-2 or IL-15 can be used to mediate the increase and activation of anticancer agents or immune cells. However, IL-2 or IL-15 has a dual function of immune response in that it suppresses antitumor immunity mediated by CD8+ T cells and natural killer cells by strongly expanding regulatory T cells expressing high-affinity IL-2 receptors. In particular, repeated administration of IL-2 or IL-15 protein causes hyporesponsiveness of natural killer cells *in vivo,* thereby reducing proliferation ability, rapid exhaustion, and anticancer ability due to imbalance in activation mechanism.

In one embodiment of the present invention, the chimeric protein may include a part of the IL-2 amino acid sequence and a part of the IL-15 amino acid sequence.

In one embodiment of the present invention, the chimeric protein may include a part of the IL-2 amino acid sequence and a part of the IL-15 amino acid sequence which are directly linked to each other or indirectly linked via a linker or the like.

In one embodiment of the present invention, the chimeric protein may be produced by substituting a part of the IL-2 amino acid sequence with the IL-15 amino acid sequence. As used herein, "protein including an amino acid sequence substituted with a certain amino acid sequence" is used interchangeably with "hybrid protein".

When a part of the IL-2 amino acid sequence is substituted with the IL-15 amino acid sequence, as in the chimeric protein of the present invention, by controlling the intensity of the signaling mechanism of the IL-2Rβ (CD122) and γ (CD132) dimeric receptors, excessive activity of natural killer cells can be prevented, cell exhaustion can be delayed, and the activity of immune cells can be maintained for a long time.

In one embodiment of the present invention, at least one domain selected from the group consisting of "Helix A" domain, the "A-B loop" domain, the "Helix B" domain, the "B-C loop" domain, the "Helix C" domain, the "C-D loop" domain, and the "Helix D" domain of IL-2 may be substituted with the "Helix A" domain, the "A-B loop" domain, the "Helix B" domain, the "B-C loop" domain, the "Helix C" domain, the "C-D loop" domain, or the "Helix D" domain of IL-15.

In a preferred embodiment of the present invention, at least one domain selected from the group consisting of the "A-B loop" domain, the "Helix B" domain, and the "C-D loop" domain of IL-2 may be substituted with the "A-B loop" domain, the "Helix B" domain, or the "C-D loop" domain of IL-15.

In a preferred embodiment of the present invention, the "A-B loop" domain, the "Helix B" domain and the "C-D loop" domain of IL-2 may be substituted with the "A-B loop" domain, the "Helix B" domain or the "C-D loop" domain of IL-15. For example, the amino acid sequence of the chimeric protein or the nucleic acid sequence for encoding the same may include or consist of the amino acid sequence of SEQ ID NO: 33 or the nucleic acid sequence encoding the amino acid sequence of SEQ ID NO: 34 in shown in Table 3 below.

[Table 3]

| | Amino acid or nucleic acid sequence | SEQ ID NO: |
|---|---|---|
| IL-2/15 chimeric protein | APTSSSTKKTQLQLEHLLLDLQMILNGSMHIDATLYTESDVHPS CKVTAMKCFLLELQVISLESKNFHLRPRDLISNINVIVLELKGS NGNVTESGCKECEELEEKNIVEFLNRWITFCQSIISTLT | 33 |
| | GCTCCTACCTCCTCCAGCACCAAGAAAACCCAGCTGCAGTTGGA GCATCTGCTGCTGGACCTGCAGATGATCCTGAACGGCTCCATGC ACATCGACGCTACCCTGTACACCGAGTCCGACGTGCACCCTTCC TGTAAAGTGACCGCCATGAAGTGCTTTCTGTTGGAGCTGCAAGT GATCTCCCTGGAATCCAAGAACTTCCACCTGAGGCCTCGGGACC TGATCTCCAACATCAACGTGATCGTGCTGGAACTGAAGGGCTCC AACGGCAACGTGACCGAGTCTGGCTGTAAAGAGTGCGAGGAACT GGAAGAGAAGAACATCGTCGAGTTTCTGAACCGGTGGATCACCT TCTGCCAGTCCATCATCTCCACACTGACC | 34 |

## Variant of chimeric protein including IL-2 and IL-15 proteins

In another aspect, the present invention is related to a variant of a chimeric protein including IL-2 and IL-15 proteins.

The variant of the chimeric protein in which one or more amino acids of the amino acid sequence of the IL-2Rβ binding domain of IL-2 is mutated according to the present invention further reduces binding affinity for IL-2Rβ and lowers STAT5 activity intensity, thereby preventing excessive activation of natural killer cells, delaying cell exhaustion, and maintaining the activity of immune cells for a long time.

As used herein, the term "variant" includes not only mutations of one or more amino acid residues, preferably substitutions, deletions, insertions, and the like of amino acid residues in the wild-type amino acid sequence, but also truncation of one or more amino acid residues at the N-terminus or C-terminus, and therefore, in the present invention, the variant of the chimeric protein is used as a broad concept including a "fragment of a chimeric protein variant".

As used herein, the term "substitution" refers to modification that includes replacing one or more amino acids with amino acids having similar biochemical properties without causing loss of biological or biochemical function.

In one embodiment of the present invention, the variant of the chimeric protein may include a mutation, preferably substitution of amino acid, at at least one position of aspartic acid (Asp(D)) at position 73, leucine (Leu(L), Leucine) at position 74, serine (Ser(S)) at position 76, asparagine (Asn(N)) at position 77, and valine (Val(V)) at position 80 in the amino acid sequence of SEQ ID NO.: 33.

In one embodiment of the present invention, the variant of the chimeric protein may include substitution of at least one amino acid of D73E, L74F, S76D, S76E, S76N, S76K, S76L, N77D and V80L in the amino acid sequence of SEQ ID NO.: 33.

In one embodiment of the present invention, the variant of the chimeric protein may include one variant combination selected from D73E/L74F, D73E/S76D, D73E/S76E, D73E/S76N, D73E/S76K, D73E/S76L, D73E/N77D, D73E/V80L, L74F/S76D, L74F/S76E, L74F/S76N, L74F/S76K, L74F/S76L, L74F/N77D, L74F/V80L, S76D/N77D, S76D/V80L, S76E/N77D, S76E/V80L, S76N/N77D, S76N/V80L, S76K/N77D, S76K/V80L, S76L/N77D, S76L/V80L, D73E/L74F/S76D, D73E/L74F/S76E, D73E/L74F/S76N, D73E/L74F/S76K, D73E/L74F/S76L, D73E/L74F/N77D, D73E/L74F/V80L, D73E/S76D/N77D, D73E/S76D/V80L, D73E/S76E/N77D, D73E/S76E/V80L, D73E/S76N/N77D, D73E/S76N/V80L, D73E/S76K/N77D, D73E/S76K/V80L, D73E/S76L/N77D, D73E/S76L/V80L, D73E/N77D/V80L, S76D/N77D/V80L, S76E/N77D/V80L, S76N/N77D/V80L, S76K/N77D/V80L, S76L/N77D/V80L, D73E/L74F/S76D/N77D, D73E/L74F/S76E/N77D, D73E/L74F/S76N/N77D, D73E/L74F/S76K/N77D, D73E/L74F/S76L/N77D, D73E/L74F/S76D/V80L, D73E/L74F/S76E/V80L, D73E/L74F/S76N/V80L, D73E/L74F/S76K/V80L, D73E/L74F/S76L/V80L, L74F/S76D/N77D/V80L, L74F/S76E/N77D/V80L, L74F/S76N/N77D/V80L, L74F/S76K/N77D/V80L, L74F/S76L/N77D/V80L, D73E/L74F/S76D/N77D/V80L, D73E/L74F/-S76E/N77D/V80L, D73E/L74F/S76N/N77D/V80L, D73E/L74F/S76K/N77D/V80L, and D73E/L74F/S76L/N77D/V80L.

In a preferred embodiment of the present invention, the variant of the chimeric protein may include at least one amino acid substitution of D73E, L74F, S76D, N77D and V80L in the amino acid sequence of SEQ ID NO.: 33.

In a preferred embodiment of the present invention, the amino acid sequence or nucleic acid sequence of the variant of the chimeric protein may include or consist of an amino acid sequence of SEQ ID NO.: 35, 37, 39, 41, 43, 45, 47, 49, 51, 53, 55, 57, 59, 61, 63, 65, 67, 69, 71, 73 or 75, or a nucleic acid sequence encoding the same of SEQ ID NO: 36, 38, 40, 42, 44, 46, 48, 50, 52, 54, 56, 58, 60, 62, 64, 66, 70, 72 or 74 as set forth in Table 4 below.

[Table 4]

| IL-2/15 variant (variation) | Amino acid or nucleic acid sequence | SEQ ID NO: |
|---|---|---|
| **IL-2/15v1 (D73E)** | APTSSSTKKTQLQLEHLLLDLQMILNGSMHIDATLYTESDVH PSCKVTAMKCFLLELQVISLESKNFHLRPR**E**LISNINVIVLE LKGSNGNVTESGCKECEELEEKNIVEFLNRWITFCQSIISTL T | 35 |
|  | GCTCCTACCTCCTCCAGCACCAAGAAAACCCAGCTGCAGTTG GAGCATCTGCTGCTGGACCTGCAGATGATCCTGAACGGCTCC ATGCACATCGACGCTACCCTGTACACCGAGTCCGACGTGCAC CCTTCCTGTAAAGTGACCGCCATGAAGTGCTTTCTGCTCGAG CTGCAAGTGATCTCCCTGGAATCCAAGAACTTCCACCTGAGG CCAAGAGAGCTGATCTCCAACATCAACGTGATCGTGCTGGAA CTGAAGGGCTCCAACGGCAACGTGACCGAGTCTGGCTGTAAA GAGTGCGAGGAACTGGAAGAGAAGAACATCGTCGAGTTTCTG AACCGGTGGATCACCTTCTGCCAGTCCATCATCTCCACACTG ACC | 36 |
| **IL-2/15v2 (L74F)** | APTSSSTKKTQLQLEHLLLDLQMILNGSMHIDATLYTESDVH PSCKVTAMKCFLLELQVISLESKNFHLRPRD**F**ISNINVIVLE LKGSNGNVTESGCKECEELEEKNIVEFLNRWITFCQSIISTL T | 37 |
|  | GCTCCTACCTCCTCCAGCACCAAGAAAACCCAGCTGCAGTTG GAGCATCTGCTGCTGGACCTGCAGATGATCCTGAACGGCTCC ATGCACATCGACGCTACCCTGTACACCGAGTCCGACGTGCAC CCTTCCTGTAAAGTGACCGCCATGAAGTGCTTTCTGCTCGAG CTGCAAGTGATCTCCCTGGAATCCAAGAACTTCCACCTGAGG CCTCGGGACTTCATCTCCAACATCAACGTGATCGTGCTGGAA CTGAAGGGCTCCAACGGCAACGTGACCGAGTCTGGCTGTAAA GAGTGCGAGGAACTGGAAGAGAAGAACATCGTCGAGTTTCTG AACCGGTGGATCACCTTCTGCCAGTCCATCATCTCCACACTG ACC | 38 |
| **IL-2/15v3 (S76D)** | APTSSSTKKTQLQLEHLLLDLQMILNGSMHIDATLYTESDVH PSCKVTAMKCFLLELQVISLESKNFHLRPRDLI**D**NINVIVLE | 39 |

(continued)

| IL-2/15 variant (variation) | Amino acid or nucleic acid sequence | SEQ ID NO: |
|---|---|---|
| | LKGSNGNVTESGCKECEELEEKNIVEFLNRWITFCQSIISTL T | |
| | GCTCCCACAAGTTCAAGTACTAAAAAAACCCAACTGCAACTG GAGCATTTGCTTTTGGATCTCCAAATGATTCTGAATGGCTCA ATGCACATTGACGCTACTCTTTACACAGAGAGTGATGTACAC CCTTCTTGCAAGGTTACCGCTATGAAATGCTTTCTCTTGGAA CTCCAAGTAATCAGTCTTGAATCTAAAAATTTTCATTTGCGC CCACGCGATCTGATAGACAACATCAACGTGATAGTACTGGAA CTCAAGGGAAGCAATGGAAACGTCACAGAATCAGGTTGTAAA GAATGTGAGGAATTGGAAGAAAGAATATAGTCGAGTTCCTC AATCGCTGGATCACTTTTTGCCAATCCATTATATCCACTCTG ACA | 40 |
| IL-2/15v4 (N77D) | APTSSSTKKTQLQLEHLLLDLQMILNGSMHIDATLYTESDVH PSCKVTAMKCFLLELQVISLESKNFHLRPRDLIS**D**INVIVLE LKGSNGNVTESGCKECEELEEKNIVEFLNRWITFCQSIISTL T | 41 |
| | GCTCCTACCTCCTCCAGCACCAAGAAAACCCAGCTGCAGTTG GAGCATCTGCTGCTGGACCTGCAGATGATCCTGAACGGCTCC ATGCACATCGACGCTACCCTGTACACCGAGTCCGACGTGCAC CCTTCCTGTAAAGTGACCGCCATGAAGTGCTTTCTGCTCGAG CTGCAAGTGATCTCCCTGGAATCCAAGAACTTCCACCTGAGG CCTCGGGACCTGATCTCCGACATCAACGTGATCGTGCTGGAA CTGAAGGGCTCCAACGGCAACGTGACCGAGTCTGGCTGTAAA GAGTGCGAGGAACTGGAAGAGAAGAACATCGTCGAGTTTCTG AACCGGTGGATCACCTTCTGCCAGTCCATCATCTCCACACTG ACC | 42 |
| IL-2/15v5 (V80L) | APTSSSTKKTQLQLEHLLLDLQMILNGSMHIDATLYTESDVH PSCKVTAMKCFLLELQVISLESKNFHLRPRDLISNIN**L**IVLE LKGSNGNVTESGCKECEELEEKNIVEFLNRWITFCQSIISTL T | 43 |
| | GCTCCTACCTCCTCCAGCACCAAGAAAACCCAGCTGCAGTTG GAGCATCTGCTGCTGGACCTGCAGATGATCCTGAACGGCTCC ATGCACATCGACGCTACCCTGTACACCGAGTCCGACGTGCAC CCTTCCTGTAAAGTGACCGCCATGAAGTGCTTTCTGCTCGAG CTGCAAGTGATCTCCCTGGAATCCAAGAACTTCCACCTGAGG CCTCGGGACCTGATCTCCAACATCAACCTGATCGTGCTGGAA CTGAAGGGCTCCAACGGCAACGTGACCGAGTCTGGCTGTAAA GAGTGCGAGGAACTGGAAGAGAAGAACATCGTCGAGTTTCTG AACCGGTGGATCACCTTCTGCCAGTCCATCATCTCCACACTG ACC | 44 |

(continued)

| IL-2/15 variant (variation) | Amino acid or nucleic acid sequence | SEQ ID NO: |
|---|---|---|
| IL-2/15v6 (L74F, S76D, N77D, V80L) | APTSSSTKKTQLQLEHLLLDLQMILNGSMHIDATLYTESDVH PSCKVTAMKCFLLELQVISLESKNFHLRPRD**F**I**DD**IN**L**IVLE LKGSNGNVTESGCKECEELEEKNIVEFLNRWITFCQSIISTL T | 45 |
| | GCTCCTACCTCCTCCAGCACCAAGAAAACCCAGCTGCAGTTG GAGCATCTGCTGCTGGACCTGCAGATGATCCTGAACGGCTCC ATGCACATCGACGCTACCCTGTACACCGAGTCCGACGTGCAC CCTTCCTGTAAAGTGACCGCCATGAAGTGCTTTCTGCTCGAG<br><br>CTGCAAGTGATCTCCCTGGAATCCAAGAACTTCCACCTGAGG CCTCGGGACTTCATCGACGACATCAACCTGATCGTGCTGGAA CTGAAGGGCTCCAACGGCAACGTGACCGAGTCTGGCTGTAAA GAGTGCGAGGAACTGGAAGAGAAGAACATCGTCGAGTTTCTG AACCGGTGGATCACCTTCTGCCAGTCCATCATCTCCACACTG ACC | 46 |
| IL-2/15v7 (D73E, S76D, N77D, V80L) | APTSSSTKKTQLQLEHLLLDLQMILNGSMHIDATLYTESDVH PSCKVTAMKCFLLELQVISLESKNFHLRPR**E**LI**DD**IN**L**IVLE LKGSNGNVTESGCKECEELEEKNIVEFLNRWITFCQSIISTL T | 47 |
| | GCTCCTACCTCCTCCAGCACCAAGAAAACCCAGCTGCAGTTG GAGCATCTGCTGCTGGACCTGCAGATGATCCTGAACGGCTCC ATGCACATCGACGCTACCCTGTACACCGAGTCCGACGTGCAC CCTTCCTGTAAAGTGACCGCCATGAAGTGCTTTCTGCTCGAG CTGCAAGTGATCTCCCTGGAATCCAAGAACTTCCACCTGAGG CCAAGAGAGCTGATCGACGACATCAACCTGATCGTGCTGGAA CTGAAGGGCTCCAACGGCAACGTGACCGAGTCTGGCTGTAAA GAGTGCGAGGAACTGGAAGAGAAGAACATCGTCGAGTTTCTG AACCGGTGGATCACCTTCTGCCAGTCCATCATCTCCACACTG ACC | 48 |
| IL-2/15v8 (D73E, L74F, N77D, V80L) | APTSSSTKKTQLQLEHLLLDLQMILNGSMHIDATLYTESDVH PSCKVTAMKCFLLELQVISLESKNFHLRPR**EF**IS**D**IN**L**IVLE LKGSNGNVTESGCKECEELEEKNIVEFLNRWITFCQSIISTL T | 49 |
| | GCTCCTACCTCCTCCAGCACCAAGAAAACCCAGCTGCAGTTG GAGCATCTGCTGCTGGACCTGCAGATGATCCTGAACGGCTCC ATGCACATCGACGCTACCCTGTACACCGAGTCCGACGTGCAC CCTTCCTGTAAAGTGACCGCCATGAAGTGCTTTCTGCTCGAG CTGCAAGTGATCTCCCTGGAATCCAAGAACTTCCACCTGAGG CCAAGAGAGTTCATCTCCGACATCAACCTGATCGTGCTGGAA CTGAAGGGCTCCAACGGCAACGTGACCGAGTCTGGCTGTAAA GAGTGCGAGGAACTGGAAGAGAAGAACATCGTCGAGTTTCTG AACCGGTGGATCACCTTCTGCCAGTCCATCATCTCCACACTG ACC | 50 |

(continued)

| IL-2/15 variant (variation) | Amino acid or nucleic acid sequence | SEQ ID NO: |
|---|---|---|
| **IL-2/15v9 (D73E, L74F, S76D, V80L)** | APTSSSTKKTQLQLEHLLLDLQMILNGSMHIDATLYTESDVH PSCKVTAMKCFLLELQVISLESKNFHLRPR**EF**I**D**NIN**L**IVLE LKGSNGNVTESGCKECEELEEKNIVEFLNRWITFCQSIISTL T | 51 |
| | GCTCCTACCTCCTCCAGCACCAAGAAAACCCAGCTGCAGTTG GAGCATCTGCTGCTGGACCTGCAGATGATCCTGAACGGCTCC ATGCACATCGACGCTACCCTGTACACCGAGTCCGACGTGCAC CCTTCCTGTAAAGTGACCGCCATGAAGTGCTTTCTGCTCGAG CTGCAAGTGATCTCCCTGGAATCCAAGAACTTCCACCTGAGG CCAAGAGAGTTCATCGACAACATCAACCTGATCGTCCTGGAA CTGAAGGGCTCCAACGGCAACGTGACCGAGTCTGGCTGTAAA GAGTGCGAGGAACTGGAAGAGAAGAACATCGTCGAGTTTCTG AACCGGTGGATCACCTTCTGCCAGTCCATCATCTCCACACTG ACC | 52 |
| **IL-2/15v10 (D73E, L74F, S76D, N77D)** | APTSSSTKKTQLQLEHLLLDLQMILNGSMHIDATLYTESDVH PSCKVTAMKCFLLELQVISLESKNFHLRPR**EF**I**DD**INVIVLE LKGSNGNVTESGCKECEELEEKNIVEFLNRWITFCQSIISTL T | 53 |
| | GCTCCTACCTCCTCCAGCACCAAGAAAACCCAGCTGCAGTTG GAGCATCTGCTGCTGGACCTGCAGATGATCCTGAACGGCTCC ATGCACATCGACGCTACCCTGTACACCGAGTCCGACGTGCAC CCTTCCTGTAAAGTGACCGCCATGAAGTGCTTTCTGCTCGAG CTGCAAGTGATCTCCCTGGAATCCAAGAACTTCCACCTGAGG CCAAGAGAGTTCATCGACGACATCAACGTGATCGTGCTGGAA CTGAAGGGCTCCAACGGCAACGTGACCGAGTCTGGCTGTAAA GAGTGCGAGGAACTGGAAGAGAAGAACATCGTCGAGTTTCTG AACCGGTGGATCACCTTCTGCCAGTCCATCATCTCCACACTG ACC | 54 |
| **IL-2/15v11 (D73E, L74F, S76D, N77D, V80L)** | APTSSSTKKTQLQLEHLLLDLQMILNGSMHIDATLYTESDVH PSCKVTAMKCFLLELQVISLESKNFHLRPR**EF**I**DD**IN**L**IVLE LKGSNGNVTESGCKECEELEEKNIVEFLNRWITFCQSIISTL T | 55 |
| | GCTCCTACCTCCTCCAGCACCAAGAAAACCCAGCTGCAGTTG GAGCATCTGCTGCTGGACCTGCAGATGATCCTGAACGGCTCC ATGCACATCGACGCTACCCTGTACACCGAGTCCGACGTGCAC CCTTCCTGTAAAGTGACCGCCATGAAGTGCTTTCTGCTCGAG CTGCAAGTGATCTCCCTGGAATCCAAGAACTTCCACCTGAGG CCAAGAGAGTTCATCGACGACATCAACCTGATCGTGCTGGAA CTGAAGGGCTCCAACGGCAACGTGACCGAGTCTGGCTGTAAA GAGTGCGAGGAACTGGAAGAGAAGAACATCGTCGAGTTTCTG AACCGGTGGATCACCTTCTGCCAGTCCATCATCTCCACACTG ACC | 56 |

(continued)

| IL-2/15 variant (variation) | Amino acid or nucleic acid sequence | SEQ ID NO: |
|---|---|---|
| IL-2/15v12 (S76E) | APTSSSTKKTQLQLEHLLLDLQMILNGSMHIDATLYTESDVH PSCKVTAMKCFLLELQVISLESKNFHLRPRDLI**E**NINVIVLE LKGSNGNVTESGCKECEELEEKNIVEFLNRWITFCQSIISTL T | 57 |
| | GCTCCTACCTCCTCCAGCACCAAGAAAACCCAGCTGCAGTTG GAGCATCTGCTGCTGGACCTGCAGATGATCCTGAACGGCTCC ATGCACATCGACGCTACCCTGTACACCGAGTCCGACGTGCAC CCTTCCTGTAAAGTGACCGCCATGAAGTGCTTTCTGTTGGAG CTGCAAGTGATCTCCCTGGAATCCAAGAACTTCCACCTGAGG CCTCGGGACCTGATCGAAAACATCAACGTGATCGTGCTGGAA CTGAAGGGCTCCAACGGCAACGTGACCGAGTCTGGCTGTAAA GAGTGCGAGGAACTGGAAGAGAAGAACATCGTCGAGTTTCTG AACCGGTGGATCACCTTCTGCCAGTCCATCATCTCCACACTG ACC | 58 |
| IL-2/15v13 (S76N) | APTSSSTKKTQLQLEHLLLDLQMILNGSMHIDATLYTESDVH PSCKVTAMKCFLLELQVISLESKNFHLRPRDLI**N**NINVIVLE LKGSNGNVTESGCKECEELEEKNIVEFLNRWITFCQSIISTL T | 59 |
| | GCTCCTACCTCCTCCAGCACCAAGAAAACCCAGCTGCAGTTG GAGCATCTGCTGCTGGACCTGCAGATGATCCTGAACGGCTCC ATGCACATCGACGCTACCCTGTACACCGAGTCCGACGTGCAC CCTTCCTGTAAAGTGACCGCCATGAAGTGCTTTCTGTTGGAG CTGCAAGTGATCTCCCTGGAATCCAAGAACTTCCACCTGAGG CCTCGGGACCTGATCAACAACATCAACGTGATCGTGCTGGAA CTGAAGGGCTCCAACGGCAACGTGACCGAGTCTGGCTGTAAA GAGTGCGAGGAACTGGAAGAGAAGAACATCGTCGAGTTTCTG AACCGGTGGATCACCTTCTGCCAGTCCATCATCTCCACACTG ACC | 60 |
| IL-2/15v14 (S76K) | APTSSSTKKTQLQLEHLLLDLQMILNGSMHIDATLYTESDVH PSCKVTAMKCFLLELQVISLESKNFHLRPRDLI**K**NINVIVLE LKGSNGNVTESGCKECEELEEKNIVEFLNRWITFCQSIISTL T | 61 |
| | GCTCCTACCTCCTCCAGCACCAAGAAAACCCAGCTGCAGTTG GAGCATCTGCTGCTGGACCTGCAGATGATCCTGAACGGCTCC ATGCACATCGACGCTACCCTGTACACCGAGTCCGACGTGCAC CCTTCCTGTAAAGTGACCGCCATGAAGTGCTTTCTGTTGGAG CTGCAAGTGATCTCCCTGGAATCCAAGAACTTCCACCTGAGG CCTCGGGACCTGATCAAGAACATCAACGTGATCGTGCTGGAA CTGAAGGGCTCCAACGGCAACGTGACCGAGTCTGGCTGTAAA GAGTGCGAGGAACTGGAAGAGAAGAACATCGTCGAGTTTCTG AACCGGTGGATCACCTTCTGCCAGTCCATCATCTCCACACTG ACC | 62 |

(continued)

| IL-2/15 variant (variation) | Amino acid or nucleic acid sequence | SEQ ID NO: |
|---|---|---|
| IL-2/15v15 (S76L) | APTSSSTKKTQLQLEHLLLDLQMILNGSMHIDATLYTESDVH PSCKVTAMKCFLLELQVISLESKNFHLRPRDLI**L**NINVIVLE LKGSNGNVTESGCKECEELEEKNIVEFLNRWITFCQSIISTL T | 63 |
| | GCTCCTACCTCCTCCAGCACCAAGAAAACCCAGCTGCAGTTG GAGCATCTGCTGCTGGACCTGCAGATGATCCTGAACGGCTCC ATGCACATCGACGCTACCCTGTACACCGAGTCCGACGTGCAC CCTTCCTGTAAAGTGACCGCCATGAAGTGCTTTCTGTTGGAG CTGCAAGTGATCTCCCTGGAATCCAAGAACTTCCACCTGAGG CCTCGGGACCTGATCCTCAACATCAACGTGATCGTGCTGGAA CTGAAGGGCTCCAACGGCAACGTGACCGAGTCTGGCTGTAAA GAGTGCGAGGAACTGGAAGAGAAGAACATCGTCGAGTTTCTG AACCGGTGGATCACCTTCTGCCAGTCCATCATCTCCACACTG ACC | 64 |
| IL-2/15v16 (L74F, S76D) | APTSSSTKKTQLQLEHLLLDLQMILNGSMHIDATLYTESDVH PSCKVTAMKCFLLELQVISLESKNFHLRPRD**F**I**D**NINVIVLE LKGSNGNVTESGCKECEELEEKNIVEFLNRWITFCQSIISTL T | 65 |
| | GCTCCTACCTCCTCCAGCACCAAGAAAACCCAGCTGCAGTTG GAGCATCTGCTGCTGGACCTGCAGATGATCCTGAACGGCTCC ATGCACATCGACGCTACCCTGTACACCGAGTCCGACGTGCAC CCTTCCTGTAAAGTGACCGCCATGAAGTGCTTTCTGCTCGAG CTGCAAGTGATCTCCCTGGAATCCAAGAACTTCCACCTGAGG CCTCGGGACTTCATCGACAACATCAACGTGATCGTGCTGGAA CTGAAGGGCTCCAACGGCAACGTGACCGAGTCTGGCTGTAAA GAGTGCGAGGAACTGGAAGAGAAGAACATCGTCGAGTTTCTG AACCGGTGGATCACCTTCTGCCAGTCCATCATCTCCACACTG ACC | 66 |
| IL-2/15v17 (S76D, N77D) | APTSSSTKKTQLQLEHLLLDLQMILNGSMHIDATLYTESDVH PSCKVTAMKCFLLELQVISLESKNFHLRPRDLI**DD**INVIVLE LKGSNGNVTESGCKECEELEEKNIVEFLNRWITFCQSIISTL T | 67 |
| | GCTCCTACCTCCTCCAGCACCAAGAAAACCCAGCTGCAGTTG GAGCATCTGCTGCTGGACCTGCAGATGATCCTGAACGGCTCC ATGCACATCGACGCTACCCTGTACACCGAGTCCGACGTGCAC CCTTCCTGTAAAGTGACCGCCATGAAGTGCTTTCTGCTCGAG CTGCAAGTGATCTCCCTGGAATCCAAGAACTTCCACCTGAGG CCTCGGGACCTGATCGACGACATCAACGTGATCGTGCTGGAA CTGAAGGGCTCCAACGGCAACGTGACCGAGTCTGGCTGTAAA GAGTGCGAGGAACTGGAAGAGAAGAACATCGTCGAGTTTCTG AACCGGTGGATCACCTTCTGCCAGTCCATCATCTCCACACTG ACC | 68 |

(continued)

| IL-2/15 variant (variation) | Amino acid or nucleic acid sequence | SEQ ID NO: |
|---|---|---|
| IL-2/15v18 (S76D, V80L) | APTSSSTKKTQLQLEHLLLDLQMILNGSMHIDATLYTESDVH PSCKVTAMKCFLLELQVISLESKNFHLRPRDLI**D**NIN**L**IVLE LKGSNGNVTESGCKECEELEEKNIVEFLNRWITFCQSIISTL T | 69 |
| | GCTCCTACCTCCTCCAGCACCAAGAAAACCCAGCTGCAGTTG GAGCATCTGCTGCTGGACCTGCAGATGATCCTGAACGGCTCC ATGCACATCGACGCTACCCTGTACACCGAGTCCGACGTGCAC CCTTCCTGTAAAGTGACCGCCATGAAGTGCTTTCTGCTCGAG CTGCAAGTGATCTCCCTGGAATCCAAGAACTTCCACCTGAGG CCTCGGGACCTGATCGACAACATCAACCTGATCGTGCTGGAA CTGAAGGGCTCCAACGGCAACGTGACCGAGTCTGGCTGTAAA GAGTGCGAGGAACTGGAAGAGAAGAACATCGTCGAGTTTCTG AACCGGTGGATCACCTTCTGCCAGTCCATCATCTCCACACTG ACC | 70 |
| IL-2/15v19 (L74F, S76D, N77D) | APTSSSTKKTQLQLEHLLLDLQMILNGSMHIDATLYTESDVH PSCKVTAMKCFLLELQVISLESKNFHLRPRD**F**I**DD**INVIVLE LKGSNGNVTESGCKECEELEEKNIVEFLNRWITFCQSIISTL T | 71 |
| | GCTCCTACCTCCTCCAGCACCAAGAAAACCCAGCTGCAGTTG GAGCATCTGCTGCTGGACCTGCAGATGATCCTGAACGGCTCC ATGCACATCGACGCTACCCTGTACACCGAGTCCGACGTGCAC CCTTCCTGTAAAGTGACCGCCATGAAGTGCTTTCTGCTCGAG CTGCAAGTGATCTCCCTGGAATCCAAGAACTTCCACCTGAGG CCTCGGGACTTCATCGACGACATCAACGTGATCGTGCTGGAA CTGAAGGGCTCCAACGGCAACGTGACCGAGTCTGGCTGTAAA GAGTGCGAGGAACTGGAAGAGAAGAACATCGTCGAGTTTCTG AACCGGTGGATCACCTTCTGCCAGTCCATCATCTCCACACTG ACC | 72 |
| IL-2/15v20 (S76D, N77D, V80L) | APTSSSTKKTQLQLEHLLLDLQMILNGSMHIDATLYTESDVH PSCKVTAMKCFLLELQVISLESKNFHLRPRDLI**DD**IN**L**IVLE LKGSNGNVTESGCKECEELEEKNIVEFLNRWITFCQSIISTL T | 73 |
| | GCTCCTACCTCCTCCAGCACCAAGAAAACCCAGCTGCAGTTG GAGCATCTGCTGCTGGACCTGCAGATGATCCTGAACGGCTCC ATGCACATCGACGCTACCCTGTACACCGAGTCCGACGTGCAC CCTTCCTGTAAAGTGACCGCCATGAAGTGCTTTCTGCTCGAG CTGCAAGTGATCTCCCTGGAATCCAAGAACTTCCACCTGAGG CCTCGGGACCTGATCGACGACATCAACCTGATCGTGCTGGAA CTGAAGGGCTCCAACGGCAACGTGACCGAGTCTGGCTGTAAA GAGTGCGAGGAACTGGAAGAGAAGAACATCGTCGAGTTTCTG AACCGGTGGATCACCTTCTGCCAGTCCATCATCTCCACACTG ACC | 74 |

(continued)

| IL-2/15 variant (variation) | Amino acid or nucleic acid sequence | SEQ ID NO: |
|---|---|---|
| IL-2/15v21 (L74F, S76D, V80L) | APTSSSTKKTQLQLEHLLLDLQMILNGSMHIDATLYTESDVH PSCKVTAMKCFLLELQVISLESKNFHLRPRD**FID**NIN**L**IVLE LKGSNGNVTESGCKECEELEEKNIVEFLNRWITFCQSIISTL T | 75 |
| | GCTCCTACCTCCTCCAGCACCAAGAAAACCCAGCTGCAGTTG GAGCATCTGCTGCTGGACCTGCAGATGATCCTGAACGGCTCC ATGCACATCGACGCTACCCTGTACACCGAGTCCGACGTGCAC CCTTCCTGTAAAGTGACCGCCATGAAGTGCTTTCTGCTCGAG CTGCAAGTGATCTCCCTGGAATCCAAGAACTTCCACCTGAGG CCTCGGGACTTCATCGACAACATCAACCTGATCGTGCTGGAA CTGAAGGGCTCCAACGGCAACGTGACCGAGTCTGGCTGTAAA GAGTGCGAGGAACTGGAAGAGAAGAACATCGTCGAGTTTCTG AACCGGTGGATCACCTTCTGCCAGTCCATCATCTCCACACTG ACC | 76 |
| * Underlined parts of amino acid sequences indicate mutation locations. | | |

**Fusion protein including chimeric protein including IL-2 and IL-15 proteins or variant thereof**

In another aspect, the present invention is related to a fusion protein including the chimeric protein or variant thereof.

As used herein, the term "fusion protein" refers to a protein including another fusion domain or amino acid sequence in addition to the chimeric protein including IL-2 and IL-15 proteins or variant thereof.

As used herein, the term "fusion protein" refers to a protein in which the chimeric protein or variant thereof is fused with an additional peptide sequence (fusion domain).

As used herein, the term "fusion domain" refers to an additional domain or moiety that can be directly or indirectly linked to and incorporated in the chimeric protein or a variant thereof according to the present invention, and the fusion domain is used to mean both a polypeptide having a function and a polypeptide having no function.

In the present invention, the fusion domain may be fused to impart expression characteristics such as secretion from cells, expression on the cell surface, anchoring to a cell membrane, or intracellular localization; to add a substrate or other recognition sequence for post-translational modification; or to change tissue localization, tissue exclusion, or other ADME characteristics; or to add a protein or peptide having another function, but is not limited thereto.

In the present invention, the fusion domain may be, for example, a transmembrane domain, a hinge domain, an intracellular signaling domain, an immune regulatory domain, a localization domain, an immune co-stimulatory factor/-receptor, a cytokine, a growth factor, an albumin-binding domain, an Fc domain, a transferrin fusion domain, albumin, PEG, hyaluronic acid, or other therapeutic peptides, but is not limited thereto, and may include any domain (or moiety) that is not fatal to the expression or activity of the chimeric protein or variant thereof including the IL-2 and IL-15 proteins of the present invention.

In the present invention, in order to fix the fusion protein expressed in the modified natural killer cell on the cell membrane or to express the same on the cell surface, the fusion domain including a membrane protein or a transmembrane domain may be used.

In the present invention, "membrane protein" means a protein inserted into or attached to the surface of a membrane including a lipid bilayer, and includes "integral membrane protein" which is a protein inserted into a lipid bilayer, "peripheral membrane protein" which is a protein attached to the surface of a lipid bilayer and the like. An "integral membrane protein" is a type of "membrane protein" that crosses the membrane.

As used herein, the term "integral membrane protein" is a protein that penetrates the cell membrane, and includes a "transmembrane domain" that penetrates the membrane, an "intracellular domain" located inside the cell, and an "extracellular domain" exposed to the outside of the cell. The number of transmembrane domains varies depending on the membrane protein. Since the lipid bilayer is composed of highly hydrophobic lipids, most transmembrane domains that pass through this region are composed of hydrophobic amino acids. Almost all transmembrane domains are composed of an α-helix structure. In membrane proteins having multiple transmembrane domains, the transmembrane domains are arrayed in a circle to form a cylindrical structure. This cylindrical structure opens and closes in response to a specific signal and thus is used as a passage for transporting specific ions or biological substances. The transmembrane domains of an extremely limited number of membrane proteins are in a β-sheet structure. The cylindrical structure formed by arraying multiple domains in a β-sheet structure in a circle is called a "β-barrel."

In the present invention, in the case of "peripheral membrane protein", a lipoprotein is formed by covalent bonding between a protein molecule and a lipid molecule, and hydrophobic bonding is formed between the lipid molecule of the lipoprotein and the lipid monolayer of the cell membrane, thus causing the protein to be fixed to the surface of the cell membrane. The peripheral membrane protein is, for example, a GPI (glycosylphosphatidylinositol)-anchored protein. The fatty acid of GPI is inserted into the lipid monolayer and the phosphate group on the other side thereof is covalently linked to the protein, so that GPI fixes the protein to the surface of the lipid bilayer.

In the present invention, examples of the membrane protein include a receptor, a ligand, an immunoglobulin, a glycophorin, or a combination thereof. The membrane protein may be selected from the group consisting of IL-15 receptor alpha (IL-15Rα), CD8α, CD4, CD3ε, CD3γ, CD3δ, CD3ζ, CD28, CD137, FcεRIγ, a T-cell receptor (TCR, for example, TCRα and/or TCRβ), a nicotinic acetylcholine receptor, a GABA receptor, and fragments thereof, but not limited thereto. Specific examples of the immunoglobulin include IgG, IgA, IgM, IgE, IgD, and combinations thereof. Specific examples of the glycophorin include, but are not limited to, glycophorin A, glycophorin D, and combinations thereof.

In the present invention, the fusion protein may further include a hinge domain or a sushi domain in addition to the transmembrane domain. As used herein, the term "hinge domain" refers to a series of amino acid sequences present between the transmembrane domain and the extracellular domain of the membrane-anchored protein. In the present invention, the domains present in the fusion protein may be in the form of a combination of domains derived from the same protein, as well as in the form of a chimeric protein in which domains derived from different proteins are combined.

In one embodiment of the present invention, the fusion protein may include an IL-15 receptor alpha (IL-15Rα) protein or a fragment thereof, in addition to the IL-2 and IL-15 chimeric protein or a variant thereof according to the present invention.

In the present invention, the IL-15 receptor alpha (IL-15Rα) protein fragment may include a "sushi domain" which is the shortest region of a receptor having IL-15 binding activity.

In the present invention, the IL-15 receptor alpha (IL-15Rα) protein fragment may include a "sushi domain", which is the shortest region of a receptor having IL-15 binding activity, and a "transmembrane domain", which is a cell membrane permeable region of the IL-15 receptor alpha. In the present invention, the fusion protein may be a fusion protein in which an IL-15 receptor alpha (IL-15Rα) protein or a fragment thereof is fused to a chimeric protein or a variant thereof.

In the present invention, the sequences of human IL-15 receptor alpha (IL-15Rα) and fragments thereof are, for example, set forth in Table 5 below, but are not limited thereto.

[Table 5]

| | Amino acid or nucleic acid sequence | SEQ ID NO: |
|---|---|---|
| **IL15Rα Full-length** | ITCPPPMSVEHADIWVKSYSLYSRERYICNSGFKRKAGTSSLT ECVLNKATNVAHWTTPSLKCIRDPALVHQRPAPPSTVTTAGVT PQPESLSPSGKEPAASSPSSNNTAATTAAIVPGSQLMPSKSPS | 77 |

(continued)

| | Amino acid or nucleic acid sequence | SEQ ID NO: |
|---|---|---|
| | TGTTEISSHESSHGTPSQTTAKNWELTASASHQPPGVYPQGHS DTTVAISTSTVLLCGLSAVSLLACYLKSRQTPPLASVEMEAME ALPVTWGTSSRDEDLENCSHHL | |
| | ATTACTTGTCCACCACCAATGTCTGTTGAGCATGCTGATATTT GGGTGAAATCTTATTCTCTCTATAGCCGGGAACGCTATATCTG CAATTCAGGATTTAAAAGAAAGGCTGGTACCTCTTCCTTGACC GAATGTGTACTGAATAAAGCTACAAACGTGGCACATTGGACCA CGCCTAGCCTTAAGTGTATAAGGGACCCCGCATTAGTGCATCA GAGACCTGCACCCCCGAGCACCGTGACAACTGCCGGTGTCACT CCTCAACCTGAATCTCTTTCTCCCAGCGGTAAGGAACCAGCTG CCAGTAGTCCGTCAAGTAATAATACCGCAGCTACTACTGCCGC CATCGTTCCCGGGTCTCAACTCATGCCAAGCAAGAGCCCCTCA ACCGGGACAACCGAAATTTCCTCTCACGAAAGTTCTCATGGGA CTCCAAGCCAAACCACCGCTAAGAATTGGGAGTTGACCGCCTC AGCAAGTCATCAACCCCCAGGGGTATACCCCCAAGGGCATTCC GATACTACCGTTGCAATTAGCACTTCTACGGTGTTACTTTGCG GATTATCTGCAGTATCCCTGTTGGCCTGTTATCTTAAAAGCCG TCAGACACCGCCACTCGCTTCCGTCGAGATGGAAGCTATGGAA GCCCTTCCCGTAACCTGGGGCACTAGTTCCCGCGACGAGGATC TGGAGAATTGTAGTCATCATTTG | 78 |
| IL15Rα membrane protein domain | VAISTSTVLLCGLSAVSLLACYLKSRQTPPLASVEMEAMEALP VTWGTSSRDEDLENCSHHL | 79 |
| | GTTGCAATTAGCACTTCTACGGTGTTACTTTGCGGATTATCTG CAGTATCCCTGTTGGCCTGTTATCTTAAAAGCCGTCAGACACC GCCACTCGCTTCCGTCGAGATGGAAGCTATGGAAGCCCTTCCC GTAACCTGGGGCACTAGTTCCCGCGACGAGGATCTGGAGAATT GTAGTCATCATTTG | 80 |
| IL15Rα/sushi | ITCPPPMSVEHADIWVKSYSLYSRERYICNSGFKRKAGTSSLT ECVLNKATNVAHWTTPSLKCIR | 81 |
| | ATTACATGCCCTCCTCCAATGTCCGTGGAACACGCCGACATCT GGGTCAAGTCCTACAGCCTGTACTCCAGAGAGCGGTACATCTG CAACTCCGGCTTCAAGAGAAAGGCCGGCACCTCTAGCCTGACC GAGTGCGTGCTGAACAAGGCCACCAATGTGGCCCACTGGACAA CCCCTAGCCTGAAGTGTATTAGA | 82 |

In the present invention, the chimeric protein or a variant thereof may be directly or indirectly linked to the IL-15 receptor alpha (IL-15Rα) protein, for example, via a linker. In one embodiment of the present invention, the fusion protein may include the structure of the following Formula (I) or Formula (II):

N'-X-[L1]$_n$-Y-C'            (Formula (I))

N'-Y-[L1]$_n$-X-C'            (Formula (II))

wherein N' is the N-terminus of the fusion protein and C' is the C-terminus of the fusion protein,
X is a chimeric protein including IL-2 and IL-15 proteins or a variant thereof,
Y is an IL-15 receptor alpha (IL-15Rα) protein or a fragment thereof,

L1 is a linker, and
n is an integer of 0 or greater, preferably 0 or 1.

In the present invention, the fragment of the IL-15Rα protein may include at least one of the IL15Rα membrane protein domain and the IL15Rα sushi domain. For example, the IL15Rα membrane protein domain may include or consist of an amino acid sequence represented by SEQ ID NO: 79, and the IL15Rα sushi domain may include or consist of an amino acid sequence represented by SEQ ID NO: 81, but is not limited thereto.

In the present invention, the linker may be any one of various linkers known in the art. For example, the linker may include, but is not limited to, a hinge domain between the transmembrane domain and the extracellular domain, a flexible linker, a rigid linker, or the like.

More specifically, the linker means an amino acid sequence having a sufficient length to allow the protein to form appropriate secondary and tertiary structures. In some embodiments, the linker is a peptide linker including at least one, but less than 100 amino acids, for example a peptide linker of 2 to 60 amino acids, preferably 10 to 40 amino acids, more preferably 15 to 40 amino acids, even more preferably 19 to 30 amino acids, most preferably 20 to 26 amino acids. In some embodiments, the linker has 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acid residues.

In the present invention, the linker is a CD4-derived hinge, a CD8α-derived hinge, a CD28-derived hinge, a CD34-derived hinge, an IgG1-derived hinge, an IgG2-derived hinge, an IgG3-derived hinge, an IgG4-derived hinge, PLrigid, a 2aa GS linker, a 6aa [GS]x linker, a 10aa [GS]x linker, a 10 aa flexible protein domain linker, a 8 aa protein domain linker, a flexible linker 2x (GGGS), a flexible linker 2x (GGGGS), a 13 amino acid linker [GGGS GGGGS GGGS], a split fluorophore linker, Freiburg standard, a 15 aa flexible glycine-serine protein domain linker, a Freiburg standard, a short linker (Gly-Gly-Ser-Gly), a middle linker (Gly-Gly-Ser-Gly)x2, a long linker (Gly-Gly-Ser-Gly)x3, a glycine linker, a (HL5)2 peptide helical linker, rigid; separate the domains of fusion proteins, glycine-serine linker (GSGGS), a glycine-serine linker (GSSGS), a (G2S)3 linker, a SEG-Linker, a GSAT-Linker, a Z-EGFR-1907_short-linker, a Z-EGFR-1907_middle-linker, a Z-EGFR-1907_SEG-linker, a (Gly4Ser)3 flexible peptide linker or an (SSSSG)x2 serine glycine linker, or a Whitlow 218 linker (GSTGSGSKPGSGEGSTKG), but is not limited thereto.

In the present invention, the linker may be any linker that 1) can perform additional functions of improving biological activity, increasing expression yield, and improving pharmacokinetic profile, 2) does not negatively affect the expression, secretion, or functional activity of each domain of the fusion protein, and 3) does not exhibit immunogenicity, in addition to the fusion of the chimeric protein or a variant thereof according to the present invention with a functional domain.

In the present invention, preferably, the linker is QSFGLLDPK (CD3-derived hinge amino acid sequence, SEQ ID NO: 83) or a variant thereof, LSEGDKVKMDSRIQVLSRGVNQT (CD4-derived hinge amino acid sequence, SEQ ID NO: 84) or a variant thereof, KPTTPAPRPPTPAPTIASQPLSLRPEACRPAAGGAVHTRGLDFACDIY (CD8α-derived hinge amino acid sequence, SEQ ID NO: 85) or a variant thereof, IEVMYPPPYLDNERSNGTIIHVKGKHLCPSPLFPGPSKP (CD28-derived hinge amino acid sequence, SEQ ID NO: 86) or a variant thereof, ELPTQGTFSNVSTNVS (CD34-derived hinge amino acid sequence, SEQ ID NO: 87) or a variant thereof, (GGGGS)n (wherein n is an integer greater than or equal to 1, SEQ ID NO: 88), or GSTGSGSKPGSGEGSTKG (Whitlow 218 linker, SEQ ID NO: 89), but is not limited thereto.

In the present invention, preferably, the linker may include a sequence represented by SEQ ID NO: 87, 88 or 89, but is not limited thereto.

In the present invention, the fusion protein may include an Fc domain of an immunoglobulin.

The Fc domain of the immunoglobulin may include a heavy chain constant region 2 (CH2) and a heavy chain constant region 3 (CH3) of the immunoglobulin. The Fc domain of the immunoglobulin may not include a variable region of the heavy and light chains of the immunoglobulin and a light chain constant region 1 (CH1). The immunoglobulin may be IgG, IgA, IgE, IgD or IgM, and preferably IgG4.

In addition, the Fc domain of the immunoglobulin may be not only a wild-type Fc domain but also an Fc domain variant. In addition, as used herein, the term "Fc domain variant" may have a different glycosylation pattern from that of a wild-type Fc domain, or may have increased glycosylation pattern compared to a wild-type Fc domain, decreased glycosylation compared to a wild-type Fc domain, or may have a deglycosylated form. The Fc domain of the immunoglobulin may include an aglycosylated Fc domain. The Fc domain or variant may have sialic acid, fucosylation, or glycosylation with contents controlled by culture conditions or genetic manipulation of the host.

In addition, the sugar chain of the Fc domain of the immunoglobulin may be modified by conventional methods such as chemical methods, enzymatic methods, and genetic engineering methods using microorganisms. In addition, the Fc domain variant may be mixed with the Fc region of immunoglobulin IgG, IgA, IgE, IgD, or IgM.

In the present invention, when the fusion protein includes an Fc domain, the chimeric protein and/or variant thereof according to the present invention may be linked to the N'-terminus and/or the C'-terminus of the Fc domain, and another fusion domain may be linked to a terminal other than the terminal to which the chimeric protein or variant thereof is linked.

In the present invention, for example, the fusion protein may include an IL-2/IL-15 chimeric protein or a variant thereof, or a sushi domain of IL-15 receptor alpha.

For example, the fusion protein may have the structure of the following Formula (III) or Formula (IV).

In an embodiment, the fusion protein may have the structure of the following Formula (III) or Formula (IV):

$$\text{N'-X-[L1]}_l\text{-Y-[L2]}_n\text{-[H]}_m\text{-Fc domain-C'} \qquad \text{(Formula (III))}$$

$$\text{N'-[H]}_m\text{-Fc domain-[L1]}_1\text{-Y-[L2]}_n\text{-X-C'} \qquad \text{(Formula (IV))}$$

wherein

N' is the N-terminus of the fusion protein and C' is the C-terminus of the fusion protein;
X is a chimeric protein including IL-2 and IL-15 proteins or a variant thereof;
Y is an IL-15 receptor alpha (IL-15Rα) protein or a fragment thereof;
L1 and L2 are linkers;
H is an Fc hinge domain of an immunoglobulin; and
l, n, and m are each independently an integer of 0 or greater, preferably 0 or 1.

In the present invention, the Fc hinge domain may be a hinge domain derived from IgG, IgA, IgE, IGD or IgM, preferably a hinge domain of IgG4, but is not limited thereto.

In the present invention, for example, when the fusion protein is to be presented on the cell membrane surface, a membrane protein or a transmembrane domain may be additionally included.

In the present invention, the IL-15Rα protein fragment may include at least one of the IL-15Rα membrane protein domain and the IL-15Rα sushi domain.

In the present invention, the fusion protein may further include an immune regulatory domain or an intracellular signaling domain.

In the present invention, the immune regulatory domain or the intracellular signaling domain is a domain located in the cytoplasmic direction of the membrane anchored protein, and refers to a site that activates or suppresses an immune response when a target antigen binds to the extracellular domain.

In the present invention, the immune regulatory domain or the intracellular signaling domain may be an intracellular signaling domain that is capable of activating natural killer cells to an appropriate level, and is, for example, an intracellular signaling domain derived from IL-15Rα, CD3, CD28, CD40L, ICOS, OX40, 4-1BB, TNFR2 DAP10, 2B4, CD3ζ, CD27, CD28, 4-1BB, OX40, CD30, CD40, PD-1, ICOS, LFA-1 (lymphocyte function-associated antigen-1), CD2, CD7, LIGHT, NKG2C, and/or B7-H3, but is not limited thereto.

In the present invention, the fusion protein may be multimerized into dimers, trimers, or high multimers depending on the linker, fusion domain, or the like. In one embodiment of the present invention, the fusion protein was used in the form of a dimer through the hinge region of the fusion protein including the Fc domain, but is not limited thereto.

As in the embodiment of the present invention, the fusion protein may include or consist of an amino acid sequence selected from, for example, amino acid sequences represented by SEQ ID NOS: 90 to 133, but is not limited thereto.

As in the embodiment of the present invention, the fusion protein may include or consist of an amino acid sequence of SEQ ID NO: 185, but is not limited thereto.

**Nucleic acids and recombinant vectors**

In another aspect, the present invention is related to a nucleic acid encoding the chimeric protein or variant thereof, or the fusion protein.

As used herein, the "nucleic acid" may be present in cells, in the cell lysate, or in the partially purified or substantially pure form. "Isolated" or "substantially pure", when referring to nucleic acids, refer to those that have been purified and thus separated from other cellular components or other contaminants, e.g., other cellular nucleic acids or proteins, by standard techniques, including alkaline/SDS treatment, CsCl banding, column chromatography, agarose gel electrophoresis, and others well known in the art. The nucleic acid of the present invention may be DNA or RNA.

In the present invention, the nucleic acid encoding the chimeric protein may include or consist of the nucleic acid sequence of SEQ ID NO: 34.

In the present invention, the nucleic acid encoding the variant of the chimeric protein may include or consist of the nucleic acid sequence of SEQ ID NO: 36, 38, 40, 42, 44, 46, 48, 50, 52, 54, 56, 58, 60, 62, 64, 66, 68, 70, 72 or 74.

In the present invention, the sequence of the nucleic acid encoding the fusion protein may be easily designed based on the amino acid sequence of the linker or fusion domain further included in addition to the amino acid sequence of the chimeric protein or the variant protein thereof according to the present invention, or the nucleic acid sequence encoding the same.

In the present invention, the nucleic acid encoding the fusion protein may include or consist of a nucleic acid sequence selected from SEQ ID NOS: 135 to 178, but is not limited thereto.

In the present invention, the nucleic acid encoding the fusion protein may include or consist of a nucleic acid sequence of SEQ ID NO: 186, but is not limited thereto.

In another aspect, the present invention is related to a recombinant vector containing the nucleic acid of the present invention.

In the present invention, the recombinant vector may be selected from vectors known in the art without limitation as long as it is capable of inducing protein expression of the nucleic acid encoding the peptide. For example, when E. *coli* is used as a host, vectors including T7 series (T7A1, T7A2, T7A3, etc.), lac, lacUV5, temperature-dependent (vectors including λphoA, phoB, rmB, tac, trc, trp or 1PL promoters) may be used. When yeast is used as a host, a vector including the ADH1, AOX1, GAL1, GAL10, PGK or TDH3 promoter may be used, and when Bacillus is used as a host, a vector including the P2 promoter may be used. These are provided only as representative embodiments and, in addition to the vectors including the promoters, any vector may be appropriately selected from various vectors known in the art by those skilled in the art without any limitation so long as it is suitable for a host as a vector including a promoter for inducing the expression of the interferon lambda variant according to the present invention.

As used herein, the term "vector" means a DNA product containing a DNA sequence operably linked to a suitable regulatory sequence capable of expressing the DNA in a suitable host. Vectors may be plasmids, phage particles or simply potential genomic inserts. When transformed into a suitable host, vectors may be replicated or perform functions independent of the host genomes, or some thereof may be integrated with the genomes. A plasmid is currently the most commonly used form of vector and thus the terms "plasmid" and "vector" are often used interchangeably. However, the present invention encompasses other forms of vectors that are known in the art or have the same functions as those known in the art. Protein expression vectors used in E. *coli* include: the pET family vectors from Novagen, Inc (USA); the pBAD family vectors from Invitrogen Corp. (USA); PHCE or pCOLD vectors from Takara Bio Inc. (Japan); and pACE family vectors from GenoFocus Inc. (South Korea). In *Bacillus subtilis,* a gene of interest may be inserted into a specific part of the genome to realize protein expression, or a pHT-family vector of MoBiTech (Germany) may be used. Even in fungi and yeast, protein expression is possible using genome insertion or self-replicating vectors. A plant protein expression vector using a T-DNA system such as *Agrobacterium tumefaciens* or *Agrobacterium rhizogenes* may be used. Typical expression vectors for expression in mammalian cell cultures are based on, for example, pRK5 (EP 307,247), pSV16B (WO 91/08291), and pVL1392 (Pharmingen).

As used herein, the term "expression control sequence" means a DNA sequence essential for the expression of a coding sequence operably linked to a particular host organism. Such a control sequence includes promoters for conducting transcription, operator sequences for controlling such transcription, sequences for encoding suitable mRNA ribosome-binding sites, and sequences for controlling the termination of transcription and translation. For example, control sequences suitable for prokaryotes include promoters, optionally operator sequences, and ribosome-binding sites. Control sequences suitable for eukaryotic cells include promoters, polyadenylation signals, and enhancers. The factor that has the greatest impact on the expression level of a gene in a plasmid is the promoter. SRα promoters, *cytomegalovirus-derived* promoters and the like are preferably used as promoters for high expression.

Any of a wide variety of expression control sequences may be used for the vector in order to express the DNA sequences of the present invention. Useful expression control sequences include, for example, early and late promoters of SV40 or adenovirus, the lac system, the trp system, the TAC or TRC system, T3 and T7 promoters, the major operator and promoter regions of phage lambda, control regions of fd code proteins, promoters of 3-phosphoglycerate kinase or other glycol lyases, promoters of the phosphatase, such as Pho5, promoters of yeast alpha-mating systems, and other sequences having configurations and induction activity known to control gene expression of prokaryotic or eukaryotic cells or viruses and various combinations thereof. The T7 RNA polymerase promoter Φ10 may be useful for expressing proteins in E. *coli.*

When a nucleic acid sequence is aligned with another nucleic acid sequence based on a functional relationship, it is "operably linked" thereto. This may be gene(s) and control sequence(s) linked in such a way so as to enable gene expression when a suitable molecule (*e.g.,* a transcriptional activator protein) is linked to the control sequence(s). For example, DNA for a pre-sequence or secretory leader is operably linked to DNA for a polypeptide when expressed as a preprotein involved in the secretion of the polypeptide; a promoter or enhancer is operably linked to a coding sequence when it affects the transcription of the sequence; or a ribosome-binding site is operably linked to a coding sequence when it affects the transcription of the sequence; or the ribosome-binding site is operably linked to a coding sequence when positioned to facilitate translation. Generally, the term "operably linked" means that the linked DNA sequence is in contact therewith, and that a secretory leader is in contact therewith and is present in the reading frame. However, the enhancer need not be in contact therewith. The linkage of these sequences is carried out by ligation (linkage) at convenient restriction enzyme sites. When no such site exists, a synthetic oligonucleotide adapter or a linker according to a conventional method is used.

As used herein, the term "expression vector" commonly refers to a recombinant carrier into which a fragment of heterologous DNA is inserted, and generally means a fragment of double-stranded DNA. Here, "heterologous DNA" means xenogenous DNA that is not naturally found in the host cell. Once an expression vector is present in a host cell, it can replicate independently of the host chromosomal DNA, and several copies of the vector and inserted (heterologous) DNA thereof can be produced.

As is well known in the art, in order to increase the expression level of a transfected gene in a recombinant cell, the gene should be operably linked to a transcriptional or translational expression control sequence that functions in the selected expression host. Preferably, the expression control sequence and the corresponding gene are included in a single expression vector containing both a bacterial selection marker and a replication origin. When the expression host is a eukaryotic cell, the expression vector should further include a useful expression marker in the eukaryotic expression host.

**Genetically modified cells expressing chimeric proteins including IL-2 and IL-15 proteins, variants thereof, and/or fusion proteins including the same**

In another aspect, the present invention is related to genetically modified cells expressing the chimeric protein, variant thereof, and/or fusion protein including the same according to the present invention.

In the present invention, the genetically modified cells may be produced by any method without limitation as long as the method is modified to express the chimeric protein, variant thereof, and/or fusion protein.

In the present invention, preferably, the modified natural killer cells may be produced by introducing a nucleic acid encoding the chimeric protein, variant thereof, and/or fusion protein or a recombinant vector containing the same into the host cells.

In the present invention, the host cells may mean cells for expression, into which a gene, recombinant vector or the like has been introduced in order to produce proteins. The host cells may be used without limitation as long as they are cells capable of expressing the chimeric protein or variant thereof, or the fusion protein, and is preferably eukaryotic cells, more preferably yeast cells, insect cells, or animal cells, and most preferably animal cells. For example, the host cells may be a CHO cell line or a HEK cell line, which are mainly used for expressing fusion proteins, but is not limited thereto.

A wide variety of expression host/vector combinations can be used to express the chimeric protein or the variant thereof, or the fusion protein. Suitable expression vectors for eukaryotic hosts include, for example, expression control sequences derived from SV40, cow papillomavirus, adenovirus, adeno-associated virus, cytomegalovirus and retrovirus. Expression vectors that may be used for bacterial hosts include bacterial plasmids, exemplified by those obtained from *E. coli,* such as pBlueScript, pGEX2T, pUC vectors, col E1, pCR1, pBR322, pMB9 and derivatives thereof, plasmids having a wide host range such as RP4, phage DNA exemplified by a wide variety of phage lambda derivatives such as Agt10, λgt11 and NM989, and other DNA phages such as M13 and filamentous single-stranded DNA phages. Expression vectors useful for yeast cells include 2μ plasmids and derivatives thereof. The vector useful for insect cells is pVL 941.

The recombinant vector may be introduced into a host cell through a method such as transformation or transfection. As used herein, the term "transformation" means introducing DNA into a host and making the DNA replicable using an extrachromosomal factor or chromosomal integration. As used herein, the term "transfection" means that an expression vector is accommodated by the host cell, regardless of whether or not any coding sequence is actually expressed.

It should be understood that not all vectors and expression control sequences function identically in expressing the DNA sequences of the present invention. Similarly, not all hosts function identically in the same expression system. However, those skilled in the art will be able to make appropriate selection from a variety of vectors, expression control sequences and hosts without excessive burden of experimentation without departing from the scope of the present invention. For example, selection of a vector should be carried out in consideration of the host, because the vector should be replicated therein. The number of replications of the vector, the ability to control the number of replications, and the expression of other proteins encoded by the corresponding vector, such as the expression of antibiotic markers, should also be considered. In selecting the expression control sequence, a number of factors should be considered. For example, the relative strength of the sequence, controllability, and compatibility with the DNA sequences of the present invention should be considered, particularly in relation to possible secondary structures. A single-celled host may be selected in consideration of factors such as the selected vector, the toxicity of the product encoded by the DNA sequence of the present invention, secretion characteristics, the ability to accurately fold proteins, culture and fermentation factors, and ease of purification of the product encoded by the DNA sequence according to the present invention from the host. Within the scope of these factors, those skilled in the art can select various vector/expression control sequences/host combinations capable of expressing the DNA sequences of the present invention in fermentation or mass animal cultures. As a screening method for cloning cDNA of proteins through expression cloning, a binding method, a panning method, a film emulsion method or the like may be applied.

The gene and recombinant vector may be introduced into host cells through various methods known in the art. The chimeric protein or the variant thereof, or the fusion protein according to the present invention may be directly introduced into the genome of a host cell and present as a factor on a chromosome. It will be apparent to those skilled in the art to which the present invention pertains that even if the gene is inserted into the genomic chromosome of the host cell, it will have the same effect as when the recombinant vector is introduced into the host cell.

**Method of producing chimeric protein including IL-2 and IL-15 proteins, or variant thereof, and/or fusion protein containing the same**

In another aspect, the present invention is related to a method of producing the chimeric protein or variant thereof, and/or the fusion protein.

The chimeric protein or variant thereof, and/or the fusion protein according to the present invention may be produced by various protein production methods known in the art.

In the present invention, the chimeric protein or variant thereof, and/or the fusion protein may be produced using the genetically modified cell.

For example, the production method includes: culturing the genetically modified cell to express the chimeric protein or a variant thereof, and/or the fusion protein; and recovering the expressed chimeric protein or variant thereof, and/or the fusion protein.

In the present invention, the chimeric protein or variant thereof, the protein complex, or the fusion protein may also be produced in a cell-free protein synthesis system.

The cell-free protein synthesis system includes adding substrates or enzymes to cell lysates or extracts, and synthesizing proteins in a test tube using major elements (ATP (adenosine triphosphate), amino acids, and the like) necessary for protein synthesis. The cell-free protein synthesis system can advantageously overcome the drawbacks of conventional protein production methods using cells and cell-free protein synthesis allows protein synthesis at a high rate without any cell culture by extracting only the intracellular machinery and factors thereof related to protein production from cells, and artificially repeating only the protein synthesis process while excluding the physiological control mechanism of the cells outside the cells, to mass-produce the target proteins within a short time, and is a completely open system with no physical barriers, compared to the cell culture process in which proteins are expressed in the space of the cell membrane and cell wall and allows the conditions of protein synthesis to be freely changed for application to various studies. In addition, the intracellular protein synthesis system has major problems in terms of the production and yield when the protein produced is cytotoxic, whereas, when the cell-free protein synthesis system is used, peptides or proteins can be produced without these problems.

In the present invention, in addition to the examples of the host cell for protein expression and production, immune cells, and the like may be used as host cells and modified to express the chimeric protein, variant thereof and/or the fusion protein containing the same according to the present invention. In such cases, the function or characteristics of the host cells (e.g., immune cells) may be modified or improved by expression of the chimeric protein, the variant thereof and/or the fusion protein containing the same according to the present invention.

In particular, in one embodiment of the present invention, in order to develop genetically modified natural killer cells that maintain appropriate levels of cell activity and cell survival ability for a longer period of time than naïve or previously reported modified natural killer cells, it was found that, when a chimeric protein including IL-2 and IL-15 proteins is expressed in natural killer cells, it has a higher expression yield than that of other proteins, suppresses side effects caused by excessive immune cell activity of IL-2, prevents rapid exhaustion of natural killer cells, and maintains significantly superior levels of cell activity and cell survival ability for a significantly longer period of time, and it was found that the genetically modified natural killer cells exhibit excellent cancer cell killing ability without side effects such as pulmonary edema.

Accordingly, in another aspect, the present invention is related to a modified immune cell expressing the chimeric protein including IL-2 and IL-15 proteins, the variant thereof and/or the fusion protein containing the same.

**Modified immune cell expressing chimeric protein including IL-2 and IL-15 proteins, variant thereof and/or fusion protein containing the same**

As used herein, the term "genetically modified immune cell expressing the chimeric protein including IL-2 and IL-15 proteins, the variant thereof, and/or the fusion protein containing the same" refers to genetically modified immune cells expressing the chimeric protein, the variant thereof, or the fusion protein containing the same according to the present invention.

The immune cell according to the present invention may be genetically modified to express the chimeric protein, the variant thereof, and/or the fusion protein.

In the present invention, the immune cell is preferably an animal-derived immune cell, more preferably a human-derived immune cell, and may include, but is not limited to, B cells, T cells (e.g., cytotoxic T cells, effector T cells, and helper T cells), natural killer cells (NK cells), NKT cells, dendritic cells, as well as cells that can differentiate into immune cells, such as hematopoietic stem cells, iPSCs, and adult stem cells.

In the present invention, the immune cells are most preferably natural killer cells.

In the present invention, "natural killer cells" ("NK cells") refers to a type of cytotoxic lymphocytes of the immune system. NK cells provide a rapid response to virus-infected cells and respond to transformed cells. Typically, immune cells detect peptides from pathogens provided by major histocompatibility complex (MHC) molecules on the surface of infected cells, thus triggering cytokine release and causing cell lysis or cell death. However, NK cells are unique in that they have the ability to recognize stressed cells regardless of whether peptides from the pathogens are present on the MHC molecule. The cells were termed "natural killers" based on the original concept that they do not require prior activation to kill the targets thereof. NK cells are known to be large granular lymphocytes (LGL) that differentiate and mature in the bone marrow and then enter the circulatory system from the bone marrow.

In some embodiments, the immune cells are mammalian immune cells. Examples of "mammalian" or "mammal" include primates (e.g., humans), canines, felines, rodents, pigs, ruminants, and the like. Specific examples include humans, dogs, cats, horses, cows, sheep, goats, rabbits, guinea pigs, rats, and mice. In certain embodiments, the mammalian immune cells are human-derived immune cells.

In the present invention, the chimeric protein, the variant, and/or the fusion protein expressed in the immune cells may be secreted extracellularly, expressed on the cell membrane, and/or expressed on the cell surface.

In the present invention, a signal sequence may be included for transport of the chimeric protein, the variant thereof, and/or the fusion protein.

In the present invention, the signal sequence (signal peptide or leader sequence) may be located at the N-terminus of the fusion protein. The signal sequence functions to enable the fusion protein to move to a desired location. For example, the signal sequence may be transported to a transport channel such as the endoplasmic reticulum or a translocon, and then cleaved at the residue in the signal sequence, without being limited thereto.

In the present invention, the signal sequence (signal peptide or leader sequence) may be located at the N-terminus of the fusion protein. The signal sequence functions to enable the fusion protein to move to a desired location. For example, the signal sequence may be transported to a transport channel such as the endoplasmic reticulum or a translocon, and then may be cleaved at a residue in the signal sequence, but is not limited thereto.

In the present invention, the chimeric protein, the variant thereof, and/or the fusion protein are more preferably fixed to the cell membrane of immune cells or expressed on the cell surface.

In the present invention, the chimeric protein, the variant thereof and/or fusion protein may be directly or indirectly (e.g., via ionic, non-ionic, covalent bond) bound (conjugated; fused) to the surface of the immune cells (e.g., on the surface of the immune cells or within the membrane of the cell) using any of various linkers known in the art (see literature [Hermanson, G., Bioconjugate Techniques, Academic Press 1996]).

In the present invention, the fusion protein is preferably a fusion protein having a structure of Formula (I) or Formula (II), as can be seen from Examples, but is not limited thereto.

In the present invention, the fusion protein may be presented on the cell membrane of immune cells, but is not limited thereto.

The genetically modified immune cell of the present invention can maintain an appropriate level of immune cell activity and cell survival ability for a long time, and thus a cell immunotherapy agent using the modified immune cells may be used for the prevention or treatment of various diseases.

Therefore, in another aspect, the present invention relates to the use of the genetically modified immune cell for the prevention or treatment of a disease.

The present invention relates to a pharmaceutical composition for preventing or treating a disease containing the genetically modified immune cell.

The present invention relates to a method of preventing or treating a disease including administering the genetically modified immune cell.

The present invention relates to the use of the genetically modified immune cell for the preparation of a drug for preventing or treating a disease.

In the present invention, any disease may be used without limitation as long it is reported to be prevented or treated using immune cells, preferably natural killer cells, and examples thereof include, but are not limited to, cancer, infectious diseases, and autoimmune diseases.

Herein, "cancer" has the same meaning as "tumor", and the composition for preventing or treating a disease according to the present invention may be applied to all types of cancer, including solid cancer and blood cancer. Unlike blood cancer, solid cancer refers to cancer that is formed in a lump in an organ and cancer that occurs in most organs. In the present invention, cancer may be selected from the group consisting of colon cancer, melanoma, stomach cancer, liver cancer, lung cancer, breast cancer, prostate cancer, ovarian cancer, pancreatic cancer, cervical cancer, bladder cancer, kidney cancer, gallbladder cancer, thyroid cancer, laryngeal cancer, acute myeloid leukemia, brain tumor, neuroblastoma, retinoblastoma, head and neck cancer, salivary gland cancer, and lymphoma.

The pharmaceutical composition for preventing or treating a disease according to the present invention may contain a pharmaceutically effective amount of the genetically modified immune cell alone or may further contain at least one pharmaceutically acceptable carrier, excipient or diluent, in addition to the compound. The term "pharmaceutically effective amount" refers to an amount sufficient to prevent, ameliorate, and treat symptoms of a disease.

The term "pharmaceutically acceptable", as used herein, means being physiologically acceptable without causing ordinary allergic reactions such as gastrointestinal disorders or dizziness or similar reactions thereto when administered to humans. Examples of the carrier, excipient and diluent include lactose, dextrose, sucrose, sorbitol, mannitol, xylitol, erythritol, maltitol, starch, gum acacia, alginate, gelatin, calcium phosphate, calcium silicate, cellulose, methyl cellulose, polyvinylpyrrolidone, water, methylhydroxybenzoate, propylhydroxybenzoate, talc, magnesium stearate, and mineral oils. In addition, the composition may further contain fillers, anti-aggregating agents, lubricants, wetting agents, flavoring agents, emulsifying agents, and preservatives.

The compositions of the present invention may be formulated using methods known in the art to provide rapid, sustained or delayed release of the active ingredient after administration to mammals. The formulation may be in the form of a powder, granule, tablet, emulsion, syrup, aerosol, soft or hard gelatin capsule, sterile injectable solution, or sterile powder.

In addition, the composition according to the present invention may be administered through any of various administration routes, including intravenous, transdermal, subcutaneous, intramuscular or oral administration, the dose of the active ingredient may be appropriately selected according to various factors such as the route of administration, the patient's age, gender and weight, and the severity of the disease of the patient, and the composition for preventing or treating a disease according to the present invention may be administered in conjunction with a known therapeutic agent or compound having an effect of preventing, ameliorating or treating the target disease.

In the present invention, specific amino acid sequences and nucleotide sequences have been described, but it will be apparent to those skilled in the art that the amino acid sequences substantially identical to the enzymes to be implemented in the present invention and the nucleotide sequences encoding the same fall within the scope of the present invention. "Substantially identical" includes the case in which the homology of an amino acid or a nucleotide sequence is very high, and means a protein that shares structural features regardless of sequence homology or has the same function as used in the present invention. A protein from which a sequence other than the sequence constituting the subject matter of the present invention is partially deleted or a fragment of a nucleotide sequence encoding the same may fall within the scope of the present invention. Therefore, the present invention includes all amino acid or nucleotide sequences having the same function as used in the present invention regardless of the length of the fragment.

## Examples

Hereinafter, the present invention will be described in more detail with reference to the following examples. However, it will be obvious to those skilled in the art that the following examples are provided only for illustration of the present invention, and should not be construed as limiting the scope of the present invention.

**Example 1. Selection of IL-2/IL-15 fusion protein candidates with reduced binding affinity to IL-2R (IL-2Rβ) compared to IL-2, Fc-IL-2**

Example 1-1: Production of fusion protein including IL-2/IL-15 chimeric protein, IL-15Rα, and Fc domain

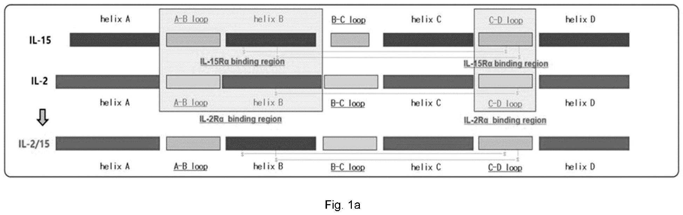

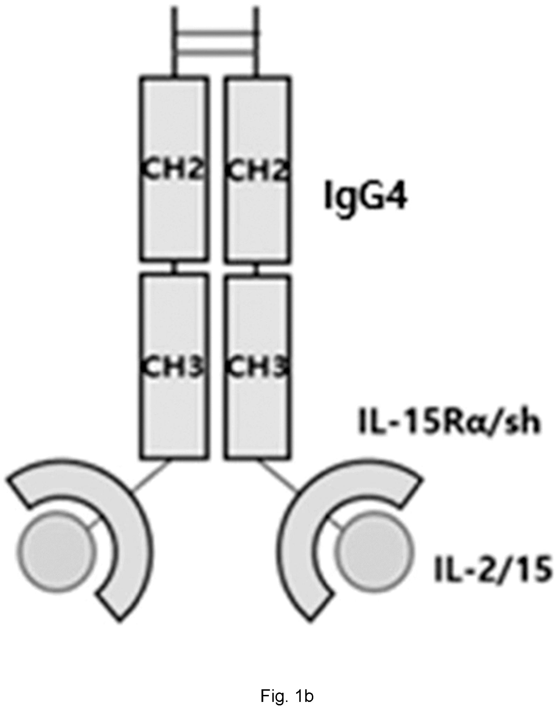

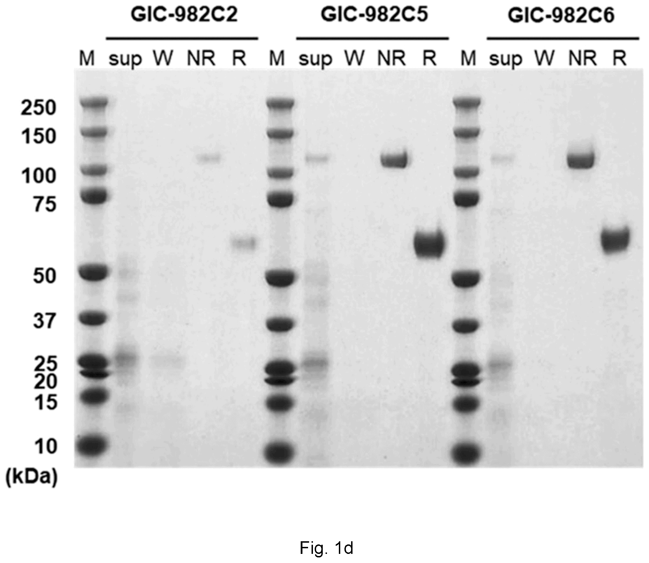

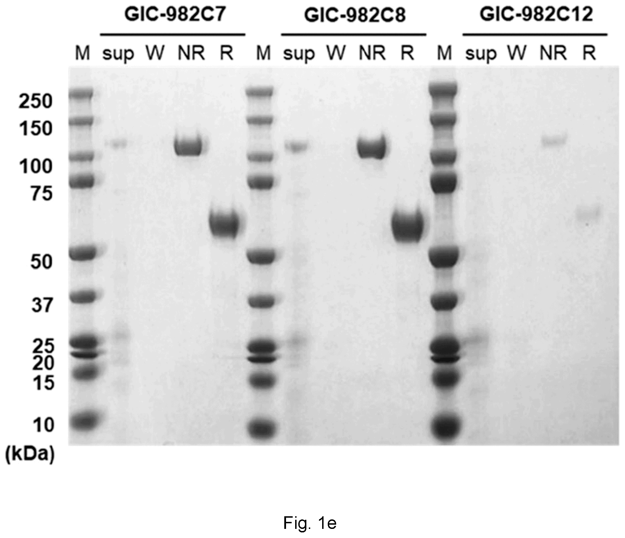

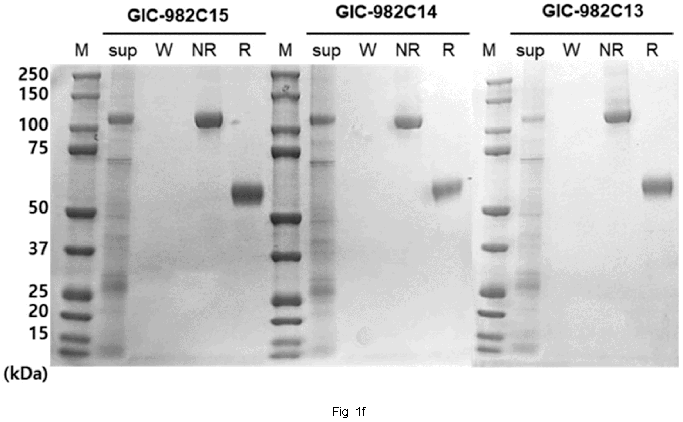

In order to produce a fusion protein including a chimeric protein or a variant thereof including an Fc domain, an IL-15Rα (sushi domain), and IL-2/IL-15, the nucleic acid of SEQ ID NO: 34, 36, 38, 40, 42, 44, 46, 48, 50, 52, 54, 56, 58, 60, 62, 64, 66, 68, 70, 72 or 74 encoding an amino acid sequence of SEQ ID NO: 33, 35, 37, 39, 41, 43, 45, 47, 49, 51, 53, 55, 57, 59, 61, 63, 65, 67, 69, 71, 73, or 75 was synthesized through the gBlock gene fragments service of Integrated DNA Technologies and then cloned into the pcDNA3.4 vector (FIG. 1B).

The produced fusion protein has the following formula:

N'-[hinge domain]-[Fc domain]-[linker]-[IL15Rα/sh]-[linker]-[IL-2/IL-15 chimeric protein or variant thereof]-C'; or
N'-[IL-2/IL-15 chimeric protein or its variant]-[linker]-[IL15Rα/sh]-[linker]-[hinge domain]-[Fc domain]-C'

The produced vectors were introduced into CHO cells (Expi-CHOTM) to express each fusion protein. After introducing the vectors, the culture was collected for 5 days in an environment of 37°C, 125 RPM, and $CO_2$ concentration of 8%, and the culture solution was purified. The names of the purified fusion proteins, the IL-2/IL-15 chimeric proteins or variants thereof contained therein, and the amino acid and nucleic acid sequence numbers thereof are as shown in Tables 6 and 7 below:

[Table 6]

| Fusion protein (IL-2/IL-15 variant) (variation ) | Amino acid sequence | SEQ ID NO: |
|---|---|---|
| GIC-982C1 (IL-2/IL-15) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY KTTPPVLDSDGSFFLYSRLTVDKSRWQEGNVFSCSVLHEALHNH YTQKSLSLSLGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PRDLISNINVIVLELKGSNGNVTESGCKECEELEEKNIVEFLNR WITFCQSIISTLT | 90 |
| GIC-982C2 (IL-2/IL-15v1) (D73E) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY KTTPPVLDSDGSFFLYSRLTVDKSRWQEGNVFSCSVLHEALHNH YTQKSLSLSLGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PRELISNINVIVLELKGSNGNVTESGCKECEELEEKNIVEFLNR WITFCQSIISTLT | 91 |
| GIC-982C3 (IL-2/IL-15v2) (L74F) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY KTTPPVLDSDGSFFLYSRLTVDKSRWQEGNVFSCSVLHEALHNH YTQKSLSLSLGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PRDFISNINVIVLELKGSNGNVTESGCKECEELEEKNIVEFLNR WITFCQSIISTLT | 92 |
| GIC-982C4 (IL-2/IL-15v3) (S76D) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY KTTPPVLDSDGSFFLYSRLTVDKSRWQEGNVFSCSVLHEALHNH YTQKSLSLSLGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PRDLIDNINVIVLELKGSNGNVTESGCKECEELEEKNIVEFLNR WITFCQSIISTLT | 93 |

(continued)

| Fusion protein (IL-2/IL-15 variant) (variation ) | Amino acid sequence | SEQ ID NO: |
|---|---|---|
| GIC-982C5 (IL-2/IL-15v4) (N77D) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY KTTPPVLDSDGSFFLYSRLTVDKSRWQEGNVFSCSVLHEALHNH YTQKSLSLSLGGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PRDLISDINVIVLELKGSNGNVTESGCKECEELEEKNIVEFLNR WITFCQSIISTLT | 94 |
| GIC-982C6 (IL-2/IL-15v5) (V80L) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY KTTPPVLDSDGSFFLYSRLTVDKSRWQEGNVFSCSVLHEALHNH YTQKSLSLSLGGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PRDLISNINLIVLELKGSNGNVTESGCKECEELEEKNIVEFLNR WITFCQSIISTLT | 95 |
| GIC-982C7 (IL-2/IL-15v6) (L74F, S76D, N77D, V80L) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY KTTPPVLDSDGSFFLYSRLTVDKSRWQEGNVFSCSVLHEALHNH YTQKSLSLSLGGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PRDFIDDINLIVLELKGSNGNVTESGCKECEELEEKNIVEFLNR WITFCQSIISTLT | 96 |
| GIC-982C8 (IL-2/IL-15v7) (D73E, S76D, N77D, V80L) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY KTTPPVLDSDGSFFLYSRLTVDKSRWQEGNVFSCSVLHEALHNH YTQKSLSLSLGGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PRELIDDINLIVLELKGSNGNVTESGCKECEELEEKNIVEFLNR WITFCQSIISTLT | 97 |

(continued)

| Fusion protein (IL-2/IL-15 variant) (variation ) | Amino acid sequence | SEQ ID NO: |
|---|---|---|
| GIC-982C9 (IL-2/IL-15v8) (D73E, L74F, N77D, V80L) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY KTTPPVLDSDGSFFLYSRLTVDKSRWQEGNVFSCSVLHEALHNH YTQKSLSLSLGGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PREFISDINLIVLELKGSNGNVTESGCKECEELEEKNIVEFLNR WITFCQSIISTLT | 98 |
| GIC-982C10 (IL-2/IL-15v9) (D73E, L74F, S76D, V80L) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY KTTPPVLDSDGSFFLYSRLTVDKSRWQEGNVFSCSVLHEALHNH YTQKSLSLSLGGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PREFIDNINLIVLELKGSNGNVTESGCKECEELEEKNIVEFLNR WITFCQSIISTLT | 99 |
| GIC-982C11 (IL-2/IL-15v10) (D73E, L74F, S76D, N77D) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY KTTPPVLDSDGSFFLYSRLTVDKSRWQEGNVFSCSVLHEALHNH YTQKSLSLSLGGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PREFIDNINVIVLELKGSNGNVTESGCKECEELEEKNIVEFLNR WITFCQSIISTLT | 100 |
| GIC-982C12 (IL-2/IL-15v11) (D73E, L74F, S76D, N77D, V80L) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY KTTPPVLDSDGSFFLYSRLTVDKSRWQEGNVFSCSVLHEALHNH YTQKSLSLSLGGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PREFIDDINLIVLELKGSNGNVTESGCKECEELEEKNIVEFLNR WITFCQSIISTLT | 101 |

(continued)

| Fusion protein (IL-2/IL-15 variant) (variation ) | Amino acid sequence | SEQ ID NO: |
|---|---|---|
| GIC-982C13 (IL-2/IL-15v6) (S76E) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY KTTPPVLDSDGSFFLYSRLTVDKSRWQEGNVFSCSVLHEALHNH YTQKSLSLSLGGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PRDLIENINVIVLELKGSNGNVTESGCKECEELEEKNIVEFLNR WITFCQSIISTLT | 102 |
| GIC-982C14 (IL-2/IL-15v7) (S76N) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY KTTPPVLDSDGSFFLYSRLTVDKSRWQEGNVFSCSVLHEALHNH YTQKSLSLSLGGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PRDLINNINVIVLELKGSNGNVTESGCKECEELEEKNIVEFLNR WITFCQSIISTLT | 103 |
| GIC-982C15 (IL-2/IL-15v8) (S76K) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY KTTPPVLDSDGSFFLYSRLTVDKSRWQEGNVFSCSVLHEALHNH YTQKSLSLSLGGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PRDLIKNINVIVLELKGSNGNVTESGCKECEELEEKNIVEFLNR WITFCQSIISTLT | 104 |
| GIC-982C16 (S76L) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY | 105 |

(continued)

| Fusion protein (IL-2/IL-15 variant) (variation ) | Amino acid sequence | SEQ ID NO: |
|---|---|---|
| | KTTPPVLDSDGSFFLYSRLTVDKSRWQEGNVFSCSVLHEALHNH YTQKSLSLSLGGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PRDLILNINVIVLELKGSNGNVTESGCKECEELEEKNIVEFLNR WITFCQSIISTLT | |
| GIC-982C17 (L74F, S76D) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY KTTPPVLDSDGSFFLYSRLTVDKSRWQEGNVFSCSVLHEALHNH YTQKSLSLSLGGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PRDFIDNINVIVLELKGSNGNVTESGCKECEELEEKNIVEFLNR WITFCQSIISTLT | 106 |
| GIC-982C18 (S76D, N77D) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY KTTPPVLDSDGSFFLYSRLTVDKSRWQEGNVFSCSVLHEALHNH YTQKSLSLSLGGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PRDLIDDINVIVLELKGSNGNVTESGCKECEELEEKNIVEFLNR WITFCQSIISTLT | 107 |
| GIC-982C19 (S76D, V80L) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY KTTPPVLDSDGSFFLYSRLTVDKSRWQEGNVFSCSVLHEALHNH YTQKSLSLSLGGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PRDLIDNINLIVLELKGSNGNVTESGCKECEELEEKNIVEFLNR WITFCQSIISTLT | 108 |

(continued)

| Fusion protein (IL-2/IL-15 variant) (variation ) | Amino acid sequence | SEQ ID NO: |
|---|---|---|
| GIC-982C20 (L74F, S76D, N77D) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY KTTPPVLDSDGSFFLYSRLTVDKSRWQEGNVFSCSVLHEALHNH YTQKSLSLSLGGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PRDFIDDINVIVLELKGSNGNVTESGCKECEELEEKNIVEFLNR WITFCQSIISTLT | 109 |
| GIC-982C21 (S76D, N77D, V80L) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY KTTPPVLDSDGSFFLYSRLTVDKSRWQEGNVFSCSVLHEALHNH YTQKSLSLSLGGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PRDLIDDINLIVLELKGSNGNVTESGCKECEELEEKNIVEFLNR WITFCQSIISTLT | 110 |
| GIC-982C22 (L74F, S76D, V80L) | GSAESKYGPPCPPCPAPEAAGGPSVFLFPPKPKDQLMISRTPEV TCVVVDVSQEDPEVQFNWYVDGVEVHNAKTKPREEQFNSTYRVV SVLTVLHQDWLNGKEYKCKVSNKGLPSSIEKTISKAKGQPREPQ VYTLPPSQEEMTKNQVSLTCLVKGFYPSDIAVEWESNGQPENNY KTTPPVLDSDGSFFLYSRLTVDKSRWQEGNVFSCSVLHEALHNH YTQKSLSLSLGGGGGSGGGGSITCPPPMSVEHADIWVKSYSLYS RERYICNSGFKRKAGTSSLTECVLNKATNVAHWTTPSLKCIRGG GGSGGGGSGGGGSGGGGSAPTSSSTKKTQLQLEHLLLDLQMILN GSMHIDATLYTESDVHPSCKVTAMKCFLLELQVISLESKNFHLR PRDFIDNINLIVLELKGSNGNVTESGCKECEELEEKNIVEFLNR WITFCQSIISTLT | 111 |
| GIC-982N1 | APTSSSTKKTQLQLEHLLLDLQMILNGSMHIDATLYTESDVHPS CKVTAMKCFLLELQVISLESKNFHLRPRDLISNINVIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 112 |

(continued)

| Fusion protein (IL-2/IL-15 variant) (variation ) | Amino acid sequence | SEQ ID NO: |
|---|---|---|
| GIC-982N2 (D73E) | APTSSSTKKTQLQLEHLLLDLQMILNGSMHIDATLYTESDVHPS CKVTAMKCFLLELQVISLESKNFHLRPRELISNINVIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 113 |
| GIC-982N3 (L74F) | APTSSSTKKTQLQLEHLLLDLQMILNGSMHIDATLYTESDVHPS CKVTAMKCFLLELQVISLESKNFHLRPRDFISNINVIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 114 |
| GIC-982N4 (S76D) | APTSSSTKKTQLQLEHLLLDLQMILNGSMHIDATLYTESDVHPS CKVTAMKCFLLELQVISLESKNFHLRPRDLIDNINVIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 115 |
| GIC-982N5 (N77D) | APTSSSTKKTQLQLEHLLLDLQMILNGSMHIDATLYTESDVHPS CKVTAMKCFLLELQVISLESKNFHLRPRDLISDINVIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 116 |

(continued)

| Fusion protein (IL-2/IL-15 variant) (variation ) | Amino acid sequence | SEQ ID NO: |
|---|---|---|
| GIC-982N6 (V80L) | APTSSSTKKTQLQLEHLLLDLQMILNGSMHIDATLYTESDVHPS CKVTAMKCFLLELQVISLESKNFHLRPRDLISNINLIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 117 |
| GIC-982N7 (L74F, S76D, N77D, V80L) | APTSSSTKKTQLQLEHLLLDLQMILNGSMHIDATLYTESDVHPS CKVTAMKCFLLELQVISLESKNFHLRPRDFIDDINLIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 118 |
| GIC-982N8 (D73E, S76D, N77D, V80L) | APTSSSTKKTQLQLEHLLLDLQMILNGSMHIDATLYTESDVHPS CKVTAMKCFLLELQVISLESKNFHLRPRELIDDINLIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 119 |
| GIC-982N9 (D73E, L74F, N77D, V80L) | APTSSSTKKTQLQLEHLLLDLQMILNGSMHIDATLYTESDVHPS CKVTAMKCFLLELQVISLESKNFHLRPREFISDINLIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 120 |

(continued)

| Fusion protein (IL-2/IL-15 variant) (variation ) | Amino acid sequence | SEQ ID NO: |
|---|---|---|
| GIC-982N10 (D73E, L74F, S76D, V80L) | APTSSSTKKTQLQLEHLLLDLQMILNGSMHIDATLYTESDVHPS CKVTAMKCFLLELQVISLESKNFHLRPREFIDNINLIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 121 |
| GIC-982N11 (D73E, L74F, S76D, N77D) | APTSSSTKKTQLQLEHLLLDLQMILNGSMHIDATLYTESDVHPS CKVTAMKCFLLELQVISLESKNFHLRPREFIDDINVIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 122 |
| GIC-982N12 (D73E, L74F, S76D, N77D, V80L) | APTSSSTKKTQLQLEHLLLDLQMILNGSMHIDATLYTESDVHPS CKVTAMKCFLLELQVISLESKNFHLRPREFIDDINLIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 123 |
| GIC-982N13 (S76E) | APTSSSTKKTQLQLEHLLLDLQMILNGSMHIDATLYTESDVHPS CKVTAMKCFLLELQVISLESKNFHLRPRDLIENINVIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 124 |

(continued)

| Fusion protein (IL-2/IL-15 variant) (variation ) | Amino acid sequence | SEQ ID NO: |
|---|---|---|
| **GIC-982N14 (S76N)** | APTSSSTKKTQLQLEHLLLDLQMILNGSMHIDATLYTESDVHPS CKVTAMKCFLLELQVISLESKNFHLRPRDLINNINVIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 125 |
| **GIC-982N15 (S76K)** | APTSSSTKKTQLQLEHLLLDLQMILNGSMHIDATLYTESDVHPS CKVTAMKCFLLELQVISLESKNFHLRPRDLIKNINVIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 126 |
| **GIC-982N16 (S76L)** | APTSSSTKKTQLQLEHLLLDLQMILNGSMHIDATLYTESDVHPS CKVTAMKCFLLELQVISLESKNFHLRPRDLILNINVIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 127 |
| **GIC-982N17 (L74F, S76D)** | APTSSSTKKTQLQLEHLLLDLQMILNGSMHIDATLYTESDVHPS CKVTAMKCFLLELQVISLESKNFHLRPRDFIDNINVIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 128 |

(continued)

| Fusion protein (IL-2/IL-15 variant) (variation ) | Amino acid sequence | SEQ ID NO: |
|---|---|---|
| GIC-982N18 (S76D, N77D) | APTSSSTKKTQLQLEHLLLDLQMILNGSMHIDATLYTESDVHPS CKVTAMKCFLLELQVISLESKNFHLRPRDLIDDINVIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 129 |
| GIC-982N19 (S76D, V80L) | APTSSSTKKTQLQLEHLLLDLQMILNGSMHIDATLYTESDVHPS CKVTAMKCFLLELQVISLESKNFHLRPRDLI**D**NINLIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 130 |
| GIC-982N20 (L74F, S76D, N77D) | APTSSSTKKTQLQLEHLLLDLQMILNGSMHIDATLYTESDVHPS CKVTAMKCFLLELQVISLESKNFHLRPRDFIDDINVIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 131 |

(continued)

| Fusion protein (IL-2/IL-15 variant) (variation ) | Amino acid sequence | SEQ ID NO: |
|---|---|---|
| GIC-982N21 (S76D, N77D, V80L) | APTSSSTKKTQLQLEHLLLDLQMILNGSMHIDATLYTESDVHPS CKVTAMKCFLLELQVISLESKNFHLRPRDLIDDINLIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 132 |
| GIC-982N22 (L74F, S76D, V80L) | APTSSSTKKTQLQLEHLLLDLQMILNGSMHIDATLYTESDVHPS CKVTAMKCFLLELQVISLESKNFHLRPRDFIDNINLIVLELKGS NGNVTESGCKECEELEEKNIVEFLNRWITFCQSIISTLTGGGGS GGGGSGGGGSGGGGSITCPPPMSVEHADIWVKSYSLYSRERYIC NSGFKRKAGTSSLTECVLNKATNVAHWTTPSLKCIRGGGGSGGG GSGSAESKYGPPCPPCPAPEAAGGPSVFLFPPKPKDQLMISRTP EVTCVVVDVSQEDPEVQFNWYVDGVEVHNAKTKPREEQFNSTYR VVSVLTVLHQDWLNGKEYKCKVSNKGLPSSIEKTISKAKGQPRE PQVYTLPPSQEEMTKNQVSLTCLVKGFYPSDIAVEWESNGQPEN NYKTTPPVLDSDGSFFLYSRLTVDKSRWQEGNVFSCSVLHEALH NHYTQKSLSLSLG | 133 |
| Fc-IL2 | AESKYGPPCPPCPAPEAAGGPSVFLFPPKPKDTLMISRTPEVTC VVVDVSQEDPEVQFNWYVDGVEVHNAKTKPREEQFNSTYRVVSV LTVLHQDWLNGKEYKCKVSNKGLPSSIEKTISKAKGQPREPQVY TLPPSQEEMTKNQVSLTCLVKGFYPSDIAVEWESNGQPENNYKT TPPVLDSDGSFFLYSRLTVDKSRWQEGNVFSCSVMHEALHNHYT QKSLSLSLGGGGGSAPTSSSTKKTQLQLEHLLLDLQMILNGINN YKNPKLTRMLTFKFYMPKKATELKHLQCLEEELKPLEEVLNLAQ SKNFHLRPRDLISNINVIVLELKGSETTFMCEYADETATIVEFL NRWITFCQSIISTLT | 134 |

[Table 7]

| Fusion protein (IL-2/IL-15 variant) (variation ) | Nucleic acid sequence | SEQ ID NO: |
|---|---|---|
| GIC-982C1 (IL-2/IL-15) | GGATCCGCCGAGTCTAAGTACGGCCCTCCTTGTCCTCCATGTCC TGCTCCAGAAGCTGCCGGAGGGCCCAGTGTGTTTCTGTTCCCTC CAAAGCCTAAGGACCAGCTGATGATCTCTCGGACCCCTGAAGTG ACCTGCGTGGTGGTGGATGTGTCCCAAGAGGACCCTGAGGTGCA GTTCAATTGGTACGTGGACGGCGTGGAAGTGCACAACGCCAAGA CCAAGCCTAGAGAGGAACAGTTCAACTCCACCTACAGAGTGGTG TCCGTGCTGACCGTGCTGCACCAGGATTGGCTGAACGGCAAAGA GTACAAGTGCAAGGTGTCCAACAAGGGCCTGCCTTCCAGCATCG AAAAGACCATCTCCAAGGCCAAGGGCCAGCCTAGGGAACCCCAG GTTTACACCCTGCCTCCAAGCCAAGAGGAAATGACCAAGAACCA GGTGTCCCTGACCTGCCTGGTCAAGGGCTTCTACCCTTCCGATA TCGCCGTGGAATGGGAGAGCAATGGCCAGCCTGAGAACAACTAC AAGACCACACCTCCTGTGCTGGACTCCGACGGCTCCTTCTTTCT GTACTCCCGCCTGACCGTGGACAAGTCCAGATGGCAAGAGGGCA ACGTGTTCTCCTGCTCTGTGCTGCACGAGGCCCTGCACAATCAC TACACCCAGAAGTCCCTGTCTCTGTCCCTTGGCGGTGGCGGAGG ATCTGGCGGAGGCGGATCTATTACATGCCCTCCTCCAATGTCCG TGGAACACGCCGACATCTGGGTCAAGTCCTACAGCCTGTACTCC AGAGAGCGGTACATCTGCAACTCCGGCTTCAAGAGAAAGGCCGG CACCTCTAGCCTGACCGAGTGCGTGCTGAACAAGGCCACCAATG TGGCCCACTGGACAACCCCTAGCCTGAAGTGTATTAGAGGCGGC GGAGGAAGCGGTGGCGGCGGTAGCGGAGGTGGTGGTTCTGGTGG TGGCGGTTCTGCTCCTACCTCCTCCAGCACCAAGAAAACCCAGC TGCAGTTGGAGCATCTGCTGCTGGACCTGCAGATGATCCTGAAC GGCTCCATGCACATCGACGCTACCCTGTACACCGAGTCCGACGT GCACCCTTCCTGTAAAGTGACCGCCATGAAGTGCTTTCTGTTGG AGCTGCAAGTGATCTCCCTGGAATCCAAGAACTTCCACCTGAGG CCTCGGGACCTGATCTCCAACATCAACGTGATCGTGCTGGAACT GAAGGGCTCCAACGGCAACGTGACCGAGTCTGGCTGTAAAGAGT GCGAGGAACTGGAAGAGAAGAACATCGTCGAGTTTCTGAACCGG TGGATCACCTTCTGCCAGTCCATCATCTCCACACTGACC | 135 |

(continued)

| Fusion protein (IL-2/IL-15 variant) (variation ) | Nucleic acid sequence | SEQ ID NO: |
|---|---|---|
| GIC-982C2 (IL-2/IL-15v1) (D73E) | GGATCCGCCGAGTCTAAGTACGGCCCTCCTTGTCCTCCATGTCC TGCTCCAGAAGCTGCCGGAGGGCCCAGTGTGTTTCTGTTCCCTC CAAAGCCTAAGGACCAGCTGATGATCTCTCGGACCCCTGAAGTG ACCTGCGTGGTGGTGGATGTGTCCCAAGAGGACCCTGAGGTGCA GTTCAATTGGTACGTGGACGGCGTGGAAGTGCACAACGCCAAGA CCAAGCCTAGAGAGGAACAGTTCAACTCCACCTACAGAGTGGTG TCCGTGCTGACCGTGCTGCACCAGGATTGGCTGAACGGCAAAGA GTACAAGTGCAAGGTGTCCAACAAGGGCCTGCCTTCCAGCATCG AAAAGACCATCTCCAAGGCCAAGGGCCAGCCTAGGGAACCCCAG GTTTACACCCTGCCTCCAAGCCAAGAGGAAATGACCAAGAACCA GGTGTCCCTGACCTGCCTGGTCAAGGGCTTCTACCCTTCCGATA TCGCCGTGGAATGGGAGAGCAATGGCCAGCCTGAGAACAACTAC AAGACCACACCTCCTGTGCTGGACTCCGACGGCTCCTTCTTTCT GTACTCCCGCCTGACCGTGGACAAGTCCAGATGGCAAGAGGGCA ACGTGTTCTCCTGCTCTGTGCTGCACGAGGCCCTGCACAATCAC TACACCCAGAAGTCCCTGTCTCTGTCCCTTGGCGGTGGCGGAGG ATCTGGCGGAGGCGGATCTATTACATGCCCTCCTCCAATGTCCG TGGAACACGCCGACATCTGGGTCAAGTCCTACAGCCTGTACTCC AGAGAGCGGTACATCTGCAACTCCGGCTTCAAGAGAAAGGCCGG CACCTCTAGCCTGACCGAGTGCGTGCTGAACAAGGCCACCAATG TGGCCCACTGGACAACCCCTAGCCTGAAGTGTATTAGAGGCGGC GGAGGAAGCGGTGGCGGCGGTAGCGGAGGTGGTGGTTCTGGTGG TGGCGGTTCTGCTCCTACCTCCTCCAGCACCAAGAAAACCCAGC TGCAGTTGGAGCATCTGCTGCTGGACCTGCAGATGATCCTGAAC GGCTCCATGCACATCGACGCTACCCTGTACACCGAGTCCGACGT GCACCCTTCCTGTAAAGTGACCGCCATGAAGTGCTTTCTGCTCG AGCTGCAAGTGATCTCCCTGGAATCCAAGAACTTCCACCTGAGG CCAAGAGAGCTGATCTCCAACATCAACGTGATCGTGCTGGAACT GAAGGGCTCCAACGGCAACGTGACCGAGTCTGGCTGTAAAGAGT GCGAGGAACTGGAAGAGAAGAACATCGTCGAGTTTCTGAACCGG TGGATCACCTTCTGCCAGTCCATCATCTCCACACTGACC | 136 |

(continued)

| Fusion protein (IL-2/IL-15 variant) (variation ) | Nucleic acid sequence | SEQ ID NO: |
|---|---|---|
| GIC-982C3 (IL-2/IL-15v2) (L74F) | GGATCCGCCGAGTCTAAGTACGGCCCTCCTTGTCCTCCATGTCC<br>TGCTCCAGAAGCTGCCGGAGGGCCCAGTGTGTTTCTGTTCCCTC<br>CAAAGCCTAAGGACCAGCTGATGATCTCTCGGACCCCTGAAGTG<br>ACCTGCGTGGTGGTGGATGTGTCCCAAGAGGACCCTGAGGTGCA<br>GTTCAATTGGTACGTGGACGGCGTGGAAGTGCACAACGCCAAGA<br>CCAAGCCTAGAGAGGAACAGTTCAACTCCACCTACAGAGTGGTG<br>TCCGTGCTGACCGTGCTGCACCAGGATTGGCTGAACGGCAAAGA<br>GTACAAGTGCAAGGTGTCCAACAAGGGCCTGCCTTCCAGCATCG<br>AAAAGACCATCTCCAAGGCCAAGGGCCAGCCTAGGGAACCCCAG<br>GTTTACACCCTGCCTCCAAGCCAAGAGGAAATGACCAAGAACCA<br>GGTGTCCCTGACCTGCCTGGTCAAGGGCTTCTACCCTTCCGATA<br>TCGCCGTGGAATGGGAGAGCAATGGCCAGCCTGAGAACAACTAC<br>AAGACCACACCTCCTGTGCTGGACTCCGACGGCTCCTTCTTTCT<br>GTACTCCCGCCTGACCGTGGACAAGTCCAGATGGCAAGAGGGCA<br>ACGTGTTCTCCTGCTCTGTGCTGCACGAGGCCCTGCACAATCAC<br>TACACCCAGAAGTCCCTGTCTCTGTCCCTTGGCGGTGGCGGAGG<br>ATCTGGCGGAGGCGGATCTATTACATGCCCTCCTCCAATGTCCG<br>TGGAACACGCCGACATCTGGGTCAAGTCCTACAGCCTGTACTCC<br>AGAGAGCGGTACATCTGCAACTCCGGCTTCAAGAGAAAGGCCGG<br>CACCTCTAGCCTGACCGAGTGCGTGCTGAACAAGGCCACCAATG<br>TGGCCCACTGGACAACCCCTAGCCTGAAGTGTATTAGAGGCGGC<br>GGAGGAAGCGGTGGCGGCGGTAGCGGAGGTGGTGGTTCTGGTGG<br>TGGCGGTTCTGCTCCTACCTCCTCCAGCACCAAGAAAACCCAGC<br>TGCAGTTGGAGCATCTGCTGCTGGACCTGCAGATGATCCTGAAC<br>GGCTCCATGCACATCGACGCTACCCTGTACACCGAGTCCGACGT<br>GCACCCTTCCTGTAAAGTGACCGCCATGAAGTGCTTTCTGCTCG<br>AGCTGCAAGTGATCTCCCTGGAATCCAAGAACTTCCACCTGAGG<br>CCTCGGGACTTCATCTCCAACATCAACGTGATCGTGCTGGAACT<br>GAAGGGCTCCAACGGCAACGTGACCGAGTCTGGCTGTAAAGAGT<br>GCGAGGAACTGGAAGAGAAGAACATCGTCGAGTTTCTGAACCGG<br>TGGATCACCTTCTGCCAGTCCATCATCTCCACACTGACC | 137 |
| GIC-982C4 (IL-2/IL-15v3) (S76D) | GGATCCGCCGAGTCTAAGTACGGCCCTCCTTGTCCTCCATGTCC<br>TGCTCCAGAAGCTGCCGGAGGGCCCAGTGTGTTTCTGTTCCCTC<br>CAAAGCCTAAGGACCAGCTGATGATCTCTCGGACCCCTGAAGTG | 138 |

(continued)

| Fusion protein (IL-2/IL-15 variant) (variation ) | Nucleic acid sequence | SEQ ID NO: |
|---|---|---|
|  | ACCTGCGTGGTGGTGGATGTGTCCCAAGAGGACCCTGAGGTGCA GTTCAATTGGTACGTGGACGGCGTGGAAGTGCACAACGCCAAGA CCAAGCCTAGAGAGGAACAGTTCAACTCCACCTACAGAGTGGTG TCCGTGCTGACCGTGCTGCACCAGGATTGGCTGAACGGCAAAGA GTACAAGTGCAAGGTGTCCAACAAGGGCCTGCCTTCCAGCATCG AAAAGACCATCTCCAAGGCCAAGGGCCAGCCTAGGGAACCCCAG GTTTACACCCTGCCTCCAAGCCAAGAGGAAATGACCAAGAACCA GGTGTCCCTGACCTGCCTGGTCAAGGGCTTCTACCCTTCCGATA TCGCCGTGGAATGGGAGAGCAATGGCCAGCCTGAGAACAACTAC AAGACCACACCTCCTGTGCTGGACTCCGACGGCTCCTTCTTTCT GTACTCCCGCCTGACCGTGGACAAGTCCAGATGGCAAGAGGGCA ACGTGTTCTCCTGCTCTGTGCTGCACGAGGCCCTGCACAATCAC TACACCCAGAAGTCCCTGTCTCTGTCCCTTGGCGGTGGCGGAGG ATCTGGCGGAGGCGGATCTATTACATGCCCTCCTCCAATGTCCG TGGAACACGCCGACATCTGGGTCAAGTCCTACAGCCTGTACTCC AGAGAGCGGTACATCTGCAACTCCGGCTTCAAGAGAAAGGCCGG CACCTCTAGCCTGACCGAGTGCGTGCTGAACAAGGCCACCAATG TGGCCCACTGGACAACCCCTAGCCTGAAGTGTATTAGAGGCGGC GGAGGAAGCGGTGGCGGCGGTAGCGGAGGTGGTGGTTCTGGTGG TGGCGGTTCTGCTCCTACCTCCTCCAGCACCAAGAAAACCCAGC TGCAGTTGGAGCATCTGCTGCTGGACCTGCAGATGATCCTGAAC GGCTCCATGCACATCGACGCTACCCTGTACACCGAGTCCGACGT GCACCCTTCCTGTAAAGTGACCGCCATGAAGTGCTTTCTGCTCG AGCTGCAAGTGATCTCCCTGGAATCCAAGAACTTCCACCTGAGG CCTCGGGACCTGATCGACAACATCAACGTGATCGTGCTGGAACT GAAGGGCTCCAACGGCAACGTGACCGAGTCTGGCTGTAAAGAGT GCGAGGAACTGGAAGAGAAGAACATCGTCGAGTTTCTGAACCGG TGGATCACCTTCTGCCAGTCCATCATCTCCACACTGACC |  |

(continued)

| Fusion protein (IL-2/IL-15 variant) (variation ) | Nucleic acid sequence | SEQ ID NO: |
|---|---|---|
| **GIC-982C5 (IL-2/IL-15v4) (N77D)** | GGATCCGCCGAGTCTAAGTACGGCCCTCCTTGTCCTCCATGTCC TGCTCCAGAAGCTGCCGGAGGGCCCAGTGTGTTTCTGTTCCCTC CAAAGCCTAAGGACCAGCTGATGATCTCTCGGACCCCTGAAGTG ACCTGCGTGGTGGTGGATGTGTCCCAAGAGGACCCTGAGGTGCA GTTCAATTGGTACGTGGACGGCGTGGAAGTGCACAACGCCAAGA CCAAGCCTAGAGAGGAACAGTTCAACTCCACCTACAGAGTGGTG TCCGTGCTGACCGTGCTGCACCAGGATTGGCTGAACGGCAAAGA GTACAAGTGCAAGGTGTCCAACAAGGGCCTGCCTTCCAGCATCG AAAAGACCATCTCCAAGGCCAAGGGCCAGCCTAGGGAACCCCAG GTTTACACCCTGCCTCCAAGCCAAGAGGAAATGACCAAGAACCA GGTGTCCCTGACCTGCCTGGTCAAGGGCTTCTACCCTTCCGATA TCGCCGTGGAATGGGAGAGCAATGGCCAGCCTGAGAACAACTAC AAGACCACACCTCCTGTGCTGGACTCCGACGGCTCCTTCTTTCT GTACTCCCGCCTGACCGTGGACAAGTCCAGATGGCAAGAGGGCA ACGTGTTCTCCTGCTCTGTGCTGCACGAGGCCCTGCACAATCAC TACACCCAGAAGTCCCTGTCTCTGTCCCTTGGCGGTGGCGGAGG ATCTGGCGGAGGCGGATCTATTACATGCCCTCCTCCAATGTCCG TGGAACACGCCGACATCTGGGTCAAGTCCTACAGCCTGTACTCC AGAGAGCGGTACATCTGCAACTCCGGCTTCAAGAGAAAGGCCGG CACCTCTAGCCTGACCGAGTGCGTGCTGAACAAGGCCACCAATG TGGCCCACTGGACAACCCCTAGCCTGAAGTGTATTAGAGGCGGC GGAGGAAGCGGTGGCGGCGGTAGCGGAGGTGGTGGTTCTGGTGG TGGCGGTTCTGCTCCTACCTCCTCCAGCACCAAGAAAACCCAGC TGCAGTTGGAGCATCTGCTGCTGGACCTGCAGATGATCCTGAAC GGCTCCATGCACATCGACGCTACCCTGTACACCGAGTCCGACGT GCACCCTTCCTGTAAAGTGACCGCCATGAAGTGCTTTCTGCTCG AGCTGCAAGTGATCTCCCTGGAATCCAAGAACTTCCACCTGAGG CCTCGGGACCTGATCTCCGACATCAACGTGATCGTGCTGGAACT GAAGGGCTCCAACGGCAACGTGACCGAGTCTGGCTGTAAAGAGT GCGAGGAACTGGAAGAGAAGAACATCGTCGAGTTTCTGAACCGG TGGATCACCTTCTGCCAGTCCATCATCTCCACACTGACC | 139 |

(continued)

| Fusion protein (IL-2/IL-15 variant) (variation ) | Nucleic acid sequence | SEQ ID NO: |
|---|---|---|
| GIC-982C6 (IL-2/IL-15v5) (V80L) | GGATCCGCCGAGTCTAAGTACGGCCCTCCTTGTCCTCCATGTCC TGCTCCAGAAGCTGCCGGAGGGCCCAGTGTGTTTCTGTTCCCTC CAAAGCCTAAGGACCAGCTGATGATCTCTCGGACCCCTGAAGTG ACCTGCGTGGTGGTGGATGTGTCCCAAGAGGACCCTGAGGTGCA GTTCAATTGGTACGTGGACGGCGTGGAAGTGCACAACGCCAAGA CCAAGCCTAGAGAGGAACAGTTCAACTCCACCTACAGAGTGGTG TCCGTGCTGACCGTGCTGCACCAGGATTGGCTGAACGGCAAAGA GTACAAGTGCAAGGTGTCCAACAAGGGCCTGCCTTCCAGCATCG AAAAGACCATCTCCAAGGCCAAGGGCCAGCCTAGGGAACCCCAG GTTTACACCCTGCCTCCAAGCCAAGAGGAAATGACCAAGAACCA GGTGTCCCTGACCTGCCTGGTCAAGGGCTTCTACCCTTCCGATA TCGCCGTGGAATGGGAGAGCAATGGCCAGCCTGAGAACAACTAC AAGACCACACCTCCTGTGCTGGACTCCGACGGCTCCTTCTTTCT GTACTCCCGCCTGACCGTGGACAAGTCCAGATGGCAAGAGGGCA ACGTGTTCTCCTGCTCTGTGCTGCACGAGGCCCTGCACAATCAC TACACCCAGAAGTCCCTGTCTCTGTCCCTTGGCGGTGGCGGAGG ATCTGGCGGAGGCGGATCTATTACATGCCCTCCTCCAATGTCCG TGGAACACGCCGACATCTGGGTCAAGTCCTACAGCCTGTACTCC AGAGAGCGGTACATCTGCAACTCCGGCTTCAAGAGAAAGGCCGG CACCTCTAGCCTGACCGAGTGCGTGCTGAACAAGGCCACCAATG TGGCCCACTGGACAACCCCTAGCCTGAAGTGTATTAGAGGCGGC GGAGGAAGCGGTGGCGGCGGTAGCGGAGGTGGTGGTTCTGGTGG TGGCGGTTCTGCTCCTACCTCCTCCAGCACCAAGAAAACCCAGC TGCAGTTGGAGCATCTGCTGCTGGACCTGCAGATGATCCTGAAC GGCTCCATGCACATCGACGCTACCCTGTACACCGAGTCCGACGT GCACCCTTCCTGTAAAGTGACCGCCATGAAGTGCTTTCTGCTCG AGCTGCAAGTGATCTCCCTGGAATCCAAGAACTTCCACCTGAGG CCTCGGGACCTGATCTCCAACATCAACCTGATCGTGCTGGAACT GAAGGGCTCCAACGGCAACGTGACCGAGTCTGGCTGTAAAGAGT GCGAGGAACTGGAAGAGAAGAACATCGTCGAGTTTCTGAACCGG TGGATCACCTTCTGCCAGTCCATCATCTCCACACTGACC | 140 |
| GIC-982C7 (IL-2/IL-15v6) (L74F, S76D, N77D, V80L) | GGATCCGCCGAGTCTAAGTACGGCCCTCCTTGTCCTCCATGTCC TGCTCCAGAAGCTGCCGGAGGGCCCAGTGTGTTTCTGTTCCCTC CAAAGCCTAAGGACCAGCTGATGATCTCTCGGACCCCTGAAGTG ACCTGCGTGGTGGTGGATGTGTCCCAAGAGGACCCTGAGGTGCA GTTCAATTGGTACGTGGACGGCGTGGAAGTGCACAACGCCAAGA CCAAGCCTAGAGAGGAACAGTTCAACTCCACCTACAGAGTGGTG | 141 |

(continued)

| Fusion protein (IL-2/IL-15 variant) (variation ) | Nucleic acid sequence | SEQ ID NO: |
|---|---|---|
| | TCCGTGCTGACCGTGCTGCACCAGGATTGGCTGAACGGCAAAGA GTACAAGTGCAAGGTGTCCAACAAGGGCCTGCCTTCCAGCATCG AAAAGACCATCTCCAAGGCCAAGGGCCAGCCTAGGGAACCCCAG GTTTACACCCTGCCTCCAAGCCAAGAGGAAATGACCAAGAACCA GGTGTCCCTGACCTGCCTGGTCAAGGGCTTCTACCCTTCCGATA TCGCCGTGGAATGGGAGAGCAATGGCCAGCCTGAGAACAACTAC AAGACCACACCTCCTGTGCTGGACTCCGACGGCTCCTTCTTTCT GTACTCCCGCCTGACCGTGGACAAGTCCAGATGGCAAGAGGGCA ACGTGTTCTCCTGCTCTGTGCTGCACGAGGCCCTGCACAATCAC TACACCCAGAAGTCCCTGTCTCTGTCCCTTGGCGGTGGCGGAGG ATCTGGCGGAGGCGGATCTATTACATGCCCTCCTCCAATGTCCG TGGAACACGCCGACATCTGGGTCAAGTCCTACAGCCTGTACTCC AGAGAGCGGTACATCTGCAACTCCGGCTTCAAGAGAAAGGCCGG CACCTCTAGCCTGACCGAGTGCGTGCTGAACAAGGCCACCAATG TGGCCCACTGGACAACCCCTAGCCTGAAGTGTATTAGAGGCGGC GGAGGAAGCGGTGGCGGCGGTAGCGGAGGTGGTGGTTCTGGTGG TGGCGGTTCTGCTCCTACCTCCTCCAGCACCAAGAAAACCCAGC TGCAGTTGGAGCATCTGCTGCTGGACCTGCAGATGATCCTGAAC GGCTCCATGCACATCGACGCTACCCTGTACACCGAGTCCGACGT GCACCCTTCCTGTAAAGTGACCGCCATGAAGTGCTTTCTGCTCG AGCTGCAAGTGATCTCCCTGGAATCCAAGAACTTCCACCTGAGG CCTCGGGACTTCATCGACGACATCAACCTGATCGTGCTGGAACT GAAGGGCTCCAACGGCAACGTGACCGAGTCTGGCTGTAAAGAGT GCGAGGAACTGGAAGAGAAGAACATCGTCGAGTTTCTGAACCGG TGGATCACCTTCTGCCAGTCCATCATCTCCACACTGACC | |

(continued)

| Fusion protein (IL-2/IL-15 variant) (variation ) | Nucleic acid sequence | SEQ ID NO: |
|---|---|---|
| GIC-982C8 (IL-2/IL-15v7) (D73E, S76D, N77D, V80L) | GGATCCGCCGAGTCTAAGTACGGCCCTCCTTGTCCTCCATGTCC TGCTCCAGAAGCTGCCGGAGGGCCCAGTGTGTTTCTGTTCCCTC CAAAGCCTAAGGACCAGCTGATGATCTCTCGGACCCCTGAAGTG ACCTGCGTGGTGGTGGATGTGTCCCAAGAGGACCCTGAGGTGCA GTTCAATTGGTACGTGGACGGCGTGGAAGTGCACAACGCCAAGA CCAAGCCTAGAGAGGAACAGTTCAACTCCACCTACAGAGTGGTG TCCGTGCTGACCGTGCTGCACCAGGATTGGCTGAACGGCAAAGA GTACAAGTGCAAGGTGTCCAACAAGGGCCTGCCTTCCAGCATCG AAAAGACCATCTCCAAGGCCAAGGGCCAGCCTAGGGAACCCCAG GTTTACACCCTGCCTCCAAGCCAAGAGGAAATGACCAAGAACCA GGTGTCCCTGACCTGCCTGGTCAAGGGCTTCTACCCTTCCGATA TCGCCGTGGAATGGGAGAGCAATGGCCAGCCTGAGAACAACTAC AAGACCACACCTCCTGTGCTGGACTCCGACGGCTCCTTCTTTCT GTACTCCCGCCTGACCGTGGACAAGTCCAGATGGCAAGAGGGCA ACGTGTTCTCCTGCTCTGTGCTGCACGAGGCCCTGCACAATCAC TACACCCAGAAGTCCCTGTCTCTGTCCCTTGGCGGTGGCGGAGG ATCTGGCGGAGGCGGATCTATTACATGCCCTCCTCCAATGTCCG TGGAACACGCCGACATCTGGGTCAAGTCCTACAGCCTGTACTCC AGAGAGCGGTACATCTGCAACTCCGGCTTCAAGAGAAAGGCCGG CACCTCTAGCCTGACCGAGTGCGTGCTGAACAAGGCCACCAATG TGGCCCACTGGACAACCCCTAGCCTGAAGTGTATTAGAGGCGGC GGAGGAAGCGGTGGCGGCGGTAGCGGAGGTGGTGGTTCTGGTGG TGGCGGTTCTGCTCCTACCTCCTCCAGCACCAAGAAAACCCAGC<br><br>TGCAGTTGGAGCATCTGCTGCTGGACCTGCAGATGATCCTGAAC GGCTCCATGCACATCGACGCTACCCTGTACACCGAGTCCGACGT GCACCCTTCCTGTAAAGTGACCGCCATGAAGTGCTTTCTGCTCG AGCTGCAAGTGATCTCCCTGGAATCCAAGAACTTCCACCTGAGG CCAAGAGAGCTGATCGACGACATCAACCTGATCGTGCTGGAACT GAAGGGCTCCAACGGCAACGTGACCGAGTCTGGCTGTAAAGAGT GCGAGGAACTGGAAGAGAAGAACATCGTCGAGTTTCTGAACCGG TGGATCACCTTCTGCCAGTCCATCATCTCCACACTGACC | 142 |

(continued)

| Fusion protein (IL-2/IL-15 variant) (variation ) | Nucleic acid sequence | SEQ ID NO: |
|---|---|---|
| GIC-982C9 (IL-2/IL-15v8) (D73E, L74F, N77D, V80L) | GGATCCGCCGAGTCTAAGTACGGCCCTCCTTGTCCTCCATGTCC<br>TGCTCCAGAAGCTGCCGGAGGGCCCAGTGTGTTTCTGTTCCCTC<br>CAAAGCCTAAGGACCAGCTGATGATCTCTCGGACCCCTGAAGTG<br>ACCTGCGTGGTGGTGGATGTGTCCCAAGAGGACCCTGAGGTGCA<br>GTTCAATTGGTACGTGGACGGCGTGGAAGTGCACAACGCCAAGA<br>CCAAGCCTAGAGAGGAACAGTTCAACTCCACCTACAGAGTGGTG<br>TCCGTGCTGACCGTGCTGCACCAGGATTGGCTGAACGGCAAAGA<br>GTACAAGTGCAAGGTGTCCAACAAGGGCCTGCCTTCCAGCATCG<br>AAAAGACCATCTCCAAGGCCAAGGGCCAGCCTAGGGAACCCCAG<br>GTTTACACCCTGCCTCCAAGCCAAGAGGAAATGACCAAGAACCA<br>GGTGTCCCTGACCTGCCTGGTCAAGGGCTTCTACCCTTCCGATA<br>TCGCCGTGGAATGGGAGAGCAATGGCCAGCCTGAGAACAACTAC<br>AAGACCACACCTCCTGTGCTGGACTCCGACGGCTCCTTCTTTCT<br>GTACTCCCGCCTGACCGTGGACAAGTCCAGATGGCAAGAGGGCA<br>ACGTGTTCTCCTGCTCTGTGCTGCACGAGGCCCTGCACAATCAC<br>TACACCCAGAAGTCCCTGTCTCTGTCCCTTGGCGGTGGCGGAGG<br>ATCTGGCGGAGGCGGATCTATTACATGCCCTCCTCCAATGTCCG<br>TGGAACACGCCGACATCTGGGTCAAGTCCTACAGCCTGTACTCC<br>AGAGAGCGGTACATCTGCAACTCCGGCTTCAAGAGAAAGGCCGG<br>CACCTCTAGCCTGACCGAGTGCGTGCTGAACAAGGCCACCAATG<br>TGGCCCACTGGACAACCCCTAGCCTGAAGTGTATTAGAGGCGGC<br>GGAGGAAGCGGTGGCGGCGGTAGCGGAGGTGGTGGTTCTGGTGG<br>TGGCGGTTCTGCTCCTACCTCCTCCAGCACCAAGAAAACCCAGC<br>TGCAGTTGGAGCATCTGCTGCTGGACCTGCAGATGATCCTGAAC<br>GGCTCCATGCACATCGACGCTACCCTGTACACCGAGTCCGACGT<br>GCACCCTTCCTGTAAAGTGACCGCCATGAAGTGCTTTCTGCTCG<br>AGCTGCAAGTGATCTCCCTGGAATCCAAGAACTTCCACCTGAGG<br>CCAAGAGAGTTCATCTCCGACATCAACCTGATCGTGCTGGAACT<br>GAAGGGCTCCAACGGCAACGTGACCGAGTCTGGCTGTAAAGAGT<br>GCGAGGAACTGGAAGAGAAGAACATCGTCGAGTTTCTGAACCGG<br>TGGATCACCTTCTGCCAGTCCATCATCTCCACACTGACC | 143 |
| GIC-982C10 (IL-2/IL-15v9) (D73E, L74F, S76D, V80L) | GGATCCGCCGAGTCTAAGTACGGCCCTCCTTGTCCTCCATGTCC<br>TGCTCCAGAAGCTGCCGGAGGGCCCAGTGTGTTTCTGTTCCCTC<br>CAAAGCCTAAGGACCAGCTGATGATCTCTCGGACCCCTGAAGTG<br>ACCTGCGTGGTGGTGGATGTGTCCCAAGAGGACCCTGAGGTGCA<br>GTTCAATTGGTACGTGGACGGCGTGGAAGTGCACAACGCCAAGA<br>CCAAGCCTAGAGAGGAACAGTTCAACTCCACCTACAGAGTGGTG<br>TCCGTGCTGACCGTGCTGCACCAGGATTGGCTGAACGGCAAAGA<br>GTACAAGTGCAAGGTGTCCAACAAGGGCCTGCCTTCCAGCATCG<br>AAAAGACCATCTCCAAGGCCAAGGGCCAGCCTAGGGAACCCCAG | 144 |

(continued)

| Fusion protein (IL-2/IL-15 variant) (variation ) | Nucleic acid sequence | SEQ ID NO: |
|---|---|---|
| | GTTTACACCCTGCCTCCAAGCCAAGAGGAAATGACCAAGAACCA GGTGTCCCTGACCTGCCTGGTCAAGGGCTTCTACCCTTCCGATA TCGCCGTGGAATGGGAGAGCAATGGCCAGCCTGAGAACAACTAC AAGACCACACCTCCTGTGCTGGACTCCGACGGCTCCTTCTTTCT GTACTCCCGCCTGACCGTGGACAAGTCCAGATGGCAAGAGGGCA ACGTGTTCTCCTGCTCTGTGCTGCACGAGGCCCTGCACAATCAC TACACCCAGAAGTCCCTGTCTCTGTCCCTTGGCGGTGGCGGAGG ATCTGGCGGAGGCGGATCTATTACATGCCCTCCTCCAATGTCCG TGGAACACGCCGACATCTGGGTCAAGTCCTACAGCCTGTACTCC AGAGAGCGGTACATCTGCAACTCCGGCTTCAAGAGAAAGGCCGG CACCTCTAGCCTGACCGAGTGCGTGCTGAACAAGGCCACCAATG TGGCCCACTGGACAACCCCTAGCCTGAAGTGTATTAGAGGCGGC GGAGGAAGCGGTGGCGGCGGTAGCGGAGGTGGTGGTTCTGGTGG TGGCGGTTCTGCTCCTACCTCCTCCAGCACCAAGAAAACCCAGC TGCAGTTGGAGCATCTGCTGCTGGACCTGCAGATGATCCTGAAC GGCTCCATGCACATCGACGCTACCCTGTACACCGAGTCCGACGT GCACCCTTCCTGTAAAGTGACCGCCATGAAGTGCTTTCTGCTCG AGCTGCAAGTGATCTCCCTGGAATCCAAGAACTTCCACCTGAGG CCAAGAGAGTTCATCGACAACATCAACCTGATCGTCCTGGAACT GAAGGGCTCCAACGGCAACGTGACCGAGTCTGGCTGTAAAGAGT GCGAGGAACTGGAAGAGAAGAACATCGTCGAGTTTCTGAACCGG TGGATCACCTTCTGCCAGTCCATCATCTCCACACTGACC | |

(continued)

| Fusion protein (IL-2/IL-15 variant) (variation ) | Nucleic acid sequence | SEQ ID NO: |
|---|---|---|
| GIC-982C11 (IL-2/IL-15v10) (D73E, L74F, S76D, N77D) | GGATCCGCCGAGTCTAAGTACGGCCCTCCTTGTCCTCCATGTCC<br>TGCTCCAGAAGCTGCCGGAGGGCCCAGTGTGTTTCTGTTCCCTC<br>CAAAGCCTAAGGACCAGCTGATGATCTCTCGGACCCCTGAAGTG<br>ACCTGCGTGGTGGTGGATGTGTCCCAAGAGGACCCTGAGGTGCA<br>GTTCAATTGGTACGTGGACGGCGTGGAAGTGCACAACGCCAAGA<br>CCAAGCCTAGAGAGGAACAGTTCAACTCCACCTACAGAGTGGTG<br>TCCGTGCTGACCGTGCTGCACCAGGATTGGCTGAACGGCAAAGA<br>GTACAAGTGCAAGGTGTCCAACAAGGGCCTGCCTTCCAGCATCG<br>AAAAGACCATCTCCAAGGCCAAGGGCCAGCCTAGGGAACCCCAG<br>GTTTACACCCTGCCTCCAAGCCAAGAGGAAATGACCAAGAACCA<br>GGTGTCCCTGACCTGCCTGGTCAAGGGCTTCTACCCTTCCGATA<br>TCGCCGTGGAATGGGAGAGCAATGGCCAGCCTGAGAACAACTAC<br>AAGACCACACCTCCTGTGCTGGACTCCGACGGCTCCTTCTTTCT<br>GTACTCCCGCCTGACCGTGGACAAGTCCAGATGGCAAGAGGGCA<br>ACGTGTTCTCCTGCTCTGTGCTGCACGAGGCCCTGCACAATCAC<br>TACACCCAGAAGTCCCTGTCTCTGTCCCTTGGCGGTGGCGGAGG<br>ATCTGGCGGAGGCGGATCTATTACATGCCCTCCTCCAATGTCCG<br>TGGAACACGCCGACATCTGGGTCAAGTCCTACAGCCTGTACTCC<br>AGAGAGCGGTACATCTGCAACTCCGGCTTCAAGAGAAAGGCCGG<br>CACCTCTAGCCTGACCGAGTGCGTGCTGAACAAGGCCACCAATG<br>TGGCCCACTGGACAACCCCTAGCCTGAAGTGTATTAGAGGCGGC<br>GGAGGAAGCGGTGGCGGCGGTAGCGGAGGTGGTGGTTCTGGTGG<br>TGGCGGTTCTGCTCCTACCTCCTCCAGCACCAAGAAAACCCAGC<br>TGCAGTTGGAGCATCTGCTGCTGGACCTGCAGATGATCCTGAAC<br>GGCTCCATGCACATCGACGCTACCCTGTACACCGAGTCCGACGT<br>GCACCCTTCCTGTAAAGTGACCGCCATGAAGTGCTTTCTGCTCG<br>AGCTGCAAGTGATCTCCCTGGAATCCAAGAACTTCCACCTGAGG<br>CCAAGAGAGTTCATCGACGACATCAACGTGATCGTGCTGGAACT<br>GAAGGGCTCCAACGGCAACGTGACCGAGTCTGGCTGTAAAGAGT<br>GCGAGGAACTGGAAGAGAAGAACATCGTCGAGTTTCTGAACCGG<br>TGGATCACCTTCTGCCAGTCCATCATCTCCACACTGACC | 145 |

(continued)

| Fusion protein (IL-2/IL-15 variant) (variation ) | Nucleic acid sequence | SEQ ID NO: |
|---|---|---|
| GIC-982C12 (IL-2/IL-15v11) (D73E, L74F, S76D, N77D, V80L) | GGATCCGCCGAGTCTAAGTACGGCCCTCCTTGTCCTCCATGTCC TGCTCCAGAAGCTGCCGGAGGGCCCAGTGTGTTTCTGTTCCCTC CAAAGCCTAAGGACCAGCTGATGATCTCTCGGACCCCTGAAGTG ACCTGCGTGGTGGTGGATGTGTCCCAAGAGGACCCTGAGGTGCA GTTCAATTGGTACGTGGACGGCGTGGAAGTGCACAACGCCAAGA CCAAGCCTAGAGAGGAACAGTTCAACTCCACCTACAGAGTGGTG TCCGTGCTGACCGTGCTGCACCAGGATTGGCTGAACGGCAAAGA GTACAAGTGCAAGGTGTCCAACAAGGGCCTGCCTTCCAGCATCG AAAAGACCATCTCCAAGGCCAAGGGCCAGCCTAGGGAACCCCAG GTTTACACCCTGCCTCCAAGCCAAGAGGAAATGACCAAGAACCA GGTGTCCCTGACCTGCCTGGTCAAGGGCTTCTACCCTTCCGATA TCGCCGTGGAATGGGAGAGCAATGGCCAGCCTGAGAACAACTAC AAGACCACACCTCCTGTGCTGGACTCCGACGGCTCCTTCTTTCT GTACTCCCGCCTGACCGTGGACAAGTCCAGATGGCAAGAGGGCA ACGTGTTCTCCTGCTCTGTGCTGCACGAGGCCCTGCACAATCAC TACACCCAGAAGTCCCTGTCTCTGTCCCTTGGCGGTGGCGGAGG ATCTGGCGGAGGCGGATCTATTACATGCCCTCCTCCAATGTCCG TGGAACACGCCGACATCTGGGTCAAGTCCTACAGCCTGTACTCC AGAGAGCGGTACATCTGCAACTCCGGCTTCAAGAGAAAGGCCGG CACCTCTAGCCTGACCGAGTGCGTGCTGAACAAGGCCACCAATG TGGCCCACTGGACAACCCCTAGCCTGAAGTGTATTAGAGGCGGC GGAGGAAGCGGTGGCGGCGGTAGCGGAGGTGGTGGTTCTGGTGG TGGCGGTTCTGCTCCTACCTCCTCCAGCACCAAGAAAACCCAGC TGCAGTTGGAGCATCTGCTGCTGGACCTGCAGATGATCCTGAAC GGCTCCATGCACATCGACGCTACCCTGTACACCGAGTCCGACGT GCACCCTTCCTGTAAAGTGACCGCCATGAAGTGCTTTCTGCTCG AGCTGCAAGTGATCTCCCTGGAATCCAAGAACTTCCACCTGAGG CCAAGAGAGTTCATCGACGACATCAACCTGATCGTGCTGGAACT GAAGGGCTCCAACGGCAACGTGACCGAGTCTGGCTGTAAAGAGT GCGAGGAACTGGAAGAGAAGAACATCGTCGAGTTTCTGAACCGG TGGATCACCTTCTGCCAGTCCATCATCTCCACACTGACC | 146 |
| GIC-982C13 (IL-2/IL-15v6) (S76E) | GGATCCGCCGAGTCTAAGTACGGCCCTCCTTGTCCTCCATGTCC TGCTCCAGAAGCTGCCGGAGGGCCCAGTGTGTTTCTGTTCCCTC CAAAGCCTAAGGACCAGCTGATGATCTCTCGGACCCCTGAAGTG ACCTGCGTGGTGGTGGATGTGTCCCAAGAGGACCCTGAGGTGCA GTTCAATTGGTACGTGGACGGCGTGGAAGTGCACAACGCCAAGA CCAAGCCTAGAGAGGAACAGTTCAACTCCACCTACAGAGTGGTG TCCGTGCTGACCGTGCTGCACCAGGATTGGCTGAACGGCAAAGA GTACAAGTGCAAGGTGTCCAACAAGGGCCTGCCTTCCAGCATCG AAAAGACCATCTCCAAGGCCAAGGGCCAGCCTAGGGAACCCCAG GTTTACACCCTGCCTCCAAGCCAAGAGGAAATGACCAAGAACCA GGTGTCCCTGACCTGCCTGGTCAAGGGCTTCTACCCTTCCGATA TCGCCGTGGAATGGGAGAGCAATGGCCAGCCTGAGAACAACTAC | 147 |

(continued)

| Fusion protein (IL-2/IL-15 variant) (variation ) | Nucleic acid sequence | SEQ ID NO: |
|---|---|---|
| | AAGACCACACCTCCTGTGCTGGACTCCGACGGCTCCTTCTTTCTGTACTCCCGCCTGACCGTGGACAAGTCCAGATGGCAAGAGGGCAACGTGTTCTCCTGCTCTGTGCTGCACGAGGCCCTGCACAATCACTACACCCAGAAGTCCCTGTCTCTGTCCCTTGGCGGTGGCGGAGGATCTGGCGGAGGCGGATCTATTACATGCCCTCCTCCAATGTCCGTGGAACACGCCGACATCTGGGTCAAGTCCTACAGCCTGTACTCCAGAGAGCGGTACATCTGCAACTCCGGCTTCAAGAGAAAGGCCGGCACCTCTAGCCTGACCGAGTGCGTGCTGAACAAGGCCACCAATGTGGCCCACTGGACAACCCCTAGCCTGAAGTGTATTAGAGGCGGCGGAGGAAGCGGTGGCGGCGGTAGCGGAGGTGGTGGTTCTGGTGGTGGCGGTTCTGCTCCTACCTCCTCCAGCACCAAGAAAACCCAGCTGCAGTTGGAGCATCTGCTGCTGGACCTGCAGATGATCCTGAACGGCTCCATGCACATCGACGCTACCCTGTACACCGAGTCCGACGTGCACCCTTCCTGTAAAGTGACCGCCATGAAGTGCTTTCTGTTGGAGCTGCAAGTGATCTCCCTGGAATCCAAGAACTTCCACCTGAGGCCTCGGGACCTGATCGAAAACATCAACGTGATCGTGCTGGAACTGAAGGGCTCCAACGGCAACGTGACCGAGTCTGGCTGTAAAGAGTGCGAGGAACTGGAAGAGAAGAACATCGTCGAGTTTCTGAACCGGTGGATCACCTTCTGCCAGTCCATCATCTCCACACTGACC | |

(continued)

| Fusion protein (IL-2/IL-15 variant) (variation ) | Nucleic acid sequence | SEQ ID NO: |
|---|---|---|
| GIC-982C14 (IL-2/IL-15v7) (S76N) | GGATCCGCCGAGTCTAAGTACGGCCCTCCTTGTCCTCCATGTCC TGCTCCAGAAGCTGCCGGAGGGCCCAGTGTGTTTCTGTTCCCTC CAAAGCCTAAGGACCAGCTGATGATCTCTCGGACCCCTGAAGTG ACCTGCGTGGTGGTGGATGTGTCCCAAGAGGACCCTGAGGTGCA GTTCAATTGGTACGTGGACGGCGTGGAAGTGCACAACGCCAAGA CCAAGCCTAGAGAGGAACAGTTCAACTCCACCTACAGAGTGGTG TCCGTGCTGACCGTGCTGCACCAGGATTGGCTGAACGGCAAAGA GTACAAGTGCAAGGTGTCCAACAAGGGCCTGCCTTCCAGCATCG AAAAGACCATCTCCAAGGCCAAGGGCCAGCCTAGGGAACCCCAG GTTTACACCCTGCCTCCAAGCCAAGAGGAAATGACCAAGAACCA GGTGTCCCTGACCTGCCTGGTCAAGGGCTTCTACCCTTCCGATA TCGCCGTGGAATGGGAGAGCAATGGCCAGCCTGAGAACAACTAC AAGACCACACCTCCTGTGCTGGACTCCGACGGCTCCTTCTTTCT GTACTCCCGCCTGACCGTGGACAAGTCCAGATGGCAAGAGGGCA ACGTGTTCTCCTGCTCTGTGCTGCACGAGGCCCTGCACAATCAC TACACCCAGAAGTCCCTGTCTCTGTCCCTTGGCGGTGGCGGAGG ATCTGGCGGAGGCGGATCTATTACATGCCCTCCTCCAATGTCCG TGGAACACGCCGACATCTGGGTCAAGTCCTACAGCCTGTACTCC AGAGAGCGGTACATCTGCAACTCCGGCTTCAAGAGAAAGGCCGG CACCTCTAGCCTGACCGAGTGCGTGCTGAACAAGGCCACCAATG TGGCCCACTGGACAACCCCTAGCCTGAAGTGTATTAGAGGCGGC GGAGGAAGCGGTGGCGGCGGTAGCGGAGGTGGTGGTTCTGGTGG TGGCGGTTCTGCTCCTACCTCCTCCAGCACCAAGAAAACCCAGC TGCAGTTGGAGCATCTGCTGCTGGACCTGCAGATGATCCTGAAC GGCTCCATGCACATCGACGCTACCCTGTACACCGAGTCCGACGT GCACCCTTCCTGTAAAGTGACCGCCATGAAGTGCTTTCTGTTGG AGCTGCAAGTGATCTCCCTGGAATCCAAGAACTTCCACCTGAGG CCTCGGGACCTGATCAACAACATCAACGTGATCGTGCTGGAACT GAAGGGCTCCAACGGCAACGTGACCGAGTCTGGCTGTAAAGAGT GCGAGGAACTGGAAGAGAAGAACATCGTCGAGTTTCTGAACCGG TGGATCACCTTCTGCCAGTCCATCATCTCCACACTGACC | 148 |

(continued)

| Fusion protein (IL-2/IL-15 variant) (variation ) | Nucleic acid sequence | SEQ ID NO: |
|---|---|---|
| GIC-982C15 (IL-2/IL-15v8) (S76K) | GGATCCGCCGAGTCTAAGTACGGCCCTCCTTGTCCTCCATGTCC TGCTCCAGAAGCTGCCGGAGGGCCCAGTGTGTTTCTGTTCCCTC CAAAGCCTAAGGACCAGCTGATGATCTCTCGGACCCCTGAAGTG ACCTGCGTGGTGGTGGATGTGTCCCAAGAGGACCCTGAGGTGCA GTTCAATTGGTACGTGGACGGCGTGGAAGTGCACAACGCCAAGA CCAAGCCTAGAGAGGAACAGTTCAACTCCACCTACAGAGTGGTG TCCGTGCTGACCGTGCTGCACCAGGATTGGCTGAACGGCAAAGA GTACAAGTGCAAGGTGTCCAACAAGGGCCTGCCTTCCAGCATCG AAAAGACCATCTCCAAGGCCAAGGGCCAGCCTAGGGAACCCCAG GTTTACACCCTGCCTCCAAGCCAAGAGGAAATGACCAAGAACCA GGTGTCCCTGACCTGCCTGGTCAAGGGCTTCTACCCTTCCGATA TCGCCGTGGAATGGGAGAGCAATGGCCAGCCTGAGAACAACTAC AAGACCACACCTCCTGTGCTGGACTCCGACGGCTCCTTCTTTCT GTACTCCCGCCTGACCGTGGACAAGTCCAGATGGCAAGAGGGCA ACGTGTTCTCCTGCTCTGTGCTGCACGAGGCCCTGCACAATCAC TACACCCAGAAGTCCCTGTCTCTGTCCCTTGGCGGTGGCGGAGG ATCTGGCGGAGGCGGATCTATTACATGCCCTCCTCCAATGTCCG TGGAACACGCCGACATCTGGGTCAAGTCCTACAGCCTGTACTCC AGAGAGCGGTACATCTGCAACTCCGGCTTCAAGAGAAAGGCCGG CACCTCTAGCCTGACCGAGTGCGTGCTGAACAAGGCCACCAATG TGGCCCACTGGACAACCCCTAGCCTGAAGTGTATTAGAGGCGGC GGAGGAAGCGGTGGCGGCGGTAGCGGAGGTGGTGGTTCTGGTGG TGGCGGTTCTGCTCCTACCTCCTCCAGCACCAAGAAAACCCAGC TGCAGTTGGAGCATCTGCTGCTGGACCTGCAGATGATCCTGAAC GGCTCCATGCACATCGACGCTACCCTGTACACCGAGTCCGACGT GCACCCTTCCTGTAAAGTGACCGCCATGAAGTGCTTTCTGTTGG AGCTGCAAGTGATCTCCCTGGAATCCAAGAACTTCCACCTGAGG CCTCGGGACCTGATCAAGAACATCAACGTGATCGTGCTGGAACT GAAGGGCTCCAACGGCAACGTGACCGAGTCTGGCTGTAAAGAGT GCGAGGAACTGGAAGAGAAGAACATCGTCGAGTTTCTGAACCGG TGGATCACCTTCTGCCAGTCCATCATCTCCACACTGACC | 149 |

(continued)

| Fusion protein (IL-2/IL-15 variant) (variation ) | Nucleic acid sequence | SEQ ID NO: |
|---|---|---|
| GIC-982C16 (S76L) | GGATCCGCCGAGTCTAAGTACGGCCCTCCTTGTCCTCCATGTCC TGCTCCAGAAGCTGCCGGAGGGCCCAGTGTGTTTCTGTTCCCTC CAAAGCCTAAGGACCAGCTGATGATCTCTCGGACCCCTGAAGTG ACCTGCGTGGTGGTGGATGTGTCCCAAGAGGACCCTGAGGTGCA GTTCAATTGGTACGTGGACGGCGTGGAAGTGCACAACGCCAAGA CCAAGCCTAGAGAGGAACAGTTCAACTCCACCTACAGAGTGGTG TCCGTGCTGACCGTGCTGCACCAGGATTGGCTGAACGGCAAAGA GTACAAGTGCAAGGTGTCCAACAAGGGCCTGCCTTCCAGCATCG AAAAGACCATCTCCAAGGCCAAGGGCCAGCCTAGGGAACCCCAG GTTTACACCCTGCCTCCAAGCCAAGAGGAAATGACCAAGAACCA GGTGTCCCTGACCTGCCTGGTCAAGGGCTTCTACCCTTCCGATA TCGCCGTGGAATGGGAGAGCAATGGCCAGCCTGAGAACAACTAC AAGACCACACCTCCTGTGCTGGACTCCGACGGCTCCTTCTTTCT GTACTCCCGCCTGACCGTGGACAAGTCCAGATGGCAAGAGGGCA ACGTGTTCTCCTGCTCTGTGCTGCACGAGGCCCTGCACAATCAC<br><br>TACACCCAGAAGTCCCTGTCTCTGTCCCTTGGCGGTGGCGGAGG ATCTGGCGGAGGCGGATCTATTACATGCCCTCCTCCAATGTCCG TGGAACACGCCGACATCTGGGTCAAGTCCTACAGCCTGTACTCC AGAGAGCGGTACATCTGCAACTCCGGCTTCAAGAGAAAGGCCGG CACCTCTAGCCTGACCGAGTGCGTGCTGAACAAGGCCACCAATG TGGCCCACTGGACAACCCCTAGCCTGAAGTGTATTAGAGGCGGC GGAGGAAGCGGTGGCGGCGGTAGCGGAGGTGGTGGTTCTGGTGG TGGCGGTTCTGCTCCTACCTCCTCCAGCACCAAGAAAACCCAGC TGCAGTTGGAGCATCTGCTGCTGGACCTGCAGATGATCCTGAAC GGCTCCATGCACATCGACGCTACCCTGTACACCGAGTCCGACGT GCACCCTTCCTGTAAAGTGACCGCCATGAAGTGCTTTCTGTTGG AGCTGCAAGTGATCTCCCTGGAATCCAAGAACTTCCACCTGAGG CCTCGGGACCTGATCCTCAACATCAACGTGATCGTGCTGGAACT GAAGGGCTCCAACGGCAACGTGACCGAGTCTGGCTGTAAAGAGT GCGAGGAACTGGAAGAGAAGAACATCGTCGAGTTTCTGAACCGG TGGATCACCTTCTGCCAGTCCATCATCTCCACACTGACC | 150 |

(continued)

| Fusion protein (IL-2/IL-15 variant) (variation ) | Nucleic acid sequence | SEQ ID NO: |
|---|---|---|
| GIC-982C17 (L74F, S76D) | GGATCCGCCGAGTCTAAGTACGGCCCTCCTTGTCCTCCATGTCC<br>TGCTCCAGAAGCTGCCGGAGGGCCCAGTGTGTTTCTGTTCCCTC<br>CAAAGCCTAAGGACCAGCTGATGATCTCTCGGACCCCTGAAGTG<br>ACCTGCGTGGTGGTGGATGTGTCCCAAGAGGACCCTGAGGTGCA<br>GTTCAATTGGTACGTGGACGGCGTGGAAGTGCACAACGCCAAGA<br>CCAAGCCTAGAGAGGAACAGTTCAACTCCACCTACAGAGTGGTG<br>TCCGTGCTGACCGTGCTGCACCAGGATTGGCTGAACGGCAAAGA<br>GTACAAGTGCAAGGTGTCCAACAAGGGCCTGCCTTCCAGCATCG<br>AAAAGACCATCTCCAAGGCCAAGGGCCAGCCTAGGGAACCCCAG<br>GTTTACACCCTGCCTCCAAGCCAAGAGGAAATGACCAAGAACCA<br>GGTGTCCCTGACCTGCCTGGTCAAGGGCTTCTACCCTTCCGATA<br>TCGCCGTGGAATGGGAGAGCAATGGCCAGCCTGAGAACAACTAC<br>AAGACCACACCTCCTGTGCTGGACTCCGACGGCTCCTTCTTTCT<br>GTACTCCCGCCTGACCGTGGACAAGTCCAGATGGCAAGAGGGCA<br>ACGTGTTCTCCTGCTCTGTGCTGCACGAGGCCCTGCACAATCAC<br>TACACCCAGAAGTCCCTGTCTCTGTCCCTTGGCGGTGGCGGAGG<br>ATCTGGCGGAGGCGGATCTATTACATGCCCTCCTCCAATGTCCG<br>TGGAACACGCCGACATCTGGGTCAAGTCCTACAGCCTGTACTCC<br>AGAGAGCGGTACATCTGCAACTCCGGCTTCAAGAGAAAGGCCGG<br>CACCTCTAGCCTGACCGAGTGCGTGCTGAACAAGGCCACCAATG<br>TGGCCCACTGGACAACCCCTAGCCTGAAGTGTATTAGAGGCGGC<br>GGAGGAAGCGGTGGCGGCGGTAGCGGAGGTGGTGGTTCTGGTGG<br>TGGCGGTTCTGCTCCTACCTCCTCCAGCACCAAGAAAACCCAGC<br>TGCAGTTGGAGCATCTGCTGCTGGACCTGCAGATGATCCTGAAC<br>GGCTCCATGCACATCGACGCTACCCTGTACACCGAGTCCGACGT<br>GCACCCTTCCTGTAAAGTGACCGCCATGAAGTGCTTTCTGCTCG<br>AGCTGCAAGTGATCTCCCTGGAATCCAAGAACTTCCACCTGAGG<br>CCTCGGGACTTCATCGACAACATCAACGTGATCGTGCTGGAACT<br>GAAGGGCTCCAACGGCAACGTGACCGAGTCTGGCTGTAAAGAGT<br>GCGAGGAACTGGAAGAGAAGAACATCGTCGAGTTTCTGAACCGG<br>TGGATCACCTTCTGCCAGTCCATCATCTCCACACTGACC | 151 |

(continued)

| Fusion protein (IL-2/IL-15 variant) (variation ) | Nucleic acid sequence | SEQ ID NO: |
|---|---|---|
| GIC-982C18 (S76D, N77D) | GGATCCGCCGAGTCTAAGTACGGCCCTCCTTGTCCTCCATGTCC TGCTCCAGAAGCTGCCGGAGGGCCCAGTGTGTTTCTGTTCCCTC CAAAGCCTAAGGACCAGCTGATGATCTCTCGGACCCCTGAAGTG ACCTGCGTGGTGGTGGATGTGTCCCAAGAGGACCCTGAGGTGCA GTTCAATTGGTACGTGGACGGCGTGGAAGTGCACAACGCCAAGA CCAAGCCTAGAGAGGAACAGTTCAACTCCACCTACAGAGTGGTG TCCGTGCTGACCGTGCTGCACCAGGATTGGCTGAACGGCAAAGA GTACAAGTGCAAGGTGTCCAACAAGGGCCTGCCTTCCAGCATCG AAAAGACCATCTCCAAGGCCAAGGGCCAGCCTAGGGAACCCCAG GTTTACACCCTGCCTCCAAGCCAAGAGGAAATGACCAAGAACCA GGTGTCCCTGACCTGCCTGGTCAAGGGCTTCTACCCTTCCGATA TCGCCGTGGAATGGGAGAGCAATGGCCAGCCTGAGAACAACTAC AAGACCACACCTCCTGTGCTGGACTCCGACGGCTCCTTCTTTCT GTACTCCCGCCTGACCGTGGACAAGTCCAGATGGCAAGAGGGCA ACGTGTTCTCCTGCTCTGTGCTGCACGAGGCCCTGCACAATCAC TACACCCAGAAGTCCCTGTCTCTGTCCCTTGGCGGTGGCGGAGG ATCTGGCGGAGGCGGATCTATTACATGCCCTCCTCCAATGTCCG TGGAACACGCCGACATCTGGGTCAAGTCCTACAGCCTGTACTCC AGAGAGCGGTACATCTGCAACTCCGGCTTCAAGAGAAAGGCCGG CACCTCTAGCCTGACCGAGTGCGTGCTGAACAAGGCCACCAATG TGGCCCACTGGACAACCCCTAGCCTGAAGTGTATTAGAGGCGGC GGAGGAAGCGGTGGCGGCGGTAGCGGAGGTGGTGGTTCTGGTGG TGGCGGTTCTGCTCCTACCTCCTCCAGCACCAAGAAAACCCAGC TGCAGTTGGAGCATCTGCTGCTGGACCTGCAGATGATCCTGAAC GGCTCCATGCACATCGACGCTACCCTGTACACCGAGTCCGACGT GCACCCTTCCTGTAAAGTGACCGCCATGAAGTGCTTTCTGCTCG AGCTGCAAGTGATCTCCCTGGAATCCAAGAACTTCCACCTGAGG CCTCGGGACCTGATCGACGACATCAACGTGATCGTGCTGGAACT GAAGGGCTCCAACGGCAACGTGACCGAGTCTGGCTGTAAAGAGT GCGAGGAACTGGAAGAGAAGAACATCGTCGAGTTTCTGAACCGG TGGATCACCTTCTGCCAGTCCATCATCTCCACACTGACC | 152 |

(continued)

| Fusion protein (IL-2/IL-15 variant) (variation ) | Nucleic acid sequence | SEQ ID NO: |
|---|---|---|
| GIC-982C19 (S76D, V80L) | GGATCCGCCGAGTCTAAGTACGGCCCTCCTTGTCCTCCATGTCC TGCTCCAGAAGCTGCCGGAGGGCCCAGTGTGTTTCTGTTCCCTC CAAAGCCTAAGGACCAGCTGATGATCTCTCGGACCCCTGAAGTG ACCTGCGTGGTGGTGGATGTGTCCCAAGAGGACCCTGAGGTGCA GTTCAATTGGTACGTGGACGGCGTGGAAGTGCACAACGCCAAGA CCAAGCCTAGAGAGGAACAGTTCAACTCCACCTACAGAGTGGTG TCCGTGCTGACCGTGCTGCACCAGGATTGGCTGAACGGCAAAGA GTACAAGTGCAAGGTGTCCAACAAGGGCCTGCCTTCCAGCATCG AAAAGACCATCTCCAAGGCCAAGGGCCAGCCTAGGGAACCCCAG GTTTACACCCTGCCTCCAAGCCAAGAGGAAATGACCAAGAACCA GGTGTCCCTGACCTGCCTGGTCAAGGGCTTCTACCCTTCCGATA TCGCCGTGGAATGGGAGAGCAATGGCCAGCCTGAGAACAACTAC AAGACCACACCTCCTGTGCTGGACTCCGACGGCTCCTTCTTTCT GTACTCCCGCCTGACCGTGGACAAGTCCAGATGGCAAGAGGGCA ACGTGTTCTCCTGCTCTGTGCTGCACGAGGCCCTGCACAATCAC TACACCCAGAAGTCCCTGTCTCTGTCCCTTGGCGGTGGCGGAGG ATCTGGCGGAGGCGGATCTATTACATGCCCTCCTCCAATGTCCG TGGAACACGCCGACATCTGGGTCAAGTCCTACAGCCTGTACTCC AGAGAGCGGTACATCTGCAACTCCGGCTTCAAGAGAAAGGCCGG CACCTCTAGCCTGACCGAGTGCGTGCTGAACAAGGCCACCAATG TGGCCCACTGGACAACCCCTAGCCTGAAGTGTATTAGAGGCGGC GGAGGAAGCGGTGGCGGCGGTAGCGGAGGTGGTGGTTCTGGTGG TGGCGGTTCTGCTCCTACCTCCTCCAGCACCAAGAAAACCCAGC TGCAGTTGGAGCATCTGCTGCTGGACCTGCAGATGATCCTGAAC GGCTCCATGCACATCGACGCTACCCTGTACACCGAGTCCGACGT GCACCCTTCCTGTAAAGTGACCGCCATGAAGTGCTTTCTGCTCG AGCTGCAAGTGATCTCCCTGGAATCCAAGAACTTCCACCTGAGG CCTCGGGACCTGATCGACAACATCAACCTGATCGTGCTGGAACT GAAGGGCTCCAACGGCAACGTGACCGAGTCTGGCTGTAAAGAGT GCGAGGAACTGGAAGAGAAGAACATCGTCGAGTTTCTGAACCGG TGGATCACCTTCTGCCAGTCCATCATCTCCACACTGACC | 153 |

(continued)

| Fusion protein (IL-2/IL-15 variant) (variation ) | Nucleic acid sequence | SEQ ID NO: |
|---|---|---|
| GIC-982C20 (L74F, S76D, N77D) | GGATCCGCCGAGTCTAAGTACGGCCCTCCTTGTCCTCCATGTCC<br>TGCTCCAGAAGCTGCCGGAGGGCCCAGTGTGTTTCTGTTCCCTC<br>CAAAGCCTAAGGACCAGCTGATGATCTCTCGGACCCCTGAAGTG<br>ACCTGCGTGGTGGTGGATGTGTCCCAAGAGGACCCTGAGGTGCA<br>GTTCAATTGGTACGTGGACGGCGTGGAAGTGCACAACGCCAAGA<br>CCAAGCCTAGAGAGGAACAGTTCAACTCCACCTACAGAGTGGTG<br>TCCGTGCTGACCGTGCTGCACCAGGATTGGCTGAACGGCAAAGA<br>GTACAAGTGCAAGGTGTCCAACAAGGGCCTGCCTTCCAGCATCG<br>AAAAGACCATCTCCAAGGCCAAGGGCCAGCCTAGGGAACCCCAG<br>GTTTACACCCTGCCTCCAAGCCAAGAGGAAATGACCAAGAACCA<br>GGTGTCCCTGACCTGCCTGGTCAAGGGCTTCTACCCTTCCGATA<br>TCGCCGTGGAATGGGAGAGCAATGGCCAGCCTGAGAACAACTAC<br>AAGACCACACCTCCTGTGCTGGACTCCGACGGCTCCTTCTTTCT<br>GTACTCCCGCCTGACCGTGGACAAGTCCAGATGGCAAGAGGGCA<br>ACGTGTTCTCCTGCTCTGTGCTGCACGAGGCCCTGCACAATCAC<br>TACACCCAGAAGTCCCTGTCTCTGTCCCTTGGCGGTGGCGGAGG<br>ATCTGGCGGAGGCGGATCTATTACATGCCCTCCTCCAATGTCCG<br>TGGAACACGCCGACATCTGGGTCAAGTCCTACAGCCTGTACTCC<br>AGAGAGCGGTACATCTGCAACTCCGGCTTCAAGAGAAAGGCCGG<br>CACCTCTAGCCTGACCGAGTGCGTGCTGAACAAGGCCACCAATG<br>TGGCCCACTGGACAACCCCTAGCCTGAAGTGTATTAGAGGCGGC<br>GGAGGAAGCGGTGGCGGCGGTAGCGGAGGTGGTGGTTCTGGTGG<br>TGGCGGTTCTGCTCCTACCTCCTCCAGCACCAAGAAAACCCAGC<br>TGCAGTTGGAGCATCTGCTGCTGGACCTGCAGATGATCCTGAAC<br>GGCTCCATGCACATCGACGCTACCCTGTACACCGAGTCCGACGT<br>GCACCCTTCCTGTAAAGTGACCGCCATGAAGTGCTTTCTGCTCG<br>AGCTGCAAGTGATCTCCCTGGAATCCAAGAACTTCCACCTGAGG<br>CCTCGGGACTTCATCGACGACATCAACGTGATCGTGCTGGAACT<br>GAAGGGCTCCAACGGCAACGTGACCGAGTCTGGCTGTAAAGAGT<br>GCGAGGAACTGGAAGAGAAGAACATCGTCGAGTTTCTGAACCGG<br>TGGATCACCTTCTGCCAGTCCATCATCTCCACACTGACC | 154 |
| GIC-982C21 (S76D, N77D, V80L) | GGATCCGCCGAGTCTAAGTACGGCCCTCCTTGTCCTCCATGTCC<br>TGCTCCAGAAGCTGCCGGAGGGCCCAGTGTGTTTCTGTTCCCTC<br>CAAAGCCTAAGGACCAGCTGATGATCTCTCGGACCCCTGAAGTG | 155 |

(continued)

| Fusion protein (IL-2/IL-15 variant) (variation ) | Nucleic acid sequence | SEQ ID NO: |
|---|---|---|
| | ACCTGCGTGGTGGTGGATGTGTCCCAAGAGGACCCTGAGGTGCA GTTCAATTGGTACGTGGACGGCGTGGAAGTGCACAACGCCAAGA CCAAGCCTAGAGAGGAACAGTTCAACTCCACCTACAGAGTGGTG TCCGTGCTGACCGTGCTGCACCAGGATTGGCTGAACGGCAAAGA GTACAAGTGCAAGGTGTCCAACAAGGGCCTGCCTTCCAGCATCG AAAAGACCATCTCCAAGGCCAAGGGCCAGCCTAGGGAACCCCAG GTTTACACCCTGCCTCCAAGCCAAGAGGAAATGACCAAGAACCA GGTGTCCCTGACCTGCCTGGTCAAGGGCTTCTACCCTTCCGATA TCGCCGTGGAATGGGAGAGCAATGGCCAGCCTGAGAACAACTAC AAGACCACACCTCCTGTGCTGGACTCCGACGGCTCCTTCTTTCT GTACTCCCGCCTGACCGTGGACAAGTCCAGATGGCAAGAGGGCA ACGTGTTCTCCTGCTCTGTGCTGCACGAGGCCCTGCACAATCAC TACACCCAGAAGTCCCTGTCTCTGTCCCTTGGCGGTGGCGGAGG ATCTGGCGGAGGCGGATCTATTACATGCCCTCCTCCAATGTCCG TGGAACACGCCGACATCTGGGTCAAGTCCTACAGCCTGTACTCC AGAGAGCGGTACATCTGCAACTCCGGCTTCAAGAGAAAGGCCGG CACCTCTAGCCTGACCGAGTGCGTGCTGAACAAGGCCACCAATG TGGCCCACTGGACAACCCCTAGCCTGAAGTGTATTAGAGGCGGC GGAGGAAGCGGTGGCGGCGGTAGCGGAGGTGGTGGTTCTGGTGG TGGCGGTTCTGCTCCTACCTCCTCCAGCACCAAGAAAACCCAGC TGCAGTTGGAGCATCTGCTGCTGGACCTGCAGATGATCCTGAAC GGCTCCATGCACATCGACGCTACCCTGTACACCGAGTCCGACGT GCACCCTTCCTGTAAAGTGACCGCCATGAAGTGCTTTCTGCTCG AGCTGCAAGTGATCTCCCTGGAATCCAAGAACTTCCACCTGAGG CCTCGGGACCTGATCGACGACATCAACCTGATCGTGCTGGAACT GAAGGGCTCCAACGGCAACGTGACCGAGTCTGGCTGTAAAGAGT GCGAGGAACTGGAAGAGAAGAACATCGTCGAGTTTCTGAACCGG TGGATCACCTTCTGCCAGTCCATCATCTCCACACTGACC | |

(continued)

| Fusion protein (IL-2/IL-15 variant) (variation ) | Nucleic acid sequence | SEQ ID NO: |
|---|---|---|
| GIC-982C22 (L74F, S76D, V80L) | GGATCCGCCGAGTCTAAGTACGGCCCTCCTTGTCCTCCATGTCC TGCTCCAGAAGCTGCCGGAGGGCCCAGTGTGTTTCTGTTCCCTC CAAAGCCTAAGGACCAGCTGATGATCTCTCGGACCCCTGAAGTG ACCTGCGTGGTGGTGGATGTGTCCCAAGAGGACCCTGAGGTGCA GTTCAATTGGTACGTGGACGGCGTGGAAGTGCACAACGCCAAGA CCAAGCCTAGAGAGGAACAGTTCAACTCCACCTACAGAGTGGTG TCCGTGCTGACCGTGCTGCACCAGGATTGGCTGAACGGCAAAGA GTACAAGTGCAAGGTGTCCAACAAGGGCCTGCCTTCCAGCATCG AAAAGACCATCTCCAAGGCCAAGGGCCAGCCTAGGGAACCCCAG GTTTACACCCTGCCTCCAAGCCAAGAGGAAATGACCAAGAACCA GGTGTCCCTGACCTGCCTGGTCAAGGGCTTCTACCCTTCCGATA TCGCCGTGGAATGGGAGAGCAATGGCCAGCCTGAGAACAACTAC AAGACCACACCTCCTGTGCTGGACTCCGACGGCTCCTTCTTTCT GTACTCCCGCCTGACCGTGGACAAGTCCAGATGGCAAGAGGGCA ACGTGTTCTCCTGCTCTGTGCTGCACGAGGCCCTGCACAATCAC TACACCCAGAAGTCCCTGTCTCTGTCCCTTGGCGGTGGCGGAGG ATCTGGCGGAGGCGGATCTATTACATGCCCTCCTCCAATGTCCG TGGAACACGCCGACATCTGGGTCAAGTCCTACAGCCTGTACTCC AGAGAGCGGTACATCTGCAACTCCGGCTTCAAGAGAAAGGCCGG CACCTCTAGCCTGACCGAGTGCGTGCTGAACAAGGCCACCAATG TGGCCCACTGGACAACCCCTAGCCTGAAGTGTATTAGAGGCGGC GGAGGAAGCGGTGGCGGCGGTAGCGGAGGTGGTGGTTCTGGTGG TGGCGGTTCTGCTCCTACCTCCTCCAGCACCAAGAAAACCCAGC TGCAGTTGGAGCATCTGCTGCTGGACCTGCAGATGATCCTGAAC GGCTCCATGCACATCGACGCTACCCTGTACACCGAGTCCGACGT GCACCCTTCCTGTAAAGTGACCGCCATGAAGTGCTTTCTGCTCG AGCTGCAAGTGATCTCCCTGGAATCCAAGAACTTCCACCTGAGG CCTCGGGACTTCATCGACAACATCAACCTGATCGTGCTGGAACT GAAGGGCTCCAACGGCAACGTGACCGAGTCTGGCTGTAAAGAGT GCGAGGAACTGGAAGAGAAGAACATCGTCGAGTTTCTGAACCGG TGGATCACCTTCTGCCAGTCCATCATCTCCACACTGACC | 156 |

(continued)

| Fusion protein (IL-2/IL-15 variant) (variation ) | Nucleic acid sequence | SEQ ID NO: |
|---|---|---|
| **GIC-982N1** | GCTCCTACCTCCTCCAGCACCAAGAAAACCCAGCTGCAGTTGGA<br>GCATCTGCTGCTGGACCTGCAGATGATCCTGAACGGCTCCATGC<br>ACATCGACGCTACCCTGTACACCGAGTCCGACGTGCACCCTTCC<br>TGTAAAGTGACCGCCATGAAGTGCTTTCTGTTGGAGCTGCAAGT<br>GATCTCCCTGGAATCCAAGAACTTCCACCTGAGGCCTCGGGACC<br>TGATCTCCAACATCAACGTGATCGTGCTGGAACTGAAGGGCTCC<br>AACGGCAACGTGACCGAGTCTGGCTGTAAAGAGTGCGAGGAACT<br>GGAAGAGAAGAACATCGTCGAGTTTCTGAACCGGTGGATCACCT<br>TCTGCCAGTCCATCATCTCCACACTGACCGGCGGCGGAGGAAGC<br>GGTGGCGGCGGTAGCGGAGGTGGTGGTTCTGGTGGTGGCGGTTC<br>TATTACATGCCCTCCTCCAATGTCCGTGGAACACGCCGACATCT<br>GGGTCAAGTCCTACAGCCTGTACTCCAGAGAGCGGTACATCTGC<br>AACTCCGGCTTCAAGAGAAAGGCCGGCACCTCTAGCCTGACCGA<br>GTGCGTGCTGAACAAGGCCACCAATGTGGCCCACTGGACAACCC<br>CTAGCCTGAAGTGTATTAGAGGTGGCGGAGGATCTGGCGGAGGC<br>GGATCTGGATCCGCCGAGTCTAAGTACGGCCCTCCTTGTCCTCC<br>ATGTCCTGCTCCAGAAGCTGCCGGAGGGCCCAGTGTGTTTCTGT<br>TCCCTCCAAAGCCTAAGGACCAGCTGATGATCTCTCGGACCCCT<br>GAAGTGACCTGCGTGGTGGTGGATGTGTCCCAAGAGGACCCTGA<br>GGTGCAGTTCAATTGGTACGTGGACGGCGTGGAAGTGCACAACG<br>CCAAGACCAAGCCTAGAGAGGAACAGTTCAACTCCACCTACAGA<br>GTGGTGTCCGTGCTGACCGTGCTGCACCAGGATTGGCTGAACGG<br>CAAAGAGTACAAGTGCAAGGTGTCCAACAAGGGCCTGCCTTCCA<br>GCATCGAAAAGACCATCTCCAAGGCCAAGGGCCAGCCTAGGGAA<br>CCCCAGGTTTACACCCTGCCTCCAAGCCAAGAGGAAATGACCAA<br>GAACCAGGTGTCCCTGACCTGCCTGGTCAAGGGCTTCTACCCTT<br>CCGATATCGCCGTGGAATGGGAGAGCAATGGCCAGCCTGAGAAC<br>AACTACAAGACCACACCTCCTGTGCTGGACTCCGACGGCTCCTT<br>CTTTCTGTACTCCCGCCTGACCGTGGACAAGTCCAGATGGCAAG<br>AGGGCAACGTGTTCTCCTGCTCTGTGCTGCACGAGGCCCTGCAC<br>AATCACTACACCCAGAAGTCCCTGTCTCTGTCCCTTGGC | 157 |
| **GIC-982N2 (D73E)** | GCTCCTACCTCCTCCAGCACCAAGAAAACCCAGCTGCAGTTGGA<br>GCATCTGCTGCTGGACCTGCAGATGATCCTGAACGGCTCCATGC<br>ACATCGACGCTACCCTGTACACCGAGTCCGACGTGCACCCTTCC<br>TGTAAAGTGACCGCCATGAAGTGCTTTCTGCTCGAGCTGCAAGT<br>GATCTCCCTGGAATCCAAGAACTTCCACCTGAGGCCAAGAGAGC<br>TGATCTCCAACATCAACGTGATCGTGCTGGAACTGAAGGGCTCC | 158 |

(continued)

| Fusion protein (IL-2/IL-15 variant) (variation ) | Nucleic acid sequence | SEQ ID NO: |
|---|---|---|
| | AACGGCAACGTGACCGAGTCTGGCTGTAAAGAGTGCGAGGAACT GGAAGAGAAGAACATCGTCGAGTTTCTGAACCGGTGGATCACCT TCTGCCAGTCCATCATCTCCACACTGACCGGCGGCGGAGGAAGC GGTGGCGGCGGTAGCGGAGGTGGTGGTTCTGGTGGTGGCGGTTC TATTACATGCCCTCCTCCAATGTCCGTGGAACACGCCGACATCT GGGTCAAGTCCTACAGCCTGTACTCCAGAGAGCGGTACATCTGC AACTCCGGCTTCAAGAGAAAGGCCGGCACCTCTAGCCTGACCGA GTGCGTGCTGAACAAGGCCACCAATGTGGCCCACTGGACAACCC CTAGCCTGAAGTGTATTAGAGGTGGCGGAGGATCTGGCGGAGGC GGATCTGGATCCGCCGAGTCTAAGTACGGCCCTCCTTGTCCTCC ATGTCCTGCTCCAGAAGCTGCCGGAGGGCCCAGTGTGTTTCTGT TCCCTCCAAAGCCTAAGGACCAGCTGATGATCTCTCGGACCCCT GAAGTGACCTGCGTGGTGGTGGATGTGTCCCAAGAGGACCCTGA GGTGCAGTTCAATTGGTACGTGGACGGCGTGGAAGTGCACAACG CCAAGACCAAGCCTAGAGAGGAACAGTTCAACTCCACCTACAGA GTGGTGTCCGTGCTGACCGTGCTGCACCAGGATTGGCTGAACGG CAAAGAGTACAAGTGCAAGGTGTCCAACAAGGGCCTGCCTTCCA GCATCGAAAAGACCATCTCCAAGGCCAAGGGCCAGCCTAGGGAA CCCCAGGTTTACACCCTGCCTCCAAGCCAAGAGGAAATGACCAA GAACCAGGTGTCCCTGACCTGCCTGGTCAAGGGCTTCTACCCTT CCGATATCGCCGTGGAATGGGAGAGCAATGGCCAGCCTGAGAAC AACTACAAGACCACACCTCCTGTGCTGGACTCCGACGGCTCCTT CTTTCTGTACTCCCGCCTGACCGTGGACAAGTCCAGATGGCAAG AGGGCAACGTGTTCTCCTGCTCTGTGCTGCACGAGGCCCTGCAC AATCACTACACCCAGAAGTCCCTGTCTCTGTCCCTTGGC | |

(continued)

| Fusion protein (IL-2/IL-15 variant) (variation ) | Nucleic acid sequence | SEQ ID NO: |
|---|---|---|
| GIC-982N3 (L74F) | GCTCCTACCTCCTCCAGCACCAAGAAAACCCAGCTGCAGTTGGAGCATCTGCTGCTGGACCTGCAGATGATCCTGAACGGCTCCATGCACATCGACGCTACCCTGTACACCGAGTCCGACGTGCACCCTTCCTGTAAAGTGACCGCCATGAAGTGCTTTCTGCTCGAGCTGCAAGTGATCTCCCTGGAATCCAAGAACTTCCACCTGAGGCCTCGGGACTTCATCTCCAACATCAACGTGATCGTGCTGGAACTGAAGGGCTCCAACGGCAACGTGACCGAGTCTGGCTGTAAAGAGTGCGAGGAACTGGAAGAGAAGAACATCGTCGAGTTTCTGAACCGGTGGATCACCTTCTGCCAGTCCATCATCTCCACACTGACCGGCGGCGGAGGAAGCGGTGGCGGCGGTAGCGGAGGTGGTGGTTCTGGTGGTGGCGGTTCTATTACATGCCCTCCTCCAATGTCCGTGGAACACGCCGACATCTGGGTCAAGTCCTACAGCCTGTACTCCAGAGAGCGGTACATCTGCAACTCCGGCTTCAAGAGAAAGGCCGGCACCTCTAGCCTGACCGAGTGCGTGCTGAACAAGGCCACCAATGTGGCCCACTGGACAACCCCTAGCCTGAAGTGTATTAGAGGTGGCGGAGGATCTGGCGGAGGCGGATCTGGATCCGCCGAGTCTAAGTACGGCCCTCCTTGTCCTCCATGTCCTGCTCCAGAAGCTGCCGGAGGGCCCAGTGTGTTTCTGTTCCCTCCAAAGCCTAAGGACCAGCTGATGATCTCTCGGACCCCTGAAGTGACCTGCGTGGTGGTGGATGTGTCCCAAGAGGACCCTGAGGTGCAGTTCAATTGGTACGTGGACGGCGTGGAAGTGCACAACGCCAAGACCAAGCCTAGAGAGGAACAGTTCAACTCCACCTACAGAGTGGTGTCCGTGCTGACCGTGCTGCACCAGGATTGGCTGAACGGCAAAGAGTACAAGTGCAAGGTGTCCAACAAGGGCCTGCCTTCCAGCATCGAAAAGACCATCTCCAAGGCCAAGGGCCAGCCTAGGGAACCCCAGGTTTACACCCTGCCTCCAAGCCAAGAGGAAATGACCAAGAACCAGGTGTCCCTGACCTGCCTGGTCAAGGGCTTCTACCCTTCCGATATCGCCGTGGAATGGGAGAGCAATGGCCAGCCTGAGAACAACTACAAGACCACACCTCCTGTGCTGGACTCCGACGGCTCCTTCTTTCTGTACTCCCGCCTGACCGTGGACAAGTCCAGATGGCAAGAGGGCAACGTGTTCTCCTGCTCTGTGCTGCACGAGGCCCTGCACAATCACTACACCCAGAAGTCCCTGTCTCTGTCCCTTGGC | 159 |

(continued)

| Fusion protein (IL-2/IL-15 variant) (variation ) | Nucleic acid sequence | SEQ ID NO: |
|---|---|---|
| GIC-982N4 (S76D) | GCTCCTACCTCCTCCAGCACCAAGAAAACCCAGCTGCAGTTGGA<br>GCATCTGCTGCTGGACCTGCAGATGATCCTGAACGGCTCCATGC<br>ACATCGACGCTACCCTGTACACCGAGTCCGACGTGCACCCTTCC<br>TGTAAAGTGACCGCCATGAAGTGCTTTCTGCTCGAGCTGCAAGT<br>GATCTCCCTGGAATCCAAGAACTTCCACCTGAGGCCTCGGGACC<br>TGATCGACAACATCAACGTGATCGTGCTGGAACTGAAGGGCTCC<br>AACGGCAACGTGACCGAGTCTGGCTGTAAAGAGTGCGAGGAACT<br>GGAAGAGAAGAACATCGTCGAGTTTCTGAACCGGTGGATCACCT<br>TCTGCCAGTCCATCATCTCCACACTGACCGGCGGCGGAGGAAGC<br>GGTGGCGGCGGTAGCGGAGGTGGTGGTTCTGGTGGTGGCGGTTC<br>TATTACATGCCCTCCTCCAATGTCCGTGGAACACGCCGACATCT<br>GGGTCAAGTCCTACAGCCTGTACTCCAGAGAGCGGTACATCTGC<br>AACTCCGGCTTCAAGAGAAAGGCCGGCACCTCTAGCCTGACCGA<br>GTGCGTGCTGAACAAGGCCACCAATGTGGCCCACTGGACAACCC<br>CTAGCCTGAAGTGTATTAGAGGTGGCGGAGGATCTGGCGGAGGC<br>GGATCTGGATCCGCCGAGTCTAAGTACGGCCCTCCTTGTCCTCC<br>ATGTCCTGCTCCAGAAGCTGCCGGAGGGCCCAGTGTGTTTCTGT<br>TCCCTCCAAAGCCTAAGGACCAGCTGATGATCTCTCGGACCCCT<br>GAAGTGACCTGCGTGGTGGTGGATGTGTCCCAAGAGGACCCTGA<br>GGTGCAGTTCAATTGGTACGTGGACGGCGTGGAAGTGCACAACG<br>CCAAGACCAAGCCTAGAGAGGAACAGTTCAACTCCACCTACAGA<br>GTGGTGTCCGTGCTGACCGTGCTGCACCAGGATTGGCTGAACGG<br>CAAAGAGTACAAGTGCAAGGTGTCCAACAAGGGCCTGCCTTCCA<br>GCATCGAAAAGACCATCTCCAAGGCCAAGGGCCAGCCTAGGGAA<br>CCCCAGGTTTACACCCTGCCTCCAAGCCAAGAGGAAATGACCAA<br>GAACCAGGTGTCCCTGACCTGCCTGGTCAAGGGCTTCTACCCTT<br>CCGATATCGCCGTGGAATGGGAGAGCAATGGCCAGCCTGAGAAC<br>AACTACAAGACCACACCTCCTGTGCTGGACTCCGACGGCTCCTT<br>CTTTCTGTACTCCCGCCTGACCGTGGACAAGTCCAGATGGCAAG<br>AGGGCAACGTGTTCTCCTGCTCTGTGCTGCACGAGGCCCTGCAC<br>AATCACTACACCCAGAAGTCCCTGTCTCTGTCCCTTGGC | 160 |
| GIC-982N5 (N77D) | GCTCCTACCTCCTCCAGCACCAAGAAAACCCAGCTGCAGTTGGA<br>GCATCTGCTGCTGGACCTGCAGATGATCCTGAACGGCTCCATGC<br>ACATCGACGCTACCCTGTACACCGAGTCCGACGTGCACCCTTCC<br>TGTAAAGTGACCGCCATGAAGTGCTTTCTGCTCGAGCTGCAAGT<br>GATCTCCCTGGAATCCAAGAACTTCCACCTGAGGCCTCGGGACC<br>TGATCTCC**GAC**ATCAACGTGATCGTGCTGGAACTGAAGGGCTCC<br>AACGGCAACGTGACCGAGTCTGGCTGTAAAGAGTGCGAGGAACT<br>GGAAGAGAAGAACATCGTCGAGTTTCTGAACCGGTGGATCACCT<br>TCTGCCAGTCCATCATCTCCACACTGACCGGCGGCGGAGGAAGC | 161 |

(continued)

| Fusion protein (IL-2/IL-15 variant) (variation ) | Nucleic acid sequence | SEQ ID NO: |
|---|---|---|
|  | GGTGGCGGCGGTAGCGGAGGTGGTGGTTCTGGTGGTGGCGGTTC TATTACATGCCCTCCTCCAATGTCCGTGGAACACGCCGACATCT GGGTCAAGTCCTACAGCCTGTACTCCAGAGAGCGGTACATCTGC AACTCCGGCTTCAAGAGAAAGGCCGGCACCTCTAGCCTGACCGA GTGCGTGCTGAACAAGGCCACCAATGTGGCCCACTGGACAACCC CTAGCCTGAAGTGTATTAGAGGTGGCGGAGGATCTGGCGGAGGC GGATCTGGATCCGCCGAGTCTAAGTACGGCCCTCCTTGTCCTCC ATGTCCTGCTCCAGAAGCTGCCGGAGGGCCCAGTGTGTTTCTGT TCCCTCCAAAGCCTAAGGACCAGCTGATGATCTCTCGGACCCCT GAAGTGACCTGCGTGGTGGTGGATGTGTCCCAAGAGGACCCTGA GGTGCAGTTCAATTGGTACGTGGACGGCGTGGAAGTGCACAACG CCAAGACCAAGCCTAGAGAGGAACAGTTCAACTCCACCTACAGA GTGGTGTCCGTGCTGACCGTGCTGCACCAGGATTGGCTGAACGG CAAAGAGTACAAGTGCAAGGTGTCCAACAAGGGCCTGCCTTCCA GCATCGAAAAGACCATCTCCAAGGCCAAGGGCCAGCCTAGGGAA CCCCAGGTTTACACCCTGCCTCCAAGCCAAGAGGAAATGACCAA GAACCAGGTGTCCCTGACCTGCCTGGTCAAGGGCTTCTACCCTT CCGATATCGCCGTGGAATGGGAGAGCAATGGCCAGCCTGAGAAC AACTACAAGACCACACCTCCTGTGCTGGACTCCGACGGCTCCTT CTTTCTGTACTCCCGCCTGACCGTGGACAAGTCCAGATGGCAAG AGGGCAACGTGTTCTCCTGCTCTGTGCTGCACGAGGCCCTGCAC AATCACTACACCCAGAAGTCCCTGTCTCTGTCCCTTGGC |  |

65

(continued)

| Fusion protein (IL-2/IL-15 variant) (variation ) | Nucleic acid sequence | SEQ ID NO: |
|---|---|---|
| GIC-982N6 (V80L) | GCTCCTACCTCCTCCAGCACCAAGAAAACCCAGCTGCAGTTGGAGCATCTGCTGCTGGACCTGCAGATGATCCTGAACGGCTCCATGCACATCGACGCTACCCTGTACACCGAGTCCGACGTGCACCCTTCCTGTAAAGTGACCGCCATGAAGTGCTTTCTGCTCGAGCTGCAAGTGATCTCCCTGGAATCCAAGAACTTCCACCTGAGGCCTCGGGACCTGATCTCCAACATCAACCTGATCGTGCTGGAACTGAAGGGCTCCAACGGCAACGTGACCGAGTCTGGCTGTAAAGAGTGCGAGGAACTGGAAGAGAAGAACATCGTCGAGTTTCTGAACCGGTGGATCACCTTCTGCCAGTCCATCATCTCCACACTGACCGGCGGCGGAGGAAGCGGTGGCGGCGGTAGCGGAGGTGGTGGTTCTGGTGGTGGCGGTTCTATTACATGCCCTCCTCCAATGTCCGTGGAACACGCCGACATCTGGGTCAAGTCCTACAGCCTGTACTCCAGAGAGCGGTACATCTGCAACTCCGGCTTCAAGAGAAAGGCCGGCACCTCTAGCCTGACCGAGTGCGTGCTGAACAAGGCCACCAATGTGGCCCACTGGACAACCCCTAGCCTGAAGTGTATTAGAGGTGGCGGAGGATCTGGCGGAGGCGGATCTGGATCCGCCGAGTCTAAGTACGGCCCTCCTTGTCCTCCATGTCCTGCTCCAGAAGCTGCCGGAGGGCCCAGTGTGTTTCTGTTCCCTCCAAAGCCTAAGGACCAGCTGATGATCTCTCGGACCCCTGAAGTGACCTGCGTGGTGGTGGATGTGTCCCAAGAGGACCCTGAGGTGCAGTTCAATTGGTACGTGGACGGCGTGGAAGTGCACAACGCCAAGACCAAGCCTAGAGAGGAACAGTTCAACTCCACCTACAGAGTGGTGTCCGTGCTGACCGTGCTGCACCAGGATTGGCTGAACGGCAAAGAGTACAAGTGCAAGGTGTCCAACAAGGGCCTGCCTTCCAGCATCGAAAAGACCATCTCCAAGGCCAAGGGCCAGCCTAGGGAACCCCAGGTTTACACCCTGCCTCCAAGCCAAGAGGAAATGACCAAGAACCAGGTGTCCCTGACCTGCCTGGTCAAGGGCTTCTACCCTTCCGATATCGCCGTGGAATGGGAGAGCAATGGCCAGCCTGAGAACAACTACAAGACCACACCTCCTGTGCTGGACTCCGACGGCTCCTTCTTTCTGTACTCCCGCCTGACCGTGGACAAGTCCAGATGGCAAGAGGGCAACGTGTTCTCCTGCTCTGTGCTGCACGAGGCCCTGCACAATCACTACACCCAGAAGTCCCTGTCTCTGTCCCTTGGC | 162 |

(continued)

| Fusion protein (IL-2/IL-15 variant) (variation ) | Nucleic acid sequence | SEQ ID NO: |
|---|---|---|
| GIC-982N7 (L74F, S76D, N77D, V80L) | GCTCCTACCTCCTCCAGCACCAAGAAAACCCAGCTGCAGTTGGAGCATCTGCTGCTGGACCTGCAGATGATCCTGAACGGCTCCATGCACATCGACGCTACCCTGTACACCGAGTCCGACGTGCACCCTTCCTGTAAAGTGACCGCCATGAAGTGCTTTCTGCTCGAGCTGCAAGTGATCTCCCTGGAATCCAAGAACTTCCACCTGAGGCCTCGGGACTTCATCGACGACATCAACCTGATCGTGCTGGAACTGAAGGGCTCCAACGGCAACGTGACCGAGTCTGGCTGTAAAGAGTGCGAGGAACTGGAAGAGAAGAACATCGTCGAGTTTCTGAACCGGTGGATCACCTTCTGCCAGTCCATCATCTCCACACTGACCGGCGGCGGAGGAAGCGGTGGCGGCGGTAGCGGAGGTGGTGGTTCTGGTGGTGGCGGTTCTATTACATGCCCTCCTCCAATGTCCGTGGAACACGCCGACATCTGGGTCAAGTCCTACAGCCTGTACTCCAGAGAGCGGTACATCTGCAACTCCGGCTTCAAGAGAAAGGCCGGCACCTCTAGCCTGACCGAGTGCGTGCTGAACAAGGCCACCAATGTGGCCCACTGGACAACCCCTAGCCTGAAGTGTATTAGAGGTGGCGGAGGATCTGGCGGAGGCGGATCTGGATCCGCCGAGTCTAAGTACGGCCCTCCTTGTCCTCCATGTCCTGCTCCAGAAGCTGCCGGAGGGCCCAGTGTGTTTCTGTTCCCTCCAAAGCCTAAGGACCAGCTGATGATCTCTCGGACCCCTGAAGTGACCTGCGTGGTGGTGGATGTGTCCCAAGAGGACCCTGAGGTGCAGTTCAATTGGTACGTGGACGGCGTGGAAGTGCACAACGCCAAGACCAAGCCTAGAGAGGAACAGTTCAACTCCACCTACAGAGTGGTGTCCGTGCTGACCGTGCTGCACCAGGATTGGCTGAACGGCAAAGAGTACAAGTGCAAGGTGTCCAACAAGGGCCTGCCTTCCAGCATCGAAAAGACCATCTCCAAGGCCAAGGGCCAGCCTAGGGAACCCCAGGTTTACACCCTGCCTCCAAGCCAAGAGGAAATGACCAAGAACCAGGTGTCCCTGACCTGCCTGGTCAAGGGCTTCTACCCTTCCGATATCGCCGTGGAATGGGAGAGCAATGGCCAGCCTGAGAACAACTACAAGACCACACCTCCTGTGCTGGACTCCGACGGCTCCTTCTTTCTGTACTCCCGCCTGACCGTGGACAAGTCCAGATGGCAAGAGGGCAACGTGTTCTCCTGCTCTGTGCTGCACGAGGCCCTGCACAATCACTACACCCAGAAGTCCCTGTCTCTGTCCCTTGGC | 163 |
| GIC-982N8 (D73E, S76D, N77D, V80L) | GCTCCTACCTCCTCCAGCACCAAGAAAACCCAGCTGCAGTTGGAGCATCTGCTGCTGGACCTGCAGATGATCCTGAACGGCTCCATGCACATCGACGCTACCCTGTACACCGAGTCCGACGTGCACCCTTCCTGTAAAGTGACCGCCATGAAGTGCTTTCTGCTCGAGCTGCAAGTGATCTCCCTGGAATCCAAGAACTTCCACCTGAGGCCAAGAGAGCTGATCGACGACATCAACCTGATCGTGCTGGAACTGAAGGGCTCCAACGGCAACGTGACCGAGTCTGGCTGTAAAGAGTGCGAGGAACTGGAAGAGAAGAACATCGTCGAGTTTCTGAACCGGTGGATCACCTTCTGCCAGTCCATCATCTCCACACTGACCGGCGGCGGAGGAAGCGGTGGCGGCGGTAGCGGAGGTGGTGGTTCTGGTGGTGGCGGTTCTATTACATGCCCTCCTCCAATGTCCGTGGAACACGCCGACATCTGGGTCAAGTCCTACAGCCTGTACTCCAGAGAGCGGTACATCTGC | 164 |

(continued)

| Fusion protein (IL-2/IL-15 variant) (variation ) | Nucleic acid sequence | SEQ ID NO: |
|---|---|---|
| | AACTCCGGCTTCAAGAGAAAGGCCGGCACCTCTAGCCTGACCGA<br>GTGCGTGCTGAACAAGGCCACCAATGTGGCCCACTGGACAACCC<br>CTAGCCTGAAGTGTATTAGAGGTGGCGGAGGATCTGGCGGAGGC<br>GGATCTGGATCCGCCGAGTCTAAGTACGGCCCTCCTTGTCCTCC<br>ATGTCCTGCTCCAGAAGCTGCCGGAGGGCCCAGTGTGTTTCTGT<br>TCCCTCCAAAGCCTAAGGACCAGCTGATGATCTCTCGGACCCCT<br>GAAGTGACCTGCGTGGTGGTGGATGTGTCCCAAGAGGACCCTGA<br>GGTGCAGTTCAATTGGTACGTGGACGGCGTGGAAGTGCACAACG<br>CCAAGACCAAGCCTAGAGAGGAACAGTTCAACTCCACCTACAGA<br>GTGGTGTCCGTGCTGACCGTGCTGCACCAGGATTGGCTGAACGG<br>CAAAGAGTACAAGTGCAAGGTGTCCAACAAGGGCCTGCCTTCCA<br>GCATCGAAAAGACCATCTCCAAGGCCAAGGGCCAGCCTAGGGAA<br>CCCCAGGTTTACACCCTGCCTCCAAGCCAAGAGGAAATGACCAA<br>GAACCAGGTGTCCCTGACCTGCCTGGTCAAGGGCTTCTACCCTT<br>CCGATATCGCCGTGGAATGGGAGAGCAATGGCCAGCCTGAGAAC<br>AACTACAAGACCACACCTCCTGTGCTGGACTCCGACGGCTCCTT<br>CTTTCTGTACTCCCGCCTGACCGTGGACAAGTCCAGATGGCAAG<br>AGGGCAACGTGTTCTCCTGCTCTGTGCTGCACGAGGCCCTGCAC<br>AATCACTACACCCAGAAGTCCCTGTCTCTGTCCCTTGGC | |

(continued)

| Fusion protein (IL-2/IL-15 variant) (variation ) | Nucleic acid sequence | SEQ ID NO: |
|---|---|---|
| GIC-982N9 (D73E, L74F, N77D, V80L) | GCTCCTACCTCCTCCAGCACCAAGAAAACCCAGCTGCAGTTGGAGCATCTGCTGCTGGACCTGCAGATGATCCTGAACGGCTCCATGCACATCGACGCTACCCTGTACACCGAGTCCGACGTGCACCCTTCCTGTAAAGTGACCGCCATGAAGTGCTTTCTGCTCGAGCTGCAAGTGATCTCCCTGGAATCCAAGAACTTCCACCTGAGGCCAAGAGAGTTCATCTCCGACATCAACCTGATCGTGCTGGAACTGAAGGGCTCCAACGGCAACGTGACCGAGTCTGGCTGTAAAGAGTGCGAGGAACTGGAAGAGAAGAACATCGTCGAGTTTCTGAACCGGTGGATCACCTTCTGCCAGTCCATCATCTCCACACTGACCGGCGGCGGAGGAAGCGGTGGCGGCGGTAGCGGAGGTGGTGGTTCTGGTGGTGGCGGTTCTATTACATGCCCTCCTCCAATGTCCGTGGAACACGCCGACATCTGGGTCAAGTCCTACAGCCTGTACTCCAGAGAGCGGTACATCTGCAACTCCGGCTTCAAGAGAAAGGCCGGCACCTCTAGCCTGACCGAGTGCGTGCTGAACAAGGCCACCAATGTGGCCCACTGGACAACCCCTAGCCTGAAGTGTATTAGAGGTGGCGGAGGATCTGGCGGAGGCGGATCTGGATCCGCCGAGTCTAAGTACGGCCCTCCTTGTCCTCCATGTCCTGCTCCAGAAGCTGCCGGAGGGCCCAGTGTGTTTCTGTTCCCTCCAAAGCCTAAGGACCAGCTGATGATCTCTCGGACCCCTGAAGTGACCTGCGTGGTGGTGGATGTGTCCCAAGAGGACCCTGAGGTGCAGTTCAATTGGTACGTGGACGGCGTGGAAGTGCACAACGCCAAGACCAAGCCTAGAGAGGAACAGTTCAACTCCACCTACAGAGTGGTGTCCGTGCTGACCGTGCTGCACCAGGATTGGCTGAACGGCAAAGAGTACAAGTGCAAGGTGTCCAACAAGGGCCTGCCTTCCAGCATCGAAAAGACCATCTCCAAGGCCAAGGGCCAGCCTAGGGAACCCCAGGTTTACACCCTGCCTCCAAGCCAAGAGGAAATGACCAAGAACCAGGTGTCCCTGACCTGCCTGGTCAAGGGCTTCTACCCTTCCGATATCGCCGTGGAATGGGAGAGCAATGGCCAGCCTGAGAACAACTACAAGACCACACCTCCTGTGCTGGACTCCGACGGCTCCTTCTTTCTGTACTCCCGCCTGACCGTGGACAAGTCCAGATGGCAAGAGGGCAACGTGTTCTCCTGCTCTGTGCTGCACGAGGCCCTGCACAATCACTACACCCAGAAGTCCCTGTCTCTGTCCCTTGGC | 165 |

(continued)

| Fusion protein (IL-2/IL-15 variant) (variation ) | Nucleic acid sequence | SEQ ID NO: |
|---|---|---|
| GIC-982N10 (D73E, L74F, S76D, V80L) | GCTCCTACCTCCTCCAGCACCAAGAAAACCCAGCTGCAGTTGGAGCATCTGCTGCTGGACCTGCAGATGATCCTGAACGGCTCCATGCACATCGACGCTACCCTGTACACCGAGTCCGACGTGCACCCTTCCTGTAAAGTGACCGCCATGAAGTGCTTTCTGCTCGAGCTGCAAGTGATCTCCCTGGAATCCAAGAACTTCCACCTGAGGCCAAGAGAGTTCATCGACAACATCAACCTGATCGTCCTGGAACTGAAGGGCTCCAACGGCAACGTGACCGAGTCTGGCTGTAAAGAGTGCGAGGAACTGGAAGAGAAGAACATCGTCGAGTTTCTGAACCGGTGGATCACCTTCTGCCAGTCCATCATCTCCACACTGACCGGCGGCGGAGGAAGCGGTGGCGGCGGTAGCGGAGGTGGTGGTTCTGGTGGTGGCGGTTCTATTACATGCCCTCCTCCAATGTCCGTGGAACACGCCGACATCTGGGTCAAGTCCTACAGCCTGTACTCCAGAGAGCGGTACATCTGCAACTCCGGCTTCAAGAGAAAGGCCGGCACCTCTAGCCTGACCGAGTGCGTGCTGAACAAGGCCACCAATGTGGCCCACTGGACAACCCCTAGCCTGAAGTGTATTAGAGGTGGCGGAGGATCTGGCGGAGGCGGATCTGGATCCGCCGAGTCTAAGTACGGCCCTCCTTGTCCTCCATGTCCTGCTCCAGAAGCTGCCGGAGGGCCCAGTGTGTTTCTGTTCCCTCCAAAGCCTAAGGACCAGCTGATGATCTCTCGGACCCCTGAAGTGACCTGCGTGGTGGTGGATGTGTCCCAAGAGGACCCTGAGGTGCAGTTCAATTGGTACGTGGACGGCGTGGAAGTGCACAACGCCAAGACCAAGCCTAGAGAGGAACAGTTCAACTCCACCTACAGAGTGGTGTCCGTGCTGACCGTGCTGCACCAGGATTGGCTGAACGGCAAAGAGTACAAGTGCAAGGTGTCCAACAAGGGCCTGCCTTCCAGCATCGAAAAGACCATCTCCAAGGCCAAGGGCCAGCCTAGGGAACCCCAGGTTTACACCCTGCCTCCAAGCCAAGAGGAAATGACCAAGAACCAGGTGTCCCTGACCTGCCTGGTCAAGGGCTTCTACCCTTCCGATATCGCCGTGGAATGGGAGAGCAATGGCCAGCCTGAGAACAACTACAAGACCACACCTCCTGTGCTGGACTCCGACGGCTCCTTCTTTCTGTACTCCCGCCTGACCGTGGACAAGTCCAGATGGCAAGAGGGCAACGTGTTCTCCTGCTCTGTGCTGCACGAGGCCCTGCACAATCACTACACCCAGAAGTCCCTGTCTCTGTCCCTTGGC | 166 |

(continued)

| Fusion protein (IL-2/IL-15 variant) (variation ) | Nucleic acid sequence | SEQ ID NO: |
|---|---|---|
| GIC-982N11 (D73E, L74F, S76D, N77D) | GCTCCTACCTCCTCCAGCACCAAGAAAACCCAGCTGCAGTTGGA GCATCTGCTGCTGGACCTGCAGATGATCCTGAACGGCTCCATGC ACATCGACGCTACCCTGTACACCGAGTCCGACGTGCACCCTTCC TGTAAAGTGACCGCCATGAAGTGCTTTCTGCTCGAGCTGCAAGT GATCTCCCTGGAATCCAAGAACTTCCACCTGAGGCCAAGAGAGT TCATCGACGACATCAACGTGATCGTGCTGGAACTGAAGGGCTCC AACGGCAACGTGACCGAGTCTGGCTGTAAAGAGTGCGAGGAACT GGAAGAGAAGAACATCGTCGAGTTTCTGAACCGGTGGATCACCT TCTGCCAGTCCATCATCTCCACACTGACCGGCGGCGGAGGAAGC GGTGGCGGCGGTAGCGGAGGTGGTGGTTCTGGTGGTGGCGGTTC TATTACATGCCCTCCTCCAATGTCCGTGGAACACGCCGACATCT GGGTCAAGTCCTACAGCCTGTACTCCAGAGAGCGGTACATCTGC AACTCCGGCTTCAAGAGAAAGGCCGGCACCTCTAGCCTGACCGA GTGCGTGCTGAACAAGGCCACCAATGTGGCCCACTGGACAACCC CTAGCCTGAAGTGTATTAGAGGTGGCGGAGGATCTGGCGGAGGC

GGATCTGGATCCGCCGAGTCTAAGTACGGCCCTCCTTGTCCTCC ATGTCCTGCTCCAGAAGCTGCCGGAGGGCCCAGTGTGTTTCTGT TCCCTCCAAAGCCTAAGGACCAGCTGATGATCTCTCGGACCCCT GAAGTGACCTGCGTGGTGGTGGATGTGTCCCAAGAGGACCCTGA GGTGCAGTTCAATTGGTACGTGGACGGCGTGGAAGTGCACAACG CCAAGACCAAGCCTAGAGAGGAACAGTTCAACTCCACCTACAGA GTGGTGTCCGTGCTGACCGTGCTGCACCAGGATTGGCTGAACGG CAAAGAGTACAAGTGCAAGGTGTCCAACAAGGGCCTGCCTTCCA GCATCGAAAAGACCATCTCCAAGGCCAAGGGCCAGCCTAGGGAA CCCCAGGTTTACACCCTGCCTCCAAGCCAAGAGGAAATGACCAA GAACCAGGTGTCCCTGACCTGCCTGGTCAAGGGCTTCTACCCTT CCGATATCGCCGTGGAATGGGAGAGCAATGGCCAGCCTGAGAAC AACTACAAGACCACACCTCCTGTGCTGGACTCCGACGGCTCCTT CTTTCTGTACTCCCGCCTGACCGTGGACAAGTCCAGATGGCAAG AGGGCAACGTGTTCTCCTGCTCTGTGCTGCACGAGGCCCTGCAC AATCACTACACCCAGAAGTCCCTGTCTCTGTCCCTTGGC | 167 |

(continued)

| Fusion protein (IL-2/IL-15 variant) (variation ) | Nucleic acid sequence | SEQ ID NO: |
|---|---|---|
| GIC-982N12 (D73E, L74F, S76D, N77D, V80L) | GCTCCTACCTCCTCCAGCACCAAGAAAACCCAGCTGCAGTTGGA GCATCTGCTGCTGGACCTGCAGATGATCCTGAACGGCTCCATGC ACATCGACGCTACCCTGTACACCGAGTCCGACGTGCACCCTTCC TGTAAAGTGACCGCCATGAAGTGCTTTCTGCTCGAGCTGCAAGT GATCTCCCTGGAATCCAAGAACTTCCACCTGAGGCCAAGAGAGT TCATCGACGACATCAACCTGATCGTGCTGGAACTGAAGGGCTCC AACGGCAACGTGACCGAGTCTGGCTGTAAAGAGTGCGAGGAACT GGAAGAGAAGAACATCGTCGAGTTTCTGAACCGGTGGATCACCT TCTGCCAGTCCATCATCTCCACACTGACCGGCGGCGGAGGAAGC GGTGGCGGCGGTAGCGGAGGTGGTGGTTCTGGTGGTGGCGGTTC TATTACATGCCCTCCTCCAATGTCCGTGGAACACGCCGACATCT GGGTCAAGTCCTACAGCCTGTACTCCAGAGAGCGGTACATCTGC AACTCCGGCTTCAAGAGAAAGGCCGGCACCTCTAGCCTGACCGA GTGCGTGCTGAACAAGGCCACCAATGTGGCCCACTGGACAACCC CTAGCCTGAAGTGTATTAGAGGTGGCGGAGGATCTGGCGGAGGC GGATCTGGATCCGCCGAGTCTAAGTACGGCCCTCCTTGTCCTCC ATGTCCTGCTCCAGAAGCTGCCGGAGGGCCCAGTGTGTTTCTGT TCCCTCCAAAGCCTAAGGACCAGCTGATGATCTCTCGGACCCCT GAAGTGACCTGCGTGGTGGTGGATGTGTCCCAAGAGGACCCTGA GGTGCAGTTCAATTGGTACGTGGACGGCGTGGAAGTGCACAACG CCAAGACCAAGCCTAGAGAGGAACAGTTCAACTCCACCTACAGA GTGGTGTCCGTGCTGACCGTGCTGCACCAGGATTGGCTGAACGG CAAAGAGTACAAGTGCAAGGTGTCCAACAAGGGCCTGCCTTCCA GCATCGAAAAGACCATCTCCAAGGCCAAGGGCCAGCCTAGGGAA CCCCAGGTTTACACCCTGCCTCCAAGCCAAGAGGAAATGACCAA GAACCAGGTGTCCCTGACCTGCCTGGTCAAGGGCTTCTACCCTT CCGATATCGCCGTGGAATGGGAGAGCAATGGCCAGCCTGAGAAC AACTACAAGACCACACCTCCTGTGCTGGACTCCGACGGCTCCTT CTTTCTGTACTCCCGCCTGACCGTGGACAAGTCCAGATGGCAAG AGGGCAACGTGTTCTCCTGCTCTGTGCTGCACGAGGCCCTGCAC AATCACTACACCCAGAAGTCCCTGTCTCTGTCCCTTGGC | 168 |

(continued)

| Fusion protein (IL-2/IL-15 variant) (variation ) | Nucleic acid sequence | SEQ ID NO: |
|---|---|---|
| GIC-982N13 (S76E) | GCTCCTACCTCCTCCAGCACCAAGAAAACCCAGCTGCAGTTGGAGCATCTGCTGCTGGACCTGCAGATGATCCTGAACGGCTCCATGCACATCGACGCTACCCTGTACACCGAGTCCGACGTGCACCCTTCCTGTAAAGTGACCGCCATGAAGTGCTTTCTGTTGGAGCTGCAAGTGATCTCCCTGGAATCCAAGAACTTCCACCTGAGGCCTCGGGACCTGATCGAAAACATCAACGTGATCGTGCTGGAACTGAAGGGCTCCAACGGCAACGTGACCGAGTCTGGCTGTAAAGAGTGCGAGGAACTGGAAGAGAAGAACATCGTCGAGTTTCTGAACCGGTGGATCACCTTCTGCCAGTCCATCATCTCCACACTGACCGGCGGCGGAGGAAGCGGTGGCGGCGGTAGCGGAGGTGGTGGTTCTGGTGGTGGCGGTTCTATTACATGCCCTCCTCCAATGTCCGTGGAACACGCCGACATCTGGGTCAAGTCCTACAGCCTGTACTCCAGAGAGCGGTACATCTGCAACTCCGGCTTCAAGAGAAAGGCCGGCACCTCTAGCCTGACCGAGTGCGTGCTGAACAAGGCCACCAATGTGGCCCACTGGACAACCCCTAGCCTGAAGTGTATTAGAGGTGGCGGAGGATCTGGCGGAGGCGGATCTGGATCCGCCGAGTCTAAGTACGGCCCTCCTTGTCCTCCATGTCCTGCTCCAGAAGCTGCCGGAGGGCCCAGTGTGTTTCTGTTCCCTCCAAAGCCTAAGGACCAGCTGATGATCTCTCGGACCCCTGAAGTGACCTGCGTGGTGGTGGATGTGTCCCAAGAGGACCCTGAGGTGCAGTTCAATTGGTACGTGGACGGCGTGGAAGTGCACAACGCCAAGACCAAGCCTAGAGAGGAACAGTTCAACTCCACCTACAGAGTGGTGTCCGTGCTGACCGTGCTGCACCAGGATTGGCTGAACGGCAAAGAGTACAAGTGCAAGGTGTCCAACAAGGGCCTGCCTTCCAGCATCGAAAAGACCATCTCCAAGGCCAAGGGCCAGCCTAGGGAACCCCAGGTTTACACCCTGCCTCCAAGCCAAGAGGAAATGACCAAGAACCAGGTGTCCCTGACCTGCCTGGTCAAGGGCTTCTACCCTTCCGATATCGCCGTGGAATGGGAGAGCAATGGCCAGCCTGAGAACAACTACAAGACCACACCTCCTGTGCTGGACTCCGACGGCTCCTTCTTTCTGTACTCCCGCCTGACCGTGGACAAGTCCAGATGGCAAGAGGGCAACGTGTTCTCCTGCTCTGTGCTGCACGAGGCCCTGCACAATCACTACACCCAGAAGTCCCTGTCTCTGTCCCTTGGC | 169 |

(continued)

| Fusion protein (IL-2/IL-15 variant) (variation ) | Nucleic acid sequence | SEQ ID NO: |
|---|---|---|
| **GIC-982N14 (S76N)** | GCTCCTACCTCCTCCAGCACCAAGAAAACCCAGCTGCAGTTGGA GCATCTGCTGCTGGACCTGCAGATGATCCTGAACGGCTCCATGC ACATCGACGCTACCCTGTACACCGAGTCCGACGTGCACCCTTCC TGTAAAGTGACCGCCATGAAGTGCTTTCTGTTGGAGCTGCAAGT GATCTCCCTGGAATCCAAGAACTTCCACCTGAGGCCTCGGGACC TGATCAACAACATCAACGTGATCGTGCTGGAACTGAAGGGCTCC AACGGCAACGTGACCGAGTCTGGCTGTAAAGAGTGCGAGGAACT GGAAGAGAAGAACATCGTCGAGTTTCTGAACCGGTGGATCACCT TCTGCCAGTCCATCATCTCCACACTGACCGGCGGCGGAGGAAGC GGTGGCGGCGGTAGCGGAGGTGGTGGTTCTGGTGGTGGCGGTTC TATTACATGCCCTCCTCCAATGTCCGTGGAACACGCCGACATCT GGGTCAAGTCCTACAGCCTGTACTCCAGAGAGCGGTACATCTGC AACTCCGGCTTCAAGAGAAAGGCCGGCACCTCTAGCCTGACCGA GTGCGTGCTGAACAAGGCCACCAATGTGGCCCACTGGACAACCC CTAGCCTGAAGTGTATTAGAGGTGGCGGAGGATCTGGCGGAGGC GGATCTGGATCCGCCGAGTCTAAGTACGGCCCTCCTTGTCCTCC ATGTCCTGCTCCAGAAGCTGCCGGAGGGCCCAGTGTGTTTCTGT TCCCTCCAAAGCCTAAGGACCAGCTGATGATCTCTCGGACCCCT GAAGTGACCTGCGTGGTGGTGGATGTGTCCCAAGAGGACCCTGA GGTGCAGTTCAATTGGTACGTGGACGGCGTGGAAGTGCACAACG CCAAGACCAAGCCTAGAGAGGAACAGTTCAACTCCACCTACAGA GTGGTGTCCGTGCTGACCGTGCTGCACCAGGATTGGCTGAACGG CAAAGAGTACAAGTGCAAGGTGTCCAACAAGGGCCTGCCTTCCA GCATCGAAAAGACCATCTCCAAGGCCAAGGGCCAGCCTAGGGAA CCCCAGGTTTACACCCTGCCTCCAAGCCAAGAGGAAATGACCAA GAACCAGGTGTCCCTGACCTGCCTGGTCAAGGGCTTCTACCCTT CCGATATCGCCGTGGAATGGGAGAGCAATGGCCAGCCTGAGAAC AACTACAAGACCACACCTCCTGTGCTGGACTCCGACGGCTCCTT CTTTCTGTACTCCCGCCTGACCGTGGACAAGTCCAGATGGCAAG AGGGCAACGTGTTCTCCTGCTCTGTGCTGCACGAGGCCCTGCAC AATCACTACACCCAGAAGTCCCTGTCTCTGTCCCTTGGC | 170 |

(continued)

| Fusion protein (IL-2/IL-15 variant) (variation ) | Nucleic acid sequence | SEQ ID NO: |
|---|---|---|
| GIC-982N15 (S76K) | GCTCCTACCTCCTCCAGCACCAAGAAAACCCAGCTGCAGTTGGA GCATCTGCTGCTGGACCTGCAGATGATCCTGAACGGCTCCATGC ACATCGACGCTACCCTGTACACCGAGTCCGACGTGCACCCTTCC TGTAAAGTGACCGCCATGAAGTGCTTTCTGTTGGAGCTGCAAGT GATCTCCCTGGAATCCAAGAACTTCCACCTGAGGCCTCGGGACC TGATCAAGAACATCAACGTGATCGTGCTGGAACTGAAGGGCTCC AACGGCAACGTGACCGAGTCTGGCTGTAAAGAGTGCGAGGAACT GGAAGAGAAGAACATCGTCGAGTTTCTGAACCGGTGGATCACCT TCTGCCAGTCCATCATCTCCACACTGACCGGCGGCGGAGGAAGC GGTGGCGGCGGTAGCGGAGGTGGTGGTTCTGGTGGTGGCGGTTC TATTACATGCCCTCCTCCAATGTCCGTGGAACACGCCGACATCT GGGTCAAGTCCTACAGCCTGTACTCCAGAGAGCGGTACATCTGC AACTCCGGCTTCAAGAGAAAGGCCGGCACCTCTAGCCTGACCGA GTGCGTGCTGAACAAGGCCACCAATGTGGCCCACTGGACAACCC CTAGCCTGAAGTGTATTAGAGGTGGCGGAGGATCTGGCGGAGGC GGATCTGGATCCGCCGAGTCTAAGTACGGCCCTCCTTGTCCTCC ATGTCCTGCTCCAGAAGCTGCCGGAGGGCCCAGTGTGTTTCTGT TCCCTCCAAAGCCTAAGGACCAGCTGATGATCTCTCGGACCCCT GAAGTGACCTGCGTGGTGGTGGATGTGTCCCAAGAGGACCCTGA GGTGCAGTTCAATTGGTACGTGGACGGCGTGGAAGTGCACAACG CCAAGACCAAGCCTAGAGAGGAACAGTTCAACTCCACCTACAGA GTGGTGTCCGTGCTGACCGTGCTGCACCAGGATTGGCTGAACGG CAAAGAGTACAAGTGCAAGGTGTCCAACAAGGGCCTGCCTTCCA GCATCGAAAAGACCATCTCCAAGGCCAAGGGCCAGCCTAGGGAA CCCCAGGTTTACACCCTGCCTCCAAGCCAAGAGGAAATGACCAA GAACCAGGTGTCCCTGACCTGCCTGGTCAAGGGCTTCTACCCTT CCGATATCGCCGTGGAATGGGAGAGCAATGGCCAGCCTGAGAAC AACTACAAGACCACACCTCCTGTGCTGGACTCCGACGGCTCCTT CTTTCTGTACTCCCGCCTGACCGTGGACAAGTCCAGATGGCAAG AGGGCAACGTGTTCTCCTGCTCTGTGCTGCACGAGGCCCTGCAC AATCACTACACCCAGAAGTCCCTGTCTCTGTCCCTTGGC | 171 |
| GIC-982N16 (S76L) | GCTCCTACCTCCTCCAGCACCAAGAAAACCCAGCTGCAGTTGGA GCATCTGCTGCTGGACCTGCAGATGATCCTGAACGGCTCCATGC ACATCGACGCTACCCTGTACACCGAGTCCGACGTGCACCCTTCC | 172 |

(continued)

| Fusion protein (IL-2/IL-15 variant) (variation ) | Nucleic acid sequence | SEQ ID NO: |
|---|---|---|
| | TGTAAAGTGACCGCCATGAAGTGCTTTCTGTTGGAGCTGCAAGT GATCTCCCTGGAATCCAAGAACTTCCACCTGAGGCCTCGGGACC TGATCCTCAACATCAACGTGATCGTGCTGGAACTGAAGGGCTCC AACGGCAACGTGACCGAGTCTGGCTGTAAAGAGTGCGAGGAACT GGAAGAGAAGAACATCGTCGAGTTTCTGAACCGGTGGATCACCT TCTGCCAGTCCATCATCTCCACACTGACCGGCGGCGGAGGAAGC GGTGGCGGCGGTAGCGGAGGTGGTGGTTCTGGTGGTGGCGGTTC TATTACATGCCCTCCTCCAATGTCCGTGGAACACGCCGACATCT GGGTCAAGTCCTACAGCCTGTACTCCAGAGAGCGGTACATCTGC AACTCCGGCTTCAAGAGAAAGGCCGGCACCTCTAGCCTGACCGA GTGCGTGCTGAACAAGGCCACCAATGTGGCCCACTGGACAACCC CTAGCCTGAAGTGTATTAGAGGTGGCGGAGGATCTGGCGGAGGC GGATCTGGATCCGCCGAGTCTAAGTACGGCCCTCCTTGTCCTCC ATGTCCTGCTCCAGAAGCTGCCGGAGGGCCCAGTGTGTTTCTGT TCCCTCCAAAGCCTAAGGACCAGCTGATGATCTCTCGGACCCCT GAAGTGACCTGCGTGGTGGTGGATGTGTCCCAAGAGGACCCTGA GGTGCAGTTCAATTGGTACGTGGACGGCGTGGAAGTGCACAACG CCAAGACCAAGCCTAGAGAGGAACAGTTCAACTCCACCTACAGA GTGGTGTCCGTGCTGACCGTGCTGCACCAGGATTGGCTGAACGG CAAAGAGTACAAGTGCAAGGTGTCCAACAAGGGCCTGCCTTCCA GCATCGAAAAGACCATCTCCAAGGCCAAGGGCCAGCCTAGGGAA CCCCAGGTTTACACCCTGCCTCCAAGCCAAGAGGAAATGACCAA GAACCAGGTGTCCCTGACCTGCCTGGTCAAGGGCTTCTACCCTT CCGATATCGCCGTGGAATGGGAGAGCAATGGCCAGCCTGAGAAC AACTACAAGACCACACCTCCTGTGCTGGACTCCGACGGCTCCTT CTTTCTGTACTCCCGCCTGACCGTGGACAAGTCCAGATGGCAAG AGGGCAACGTGTTCTCCTGCTCTGTGCTGCACGAGGCCCTGCAC AATCACTACACCCAGAAGTCCCTGTCTCTGTCCCTTGGC | |

(continued)

| Fusion protein (IL-2/IL-15 variant) (variation ) | Nucleic acid sequence | SEQ ID NO: |
|---|---|---|
| GIC-982N17 (L74F, S76D) | GCTCCTACCTCCTCCAGCACCAAGAAAACCCAGCTGCAGTTGGA GCATCTGCTGCTGGACCTGCAGATGATCCTGAACGGCTCCATGC ACATCGACGCTACCCTGTACACCGAGTCCGACGTGCACCCTTCC TGTAAAGTGACCGCCATGAAGTGCTTTCTGCTCGAGCTGCAAGT GATCTCCCTGGAATCCAAGAACTTCCACCTGAGGCCTCGGGACT TCATCGACAACATCAACGTGATCGTGCTGGAACTGAAGGGCTCC AACGGCAACGTGACCGAGTCTGGCTGTAAAGAGTGCGAGGAACT GGAAGAGAAGAACATCGTCGAGTTTCTGAACCGGTGGATCACCT TCTGCCAGTCCATCATCTCCACACTGACCGGCGGCGGAGGAAGC GGTGGCGGCGGTAGCGGAGGTGGTGGTTCTGGTGGTGGCGGTTC TATTACATGCCCTCCTCCAATGTCCGTGGAACACGCCGACATCT GGGTCAAGTCCTACAGCCTGTACTCCAGAGAGCGGTACATCTGC AACTCCGGCTTCAAGAGAAAGGCCGGCACCTCTAGCCTGACCGA GTGCGTGCTGAACAAGGCCACCAATGTGGCCCACTGGACAACCC CTAGCCTGAAGTGTATTAGAGGTGGCGGAGGATCTGGCGGAGGC GGATCTGGATCCGCCGAGTCTAAGTACGGCCCTCCTTGTCCTCC ATGTCCTGCTCCAGAAGCTGCCGGAGGGCCCAGTGTGTTTCTGT TCCCTCCAAAGCCTAAGGACCAGCTGATGATCTCTCGGACCCCT GAAGTGACCTGCGTGGTGGTGGATGTGTCCCAAGAGGACCCTGA GGTGCAGTTCAATTGGTACGTGGACGGCGTGGAAGTGCACAACG CCAAGACCAAGCCTAGAGAGGAACAGTTCAACTCCACCTACAGA GTGGTGTCCGTGCTGACCGTGCTGCACCAGGATTGGCTGAACGG CAAAGAGTACAAGTGCAAGGTGTCCAACAAGGGCCTGCCTTCCA GCATCGAAAAGACCATCTCCAAGGCCAAGGGCCAGCCTAGGGAA CCCCAGGTTTACACCCTGCCTCCAAGCCAAGAGGAAATGACCAA GAACCAGGTGTCCCTGACCTGCCTGGTCAAGGGCTTCTACCCTT CCGATATCGCCGTGGAATGGGAGAGCAATGGCCAGCCTGAGAAC AACTACAAGACCACACCTCCTGTGCTGGACTCCGACGGCTCCTT CTTTCTGTACTCCCGCCTGACCGTGGACAAGTCCAGATGGCAAG AGGGCAACGTGTTCTCCTGCTCTGTGCTGCACGAGGCCCTGCAC AATCACTACACCCAGAAGTCCCTGTCTCTGTCCCTTGGC | 173 |

(continued)

| Fusion protein (IL-2/IL-15 variant) (variation ) | Nucleic acid sequence | SEQ ID NO: |
|---|---|---|
| GIC-982N18 (S76D, N77D) | GCTCCTACCTCCTCCAGCACCAAGAAAACCCAGCTGCAGTTGGA GCATCTGCTGCTGGACCTGCAGATGATCCTGAACGGCTCCATGC ACATCGACGCTACCCTGTACACCGAGTCCGACGTGCACCCTTCC TGTAAAGTGACCGCCATGAAGTGCTTTCTGCTCGAGCTGCAAGT GATCTCCCTGGAATCCAAGAACTTCCACCTGAGGCCTCGGGACC TGATCGACGACATCAACGTGATCGTGCTGGAACTGAAGGGCTCC AACGGCAACGTGACCGAGTCTGGCTGTAAAGAGTGCGAGGAACT GGAAGAGAAGAACATCGTCGAGTTTCTGAACCGGTGGATCACCT TCTGCCAGTCCATCATCTCCACACTGACCGGCGGCGGAGGAAGC GGTGGCGGCGGTAGCGGAGGTGGTGGTTCTGGTGGTGGCGGTTC TATTACATGCCCTCCTCCAATGTCCGTGGAACACGCCGACATCT GGGTCAAGTCCTACAGCCTGTACTCCAGAGAGCGGTACATCTGC AACTCCGGCTTCAAGAGAAAGGCCGGCACCTCTAGCCTGACCGA GTGCGTGCTGAACAAGGCCACCAATGTGGCCCACTGGACAACCC CTAGCCTGAAGTGTATTAGAGGTGGCGGAGGATCTGGCGGAGGC GGATCTGGATCCGCCGAGTCTAAGTACGGCCCTCCTTGTCCTCC ATGTCCTGCTCCAGAAGCTGCCGGAGGGCCCAGTGTGTTTCTGT TCCCTCCAAAGCCTAAGGACCAGCTGATGATCTCTCGGACCCCT GAAGTGACCTGCGTGGTGGTGGATGTGTCCCAAGAGGACCCTGA GGTGCAGTTCAATTGGTACGTGGACGGCGTGGAAGTGCACAACG CCAAGACCAAGCCTAGAGAGGAACAGTTCAACTCCACCTACAGA GTGGTGTCCGTGCTGACCGTGCTGCACCAGGATTGGCTGAACGG CAAAGAGTACAAGTGCAAGGTGTCCAACAAGGGCCTGCCTTCCA GCATCGAAAAGACCATCTCCAAGGCCAAGGGCCAGCCTAGGGAA CCCCAGGTTTACACCCTGCCTCCAAGCCAAGAGGAAATGACCAA GAACCAGGTGTCCCTGACCTGCCTGGTCAAGGGCTTCTACCCTT CCGATATCGCCGTGGAATGGGAGAGCAATGGCCAGCCTGAGAAC AACTACAAGACCACACCTCCTGTGCTGGACTCCGACGGCTCCTT CTTTCTGTACTCCCGCCTGACCGTGGACAAGTCCAGATGGCAAG AGGGCAACGTGTTCTCCTGCTCTGTGCTGCACGAGGCCCTGCAC AATCACTACACCCAGAAGTCCCTGTCTCTGTCCCTTGGC | 174 |
| GIC-982N19 (S76D, V80L) | GCTCCTACCTCCTCCAGCACCAAGAAAACCCAGCTGCAGTTGGA GCATCTGCTGCTGGACCTGCAGATGATCCTGAACGGCTCCATGC ACATCGACGCTACCCTGTACACCGAGTCCGACGTGCACCCTTCC TGTAAAGTGACCGCCATGAAGTGCTTTCTGCTCGAGCTGCAAGT GATCTCCCTGGAATCCAAGAACTTCCACCTGAGGCCTCGGGACC TGATCGACAACATCAACCTGATCGTGCTGGAACTGAAGGGCTCC | 175 |

(continued)

| Fusion protein (IL-2/IL-15 variant) (variation ) | Nucleic acid sequence | SEQ ID NO: |
|---|---|---|
| | AACGGCAACGTGACCGAGTCTGGCTGTAAAGAGTGCGAGGAACT GGAAGAGAAGAACATCGTCGAGTTTCTGAACCGGTGGATCACCT TCTGCCAGTCCATCATCTCCACACTGACCGGCGGCGGAGGAAGC GGTGGCGGCGGTAGCGGAGGTGGTGGTTCTGGTGGTGGCGGTTC TATTACATGCCCTCCTCCAATGTCCGTGGAACACGCCGACATCT GGGTCAAGTCCTACAGCCTGTACTCCAGAGAGCGGTACATCTGC AACTCCGGCTTCAAGAGAAAGGCCGGCACCTCTAGCCTGACCGA GTGCGTGCTGAACAAGGCCACCAATGTGGCCCACTGGACAACCC CTAGCCTGAAGTGTATTAGAGGTGGCGGAGGATCTGGCGGAGGC GGATCTGGATCCGCCGAGTCTAAGTACGGCCCTCCTTGTCCTCC ATGTCCTGCTCCAGAAGCTGCCGGAGGGCCCAGTGTGTTTCTGT TCCCTCCAAAGCCTAAGGACCAGCTGATGATCTCTCGGACCCCT GAAGTGACCTGCGTGGTGGTGGATGTGTCCCAAGAGGACCCTGA GGTGCAGTTCAATTGGTACGTGGACGGCGTGGAAGTGCACAACG CCAAGACCAAGCCTAGAGAGGAACAGTTCAACTCCACCTACAGA GTGGTGTCCGTGCTGACCGTGCTGCACCAGGATTGGCTGAACGG CAAAGAGTACAAGTGCAAGGTGTCCAACAAGGGCCTGCCTTCCA GCATCGAAAAGACCATCTCCAAGGCCAAGGGCCAGCCTAGGGAA CCCCAGGTTTACACCCTGCCTCCAAGCCAAGAGGAAATGACCAA GAACCAGGTGTCCCTGACCTGCCTGGTCAAGGGCTTCTACCCTT CCGATATCGCCGTGGAATGGGAGAGCAATGGCCAGCCTGAGAAC AACTACAAGACCACACCTCCTGTGCTGGACTCCGACGGCTCCTT CTTTCTGTACTCCCGCCTGACCGTGGACAAGTCCAGATGGCAAG AGGGCAACGTGTTCTCCTGCTCTGTGCTGCACGAGGCCCTGCAC AATCACTACACCCAGAAGTCCCTGTCTCTGTCCCTTGGC | |

(continued)

| Fusion protein (IL-2/IL-15 variant) (variation ) | Nucleic acid sequence | SEQ ID NO: |
|---|---|---|
| GIC-982N20 (L74F, S76D, N77D) | GCTCCTACCTCCTCCAGCACCAAGAAAACCCAGCTGCAGTTGGA GCATCTGCTGCTGGACCTGCAGATGATCCTGAACGGCTCCATGC ACATCGACGCTACCCTGTACACCGAGTCCGACGTGCACCCTTCC TGTAAAGTGACCGCCATGAAGTGCTTTCTGCTCGAGCTGCAAGT GATCTCCCTGGAATCCAAGAACTTCCACCTGAGGCCTCGGGACT TCATCGACGACATCAACGTGATCGTGCTGGAACTGAAGGGCTCC AACGGCAACGTGACCGAGTCTGGCTGTAAAGAGTGCGAGGAACT GGAAGAGAAGAACATCGTCGAGTTTCTGAACCGGTGGATCACCT TCTGCCAGTCCATCATCTCCACACTGACCGGCGGCGGAGGAAGC GGTGGCGGCGGTAGCGGAGGTGGTGGTTCTGGTGGTGGCGGTTC TATTACATGCCCTCCTCCAATGTCCGTGGAACACGCCGACATCT GGGTCAAGTCCTACAGCCTGTACTCCAGAGAGCGGTACATCTGC AACTCCGGCTTCAAGAGAAAGGCCGGCACCTCTAGCCTGACCGA GTGCGTGCTGAACAAGGCCACCAATGTGGCCCACTGGACAACCC CTAGCCTGAAGTGTATTAGAGGTGGCGGAGGATCTGGCGGAGGC GGATCTGGATCCGCCGAGTCTAAGTACGGCCCTCCTTGTCCTCC ATGTCCTGCTCCAGAAGCTGCCGGAGGGCCCAGTGTGTTTCTGT TCCCTCCAAAGCCTAAGGACCAGCTGATGATCTCTCGGACCCCT GAAGTGACCTGCGTGGTGGTGGATGTGTCCCAAGAGGACCCTGA GGTGCAGTTCAATTGGTACGTGGACGGCGTGGAAGTGCACAACG CCAAGACCAAGCCTAGAGAGGAACAGTTCAACTCCACCTACAGA GTGGTGTCCGTGCTGACCGTGCTGCACCAGGATTGGCTGAACGG CAAAGAGTACAAGTGCAAGGTGTCCAACAAGGGCCTGCCTTCCA GCATCGAAAAGACCATCTCCAAGGCCAAGGGCCAGCCTAGGGAA CCCCAGGTTTACACCCTGCCTCCAAGCCAAGAGGAAATGACCAA GAACCAGGTGTCCCTGACCTGCCTGGTCAAGGGCTTCTACCCTT CCGATATCGCCGTGGAATGGGAGAGCAATGGCCAGCCTGAGAAC AACTACAAGACCACACCTCCTGTGCTGGACTCCGACGGCTCCTT CTTTCTGTACTCCCGCCTGACCGTGGACAAGTCCAGATGGCAAG AGGGCAACGTGTTCTCCTGCTCTGTGCTGCACGAGGCCCTGCAC AATCACTACACCCAGAAGTCCCTGTCTCTGTCCCTTGGC | 176 |

(continued)

| Fusion protein (IL-2/IL-15 variant) (variation ) | Nucleic acid sequence | SEQ ID NO: |
|---|---|---|
| GIC-982N21 (S76D, N77D, V80L) | GCTCCTACCTCCTCCAGCACCAAGAAAACCCAGCTGCAGTTGGA GCATCTGCTGCTGGACCTGCAGATGATCCTGAACGGCTCCATGC ACATCGACGCTACCCTGTACACCGAGTCCGACGTGCACCCTTCC TGTAAAGTGACCGCCATGAAGTGCTTTCTGCTCGAGCTGCAAGT GATCTCCCTGGAATCCAAGAACTTCCACCTGAGGCCTCGGGACC TGATCGACGACATCAACCTGATCGTGCTGGAACTGAAGGGCTCC AACGGCAACGTGACCGAGTCTGGCTGTAAAGAGTGCGAGGAACT GGAAGAGAAGAACATCGTCGAGTTTCTGAACCGGTGGATCACCT TCTGCCAGTCCATCATCTCCACACTGACCGGCGGCGGAGGAAGC GGTGGCGGCGGTAGCGGAGGTGGTGGTTCTGGTGGTGGCGGTTC TATTACATGCCCTCCTCCAATGTCCGTGGAACACGCCGACATCT GGGTCAAGTCCTACAGCCTGTACTCCAGAGAGCGGTACATCTGC AACTCCGGCTTCAAGAGAAAGGCCGGCACCTCTAGCCTGACCGA GTGCGTGCTGAACAAGGCCACCAATGTGGCCCACTGGACAACCC CTAGCCTGAAGTGTATTAGAGGTGGCGGAGGATCTGGCGGAGGC GGATCTGGATCCGCCGAGTCTAAGTACGGCCCTCCTTGTCCTCC ATGTCCTGCTCCAGAAGCTGCCGGAGGGCCCAGTGTGTTTCTGT TCCCTCCAAAGCCTAAGGACCAGCTGATGATCTCTCGGACCCCT GAAGTGACCTGCGTGGTGGTGGATGTGTCCCAAGAGGACCCTGA GGTGCAGTTCAATTGGTACGTGGACGGCGTGGAAGTGCACAACG CCAAGACCAAGCCTAGAGAGGAACAGTTCAACTCCACCTACAGA GTGGTGTCCGTGCTGACCGTGCTGCACCAGGATTGGCTGAACGG CAAAGAGTACAAGTGCAAGGTGTCCAACAAGGGCCTGCCTTCCA GCATCGAAAAGACCATCTCCAAGGCCAAGGGCCAGCCTAGGGAA CCCCAGGTTTACACCCTGCCTCCAAGCCAAGAGGAAATGACCAA GAACCAGGTGTCCCTGACCTGCCTGGTCAAGGGCTTCTACCCTT CCGATATCGCCGTGGAATGGGAGAGCAATGGCCAGCCTGAGAAC AACTACAAGACCACACCTCCTGTGCTGGACTCCGACGGCTCCTT CTTTCTGTACTCCCGCCTGACCGTGGACAAGTCCAGATGGCAAG AGGGCAACGTGTTCTCCTGCTCTGTGCTGCACGAGGCCCTGCAC AATCACTACACCCAGAAGTCCCTGTCTCTGTCCCTTGGC | 177 |
| GIC-982N22 (L74F, S76D, V80L) | GCTCCTACCTCCTCCAGCACCAAGAAAACCCAGCTGCAGTTGGA GCATCTGCTGCTGGACCTGCAGATGATCCTGAACGGCTCCATGC ACATCGACGCTACCCTGTACACCGAGTCCGACGTGCACCCTTCC TGTAAAGTGACCGCCATGAAGTGCTTTCTGCTCGAGCTGCAAGT GATCTCCCTGGAATCCAAGAACTTCCACCTGAGGCCTCGGGACT TCATCGACAACATCAACCTGATCGTGCTGGAACTGAAGGGCTCC AACGGCAACGTGACCGAGTCTGGCTGTAAAGAGTGCGAGGAACT GGAAGAGAAGAACATCGTCGAGTTTCTGAACCGGTGGATCACCT TCTGCCAGTCCATCATCTCCACACTGACCGGCGGCGGAGGAAGC | 178 |

(continued)

| Fusion protein (IL-2/IL-15 variant) (variation ) | Nucleic acid sequence | SEQ ID NO: |
|---|---|---|
|  | GGTGGCGGCGGTAGCGGAGGTGGTGGTTCTGGTGGTGGCGGTTC TATTACATGCCCTCCTCCAATGTCCGTGGAACACGCCGACATCT GGGTCAAGTCCTACAGCCTGTACTCCAGAGAGCGGTACATCTGC AACTCCGGCTTCAAGAGAAAGGCCGGCACCTCTAGCCTGACCGA GTGCGTGCTGAACAAGGCCACCAATGTGGCCCACTGGACAACCC CTAGCCTGAAGTGTATTAGAGGTGGCGGAGGATCTGGCGGAGGC GGATCTGGATCCGCCGAGTCTAAGTACGGCCCTCCTTGTCCTCC ATGTCCTGCTCCAGAAGCTGCCGGAGGGCCCAGTGTGTTTCTGT TCCCTCCAAAGCCTAAGGACCAGCTGATGATCTCTCGGACCCCT GAAGTGACCTGCGTGGTGGTGGATGTGTCCCAAGAGGACCCTGA GGTGCAGTTCAATTGGTACGTGGACGGCGTGGAAGTGCACAACG CCAAGACCAAGCCTAGAGAGGAACAGTTCAACTCCACCTACAGA GTGGTGTCCGTGCTGACCGTGCTGCACCAGGATTGGCTGAACGG CAAAGAGTACAAGTGCAAGGTGTCCAACAAGGGCCTGCCTTCCA GCATCGAAAAGACCATCTCCAAGGCCAAGGGCCAGCCTAGGGAA CCCCAGGTTTACACCCTGCCTCCAAGCCAAGAGGAAATGACCAA GAACCAGGTGTCCCTGACCTGCCTGGTCAAGGGCTTCTACCCTT CCGATATCGCCGTGGAATGGGAGAGCAATGGCCAGCCTGAGAAC AACTACAAGACCACACCTCCTGTGCTGGACTCCGACGGCTCCTT CTTTCTGTACTCCCGCCTGACCGTGGACAAGTCCAGATGGCAAG AGGGCAACGTGTTCTCCTGCTCTGTGCTGCACGAGGCCCTGCAC AATCACTACACCCAGAAGTCCCTGTCTCTGTCCCTTGGC |  |

(continued)

| Fusion protein (IL-2/IL-15 variant) (variation ) | Nucleic acid sequence | SEQ ID NO: |
|---|---|---|
| Fc-IL2 | GCCGAGTCTAAGTACGGACCTCCTTGTCCTCCATGTCCTGCTCC<br>AGAAGCTGCTGGCGGCCCTTCCGTGTTTCTGTTCCCTCCAAAGC<br>CTAAGGACACCCTGATGATCTCTCGGACCCCTGAAGTGACCTGC<br>GTGGTGGTGGATGTGTCCCAAGAGGATCCCGAGGTGCAGTTCAA<br>TTGGTACGTGGACGGCGTGGAAGTGCACAACGCCAAGACCAAGC<br>CTAGAGAGGAACAGTTCAACTCCACCTACAGAGTGGTGTCCGTG<br>CTGACCGTGCTGCACCAGGATTGGCTGAACGGCAAAGAGTACAA<br>GTGCAAGGTGTCCAACAAGGGCCTGCCTTCCAGCATCGAAAAGA<br>CCATCTCCAAGGCCAAGGGCCAGCCTAGGGAACCCCAGGTTTAC<br>ACCCTGCCTCCAAGCCAAGAGGAAATGACCAAGAACCAGGTGTC<br>CCTGACCTGCCTGGTCAAGGGCTTCTACCCTTCCGATATCGCCG<br>TGGAATGGGAGAGCAATGGCCAGCCTGAGAACAACTACAAGACC<br>ACACCTCCTGTGCTGGACTCCGACGGCTCCTTCTTTCTGTACTC<br>CCGCCTGACCGTGGACAAGTCCAGATGGCAAGAGGGCAACGTGT<br>TCTCCTGCTCCGTGATGCACGAGGCCCTGCACAATCACTACACC<br>CAGAAGTCCCTGTCTCTGTCCCTTGGAGGTGGCGGAGGATCTGC<br>TCCTACCTCCTCCAGCACCAAGAAACCCAGCTGCAGTTGGAGC<br>ATCTGCTGCTGGACCTGCAGATGATCCTGAATGGCATCAACAAT<br>TACAAGAACCCCAAGCTGACCCGGATGCTGACCTTCAAGTTCTA<br>CATGCCCAAGAAGGCCACCGAGCTGAAACATCTGCAGTGCCTGG<br>AAGAGGAACTGAAGCCCCTGGAAGAAGTGCTGAATCTGGCCCAG<br>TCCAAGAACTTCCACCTGAGGCCTCGGGACCTGATCTCCAACAT<br>CAACGTGATCGTGCTCGAGCTGAAGGGCTCCGAGACAACCTTCA<br>TGTGCGAGTACGCCGACGAGACAGCTACCATCGTGGAATTTCTG<br>AACCGGTGGATCACCTTCTGCCAGTCCATCATCAGCACCCTGAC<br>C | 179 |

GIC-982C1 to GIC-982C15 were purified using an open column containing Protein A resin. The Protein A resin from Repligen was washed and equilibrated using PBS and Protein A binding buffer from Thermo. Then, the supernatant filtered through a 0.22 um filter was reacted with Protein A resin. The Protein A resin reacted in the open column washed with PBS was collected and the column was washed using Protein A binding buffer in an amount corresponding to 10 times the resin volume. Then, binding buffer was added to a collection tube in an amount of 1/10 times the elution buffer and then Thermo's IgG elution buffer was added to elute and collect. The collected fusion protein was replaced with PBS buffer.

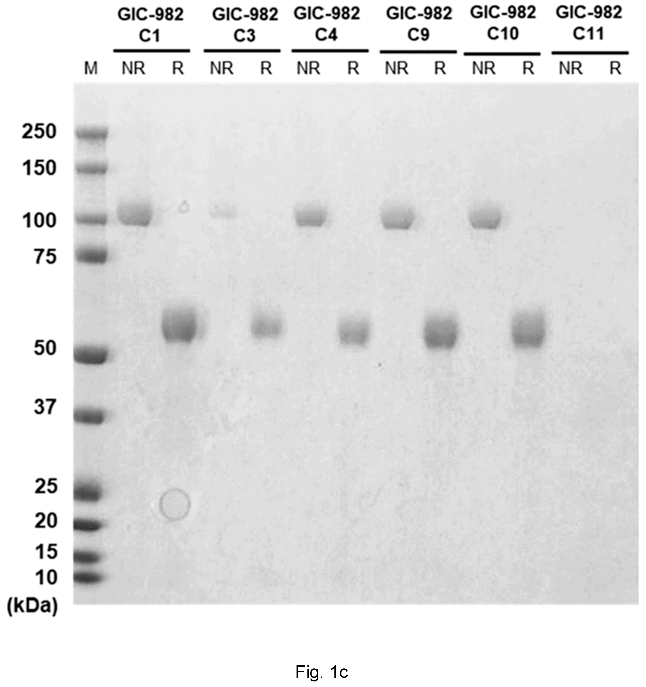

At this time, the separated and purified fusion protein was subjected to SDS-PAGE under reducing (R) or non-reducing (NR) conditions and confirmed by staining with Coomassie blue (FIGS. 1C to 1F).

Example 1-2: Measurement of IL-2Rβ binding affinity using Octet binding assay

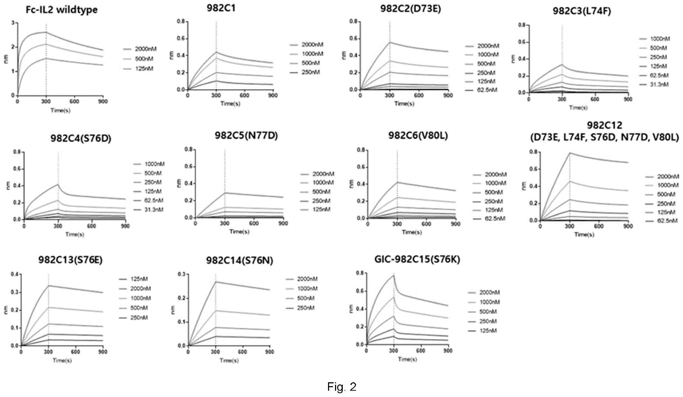

In order to determine the binding affinity of GIC-982C1 to GIC-982C6 and GIC-982C12 to GIC-982C15 to IL-2Rβ, an Octet binding assay was performed using a Ni-NTA sensor chip (ForteBio 18-5101). Fc-IL2 was selected as a control group for analysis. First, the sensor chip was activated through immersion in distilled water for 5 minutes. The sensor was baselined in PBS for 3 minutes, and then immersed in a solution containing 6 μg/ml of IL-2Rβ-his (Acro CD2-H5221) to bind to the sensor. Then, the baseline was maintained in PBS for 3 minutes and then immersed in a test substance serially diluted by 1/2 from 2,000 nM to 31.25 nM for 5 minutes to associate with the IL-2Rβ. At this time, PBS buffer not containing the test substance was used as a blank value for analysis. Then, the result was transferred back to PBS and dissociated for 10 minutes. All steps were performed at room temperature and the plate was shaken at 1,000 RPM. The experimental results derived through ForteBio data analysis software are shown in Table 8 and FIG. 2 below.

The binding affinity was confirmed through Octet. The result showed that GIC-982C1 to GIC-982C6 and GIC-982C12 to GIC-982C15 showed relatively higher dissociation constants (Kd) than Fc-IL2, which means that the binding affinity to IL-2Rβ was weakened.

[Table 8]

| Item | $K_d$ (M) | $k_{on}$ (1/Ms) | $k_{dis}$ (1/s) | Full $R^2$ |
|---|---|---|---|---|
| Fc-IL2 | 1.79E-08 | 4.10E+04 | 7.33E-04 | 0.9939 |
| GIC-982C1 | 3.87E-07 | 8.66E+02 | 3.35E-04 | 0.9994 |
| GIC-982C2 | 2.15E-07 | 6.86E+02 | 1.48E-04 | 0.9996 |
| GIC-982C3 | 3.017E-07 | 1.594E+03 | 4.810E-04 | 0.9993 |
| GIC-982C4 | 1.435E-07 | 2.353E+03 | 3.377E-04 | 0.9989 |
| GIC-982C5 | 1.29E-06 | 2.42E+02 | 3.132E-04 | 0.9970 |
| GIC-982C6 | 3.86E-07 | 1.13E+03 | 4.35E-04 | 0.9917 |
| GIC-982C12 | 2.65E-07 | 3.44E+02 | 9.11E-05 | 0.9999 |
| GIC-982C13 | 1.05E-07 | 1.93E+03 | 2.04E-04 | 0.9969 |
| GIC-982C14 | 3.27E-07 | 6.86E+02 | 2.23E-04 | 0.9905 |
| GIC-982C15 | 1.78E-07 | 2.67E+03 | 4.76E-04 | 0.9972 |

Example 1-3: Confirmation of the effect of IL-2 receptor (IL-2R)-mediated STATS mechanism activity using STATS signaling assay

To evaluate the STAT5 activity of GIC-982C1 to GIC-982C6 and GIC-982C12 to GIC-982C15, HEK-Blue CD122/132 cells (InvivoGen hkb-il2bg) expressing IL-2Rβ and common γ chain were cultured and STAT5 activity was analyzed. IL2-Fc was selected as a control group for analysis.

The HEK-Blue CD122/132 cells were cultured using DMEM [(Gibco, Cat No.10569010) + 10% FBS (Gibco, Cat No.26140079) + penicillin/streptomycin (Gibco, Cat No.15140122) + Normocin (InvivoGen, Ant-Nr-2) + 1X HEK- Blue™ Selection (InvivoGen, Hb-Sel) + 1 μg/mL Puromycin (InvivoGen, Ant-Pr-1)], and activity test was performed using DMEM [(Gibco, Cat No.10569010) + 10% FBS (Gibco, Cat No.26140079) + penicillin/streptomycin (Gibco, Cat No.15140122)] containing no antibiotic for screening. The cultured cells were treated with Trypsin-EDTA, collected and then washed with added medium. The collected HEK-Blue CD122/132 cells were centrifuged at 300 x g for 5 minutes, the supernatant was removed, and the residue was resuspended in fresh medium. Then, the HEK-Blue CD122/132 cells were counted and diluted with medium such that the number of cells was adjusted to $2.5 \times 10^5$ cells/mL. The protein of the control group was diluted to the highest concentration to be used in the test (100 ng/mL based on IL-2), loaded onto the plate, and serially diluted by 1/10. 20 mL of the diluted protein was loaded onto the flat-bottom plate and 180 mL (45,000 cells) of the previously prepared cells were added. The plate was cultured for 24 hours at 37°C in the presence of $CO_2$ 5%.

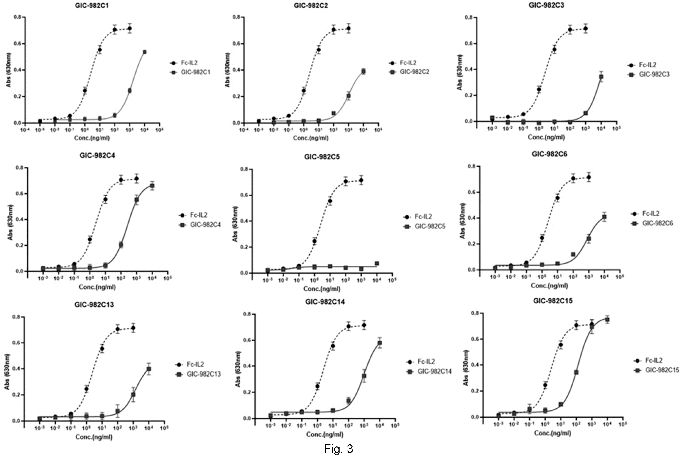

To prepare QUANTI-Blue™ solution (InvivoGen, rep-qbs) for detection, 750 mL of QB reagent and QB buffer were added to 73.5 mL of distilled water, vortexed to mix well, and incubated at room temperature for 10 minutes. 20 mL of each supernatant from the plate cultured for 24 hours was loaded onto a fresh plate, 180 mL of the previously prepared QUANTI-Blue™ solution was added thereto and incubated in the absence of light at 37°C for 1 hour. The absorbance measured at 630 nm using a microplate reader is shown in FIG. 3 and $EC_{50}$ derived therefrom is shown in Table 9.

As a result, GIC-982C1 to GIC-982C5 and GIC-982C13 to GIC-982C15 were found to have lower STAT5 activity intensity than Fc-IL2 and to be substances that can maintain an appropriate level of cell activity while preventing cell hyperactivity.

[Table 9]

| Item | Name | $EC_{50}$ (ng/ml) |
|---|---|---|
| Control group | Fc-IL2 | 2.423 |

(continued)

| Item | Name | EC$_{50}$ (ng/ml) |
|---|---|---|
| | GIC-982C1 | 1726 |
| | GIC-982C2 | 1080 |
| | GIC-982C3 | 9741 |
| | GIC-982C4 | 255.3 |
| Test group | GIC-982C5 | - |
| | GIC-982C6 | 722.9 |
| | GIC-982C13 | 1392 |
| | GIC-982C14 | 997 |
| | GIC-982C15 | 133.6 |

Example 1-4: Determination of anticancer efficacy and reduction of side effects of IL-2/IL-15 fusion protein in *in vivo* mouse anticancer model

To determine the anticancer efficacy of IL-2/IL-15 chimeric protein and reduction of side effects of pulmonary edema due to immune overactivation, a mouse model was established by subcutaneously administering 5x10$^5$ cells/head of CT26, a colon cancer cell line, to Balb/c mice. The volume of the generated tumor was measured using Vernier calipers, and group separation was performed when the volume was approximately 70 mm$^3$. Then, IL2-Fc, GIC-982C1, GIC-982C4, and GIC-982C15 were administered intraperitoneally to the mice twice a week for 2 weeks for a total of 4 times at a dose of 10 mg/kg. The tumor volume was measured on days 1, 4, 7, 11, 14, 16, and 18 after administration to determine the anticancer efficacy, and the results are shown in Table 10.

In addition, the condition of the mice was observed to determine whether or not side effects occurred due to administration. Whether or not adverse reactions of dead subjects occurred was determined with the naked eye and the dead subjects were autopsied to check the condition of the internal organs. To determine the occurrence of side effects due to drug administration, the extracted lung was weighed, dried for 2 days, and then weighted again to calculate the wet/dry ratio. The results are shown in Table 11.

As a result, Fc-IL2 brought about lung death due to pulmonary edema caused by immune hyperactivity under the condition of a high dose of 10 mpk, while GIC-982C1, GIC-982C4, and GIC-982C15 had excellent cancer cell proliferation inhibition activity. In addition, Fc-IL2 had an increase in wet lung weight, while GIC-982C1, GIC-982C4, and GIC-982C15 had the same wet/dry ratio as a vehicle, which indicates that no pulmonary edema occurred.

[Table 10]

| Administration group (dose, mg/kg) | | Tumor volume (mm$^3$) | | | | | | |
|---|---|---|---|---|---|---|---|---|
| | | Time after administration (days) | | | | | | |
| | | 1 day | 4 days | 7 days | 11 days | 14 days | 16 days | 18 days |
| Vehicle (0 mpk) | Mean | 70 | 184 | 262 | 622 | 970 | 1378 | 1802 |
| | S.E. | 5 | 16 | 38 | 146 | 227 | 319 | 464 |
| | No. of subjects | 8 | 8 | 8 | 8 | 8 | 8 | 8 |
| Fc-IL2 (10 mpk) | Mean | 70 | 83 | death | | | | |
| | S.E | 5 | 5 | | | | | |
| | No. of subjects | 8 | 8 | | | | | |
| GIC-982C1 (10 mpk) | Mean | 70 | 134 | 168 | 319 | 552 | 778 | 1162 |
| | S.E | 6 | 11 | 15 | 53 | 96 | 139 | 229 |
| | No. of subjects | 7 | 7 | 7 | 7 | 7 | 7 | 7 |

(continued)

| Administration group (dose, mg/kg) | | Tumor volume (mm$^3$) | | | | | | |
|---|---|---|---|---|---|---|---|---|
| | | Time after administration (days) | | | | | | |
| | | 1 day | 4 days | 7 days | 11 days | 14 days | 16 days | 18 days |
| GIC-982C4 (10 mpk) | Mean | 70 | 132 | 175 | 281 | 416 | 602 | 844 |
| | S.E | 5 | 29 | 49 | 136 | 209 | 349 | 506 |
| | No. of subjects | 4 | 4 | 4 | 4 | 4 | 4 | 4 |
| GIC-982C15 (10 mpk) | Mean | 71 | 181 | 199 | 345 | 489 | 729 | 1057 |
| | S.E | 3 | 66 | 111 | 203 | 302 | 446 | 575 |
| | No. of subjects | 3 | 3 | 3 | 3 | 3 | 3 | 3 |

[Table 11]

| Administration group (dose, mg/kg) | | Wet lung weight | Dry lung weight | Ratio |
|---|---|---|---|---|
| Vehicle (0 mpk) | Mean | 0.141 | 0.031 | 4.5 |
| | S.E | 0.006 | 0.001 | 0.2 |
| | No. of subjects | 8 | 8 | 8 |
| Fc-IL2 (10 mpk) | Mean | 0.413 | 0.058 | 7.1 |
| | S.E | 0.047 | 0.004 | 0.8 |
| | No. of subjects | 8 | 8 | 8 |
| GIC-982C1 (10 mpk) | Mean | 0.133 | 0.030 | 4.4 |
| | S.E | 0.004 | 0.001 | 0.1 |
| | No. of subjects | 7 | 7 | 7 |
| GIC-982C4 (10 mpk) | Mean | 0.133 | 0.029 | 4.6 |
| | S.E | 0.005 | 0.001 | 0.2 |
| | No. of subjects | 4 | 4 | 4 |
| GIC-982C15 (10 mpk) | Mean | 0.151 | 0.033 | 4.6 |
| | S.E | 0.006 | 0.002 | 0.2 |
| | No. of subjects | 3 | 3 | 3 |

**Example 2. Production of immune cells modified to express IL-2/IL-15 fusion protein and confirmation of effect**

Example 2-1: Plasmid construction

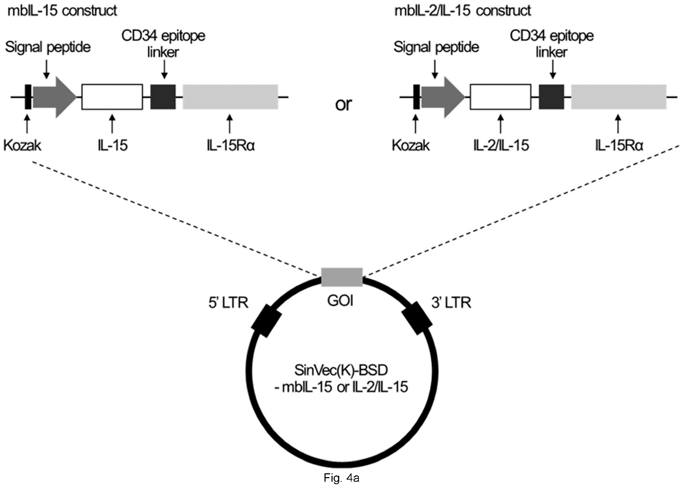

Two clone vectors containing amino acid sequences of SEQ ID NOS: 183 and 185 and nucleic acid sequences of SEQ ID NOS: 184 and 186 shown in Table 12 below were produced using a retro-virus vector (Biovec pharma, SinVec(K)-GFP-BSD, Vec-033) and the schematic diagram is shown in FIG. 4A.

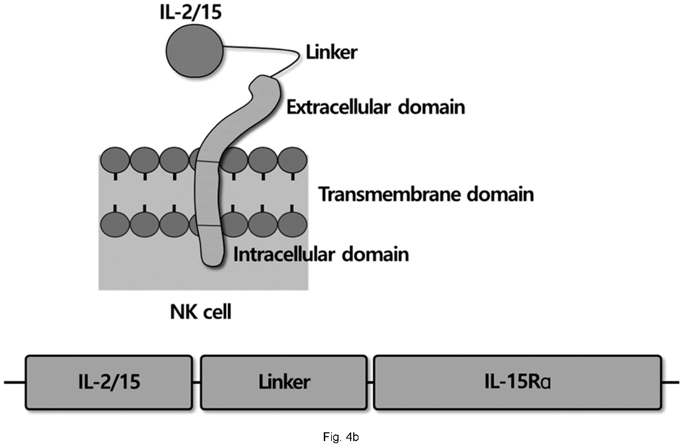

The schematic diagram of the immune cells expressing the IL-2/IL-15 fusion protein of the vector is shown in FIG. 4B and the expressed fusion protein is as follows:

N'-[IL-2/IL-15 chimeric protein]-[linker]-[IL15Rα]-C'

[Table 12]

| Component | Amino acid or nucleic acid sequence | SEQ ID NO: |
|---|---|---|
| Signal Peptide | MDWTWILFLVAAATRVHS | 180 |
| | ATGGATTGGACATGGATACTCTTTCTTGTTGCCGCAGCCAC GCGAGTCCATTCC | 181 |
| Linker (CD34 Hinge) | ELPTQGTFSNVSTNVS | 87 |
| | GAGTTACCTACCCAGGGAACATTTTCAAATGTTTCTACAAA TGTATCC | 182 |
| IL-15Rα Full-length | ITCPPPMSVEHADIWVKSYSLYSRERYICNSGFKRKAGTSS LTECVLNKATNVAHWTTPSLKCIRDPALVHQRPAPPSTVTT AGVTPQPESLSPSGKEPAASSPSSNNTAATTAAIVPGSQLM PSKSPSTGTTEISSHESSHGTPSQTTAKNWELTASASHQPP GVYPQGHSDTTVAISTSTVLLCGLSAVSLLACYLKSRQTPP LASVEMEAMEALPVTWGTSSRDEDLENCSHHL | 77 |
| | ATTACTTGTCCACCACCAATGTCTGTTGAGCATGCTGATAT TTGGGTGAAATCTTATTCTCTCTATAGCCGGGAACGCTATA TCTGCAATTCAGGATTTAAAAGAAAGGCTGGTACCTCTTCC TTGACCGAATGTGTACTGAATAAAGCTACAAACGTGGCACA TTGGACCACGCCTAGCCTTAAGTGTATAAGGGACCCCGCAT TAGTGCATCAGAGACCTGCACCCCCGAGCACCGTGACAACT GCCGGTGTCACTCCTCAACCTGAATCTCTTTCTCCCAGCGG TAAGGAACCAGCTGCCAGTAGTCCGTCAAGTAATAATACCG CAGCTACTACTGCCGCCATCGTTCCCGGGTCTCAACTCATG CCAAGCAAGAGCCCCTCAACCGGGACAACCGAAATTTCCTC TCACGAAAGTTCTCATGGGACTCCAAGCCAAACCACCGCTA AGAATTGGGAGTTGACCGCCTCAGCAAGTCATCAACCCCCA GGGGTATACCCCCAAGGGCATTCCGATACTACCGTTGCAAT TAGCACTTCTACGGTGTTACTTTGCGGATTATCTGCAGTAT CCCTGTTGGCCTGTTATCTTAAAAGCCGTCAGACACCGCCA CTCGCTTCCGTCGAGATGGAAGCTATGGAAGCCCTTCCCGT AACCTGGGGCACTAGTTCCCGCGACGAGGATCTGGAGAATT GTAGTCATCATTTGTAA | 78 |

(continued)

| Component | Amino acid or nucleic acid sequence | SEQ ID NO: |
|---|---|---|
| **mbIL-15wt+ IL-15Rα** | GIHVFILGCFSAGLPKTEANWVNVISDLKKIEDLIQSMHID ATLYTESDVHPSCKVTAMKCFLLELQVISLESGDASIHDTV ENLIILANNSLSSNGNVTESGCKECEELEEKNIKEFLQSFV HIVQMFINTSELPTQGTFSNVSTNVSITCPPPMSVEHADIW VKSYSLYSRERYICNSGFKRKAGTSSLTECVLNKATNVAHW TTPSLKCIRDPALVHQRPAPPSTVTTAGVTPQPESLSPSGK EPAASSPSSNNTAATTAAIVPGSQLMPSKSPSTGTTEISSH ESSHGTPSQTTAKNWELTASASHQPPGVYPQGHSDTTVAIS TSTVLLCGLSAVSLLACYLKSRQTPPLASVEMEAMEALPVT WGTSSRDEDLENCSHHL | 183 |
| | GGCATCCACGTGTTCATTCTGGGATGCTTCAGCGCAGGCCT GCCTAAAACCGAGGCGAACTGGGTTAACGTGATCAGCGATT TGAAAAAGATTGAGGATCTGATCCAGTCCATGCATATTGAC GCAACCTTGTACACAGAGTCAGATGTGCACCCTTCCTGCAA AGTTACGGCTATGAAGTGTTTTTTGCTTGAATTGCAAGTAA TAAGTTTGGAGTCTGGCGATGCATCTATACATGATACCGTA GAAAATTTGATCATCCTGGCTAATAATAGTTTGTCCAGTAA TGGTAACGTAACAGAGAGCGGTTGTAAAGAGTGTGAAGAGC TCGAGGAAAAGAATATAAAAGAATTCTTGCAATCCTTTGTA CATATCGTGCAAATGTTCATTAATACAAGCGAGTTACCTAC CCAGGGAACATTTTCAAATGTTTCTACAAATGTATCCATTA CTTGTCCACCACCAATGTCTGTTGAGCATGCTGATATTTGG GTGAAATCTTATTCTCTCTATAGCCGGGAACGCTATATCTG CAATTCAGGATTTAAAAGAAAGGCTGGTACCTCTTCCTTGA CCGAATGTGTACTGAATAAAGCTACAAACGTGGCACATTGG ACCACGCCTAGCCTTAAGTGTATAAGGGACCCCGCATTAGT GCATCAGAGACCTGCACCCCCGAGCACCGTGACAACTGCCG GTGTCACTCCTCAACCTGAATCTCTTTCTCCCAGCGGTAAG GAACCAGCTGCCAGTAGTCCGTCAAGTAATAATACCGCAGC TACTACTGCCGCCATCGTTCCCGGGTCTCAACTCATGCCAA GCAAGAGCCCCTCAACCGGGACAACCGAAATTTCCTCTCAC GAAAGTTCTCATGGGACTCCAAGCCAAACCACCGCTAAGAA TTGGGAGTTGACCGCCTCAGCAAGTCATCAACCCCCAGGGG TATACCCCCAAGGGCATTCCGATACTACCGTTGCAATTAGC ACTTCTACGGTGTTACTTTGCGGATTATCTGCAGTATCCCT GTTGGCCTGTTATCTTAAAAGCCGTCAGACACCGCCACTCG CTTCCGTCGAGATGGAAGCTATGGAAGCCCTTCCCGTAACC TGGGGCACTAGTTCCCGCGACGAGGATCTGGAGAATTGTAG TCATCATTTGTAA | 184 |

(continued)

| Component | Amino acid or nucleic acid sequence | SEQ ID NO: |
|---|---|---|
| mbl-L-2/IL-15v3(-S76D)+IL -15Rα | APTSSSTKKTQLQLEHLLLDLQMILNGSMHIDATLYTESDV HPSCKVTAMKCFLLELQVISLESKNFHLRPRDLIDNINVIV LELKGSNGNVTESGCKECEELEEKNIVEFLNRWITFCQSII STLTELPTQGTFSNVSTNVSITCPPPMSVEHADIWVKSYSL YSRERYICNSGFKRKAGTSSLTECVLNKATNVAHWTTPSLK CIRDPALVHQRPAPPSTVTTAGVTPQPESLSPSGKEPAASS PSSNNTAATTAAIVPGSQLMPSKSPSTGTTEISSHESSHGT PSQTTAKNWELTASASHQPPGVYPQGHSDTTVAISTSTVLL CGLSAVSLLACYLKSRQTPPLASVEMEAMEALPVTWGTSSR DEDLENCSHHL | 185 |
|  | ATGGATTGGACATGGATACTCTTTCTTGTTGCCGCAGCCAC GCGAGTCCATTCCGCTCCCACAAGTTCAAGTACTAAAAAAA CCCAACTGCAACTGGAGCATTTGCTTTTGGATCTCCAAATG ATTCTGAATGGCTCAATGCACATTGACGCTACTCTTTACAC AGAGAGTGATGTACACCCTTCTTGCAAGGTTACCGCTATGA AATGCTTTCTCTTGGAACTCCAAGTAATCAGTCTTGAATCT AAAAATTTTCATTTGCGCCCACGCGATCTGATAGACAACAT CAACGTGATAGTACTGGAACTCAAGGGAAGCAATGGAAACG TCACAGAATCAGGTTGTAAAGAATGTGAGGAATTGGAAGAA AAGAATATAGTCGAGTTCCTCAATCGCTGGATCACTTTTTG CCAATCCATTATATCCACTCTGACAGAGTTACCTACCCAGG GAACATTTTCAAATGTTTCTACAAATGTATCCATTACTTGT CCACCACCAATGTCTGTTGAGCATGCTGATATTTGGGTGAA ATCTTATTCTCTCTATAGCCGGGAACGCTATATCTGCAATT CAGGATTTAAAAGAAAGGCTGGTACCTCTTCCTTGACCGAA TGTGTACTGAATAAAGCTACAAACGTGGCACATTGGACCAC GCCTAGCCTTAAGTGTATAAGGGACCCCGCATTAGTGCATC AGAGACCTGCACCCCCGAGCACCGTGACAACTGCCGGTGTC ACTCCTCAACCTGAATCTCTTTCTCCCAGCGGTAAGGAACC AGCTGCCAGTAGTCCGTCAAGTAATAATACCGCAGCTACTA CTGCCGCCATCGTTCCCGGGTCTCAACTCATGCCAAGCAAG AGCCCCTCAACCGGGACAACCGAAATTTCCTCTCACGAAAG TTCTCATGGGACTCCAAGCCAAACCACCGCTAAGAATTGGG AGTTGACCGCCTCAGCAAGTCATCAACCCCCAGGGGTATAC CCCCAAGGGCATTCCGATACTACCGTTGCAATTAGCACTTC TACGGTGTTACTTTGCGGATTATCTGCAGTATCCCTGTTGG CCTGTTATCTTAAAGCCGTCAGACACCGCCACTCGCTTCC GTCGAGATGGAAGCTATGGAAGCCCTTCCCGTAACCTGGGG CACTAGTTCCCGCGACGAGGATCTGGAGAATTGTAGTCATC ATTTGTAA | 186 |

Example 2-2: Retrovirus production and concentration

To produce BaEV-pseudotyped retrovirus, 6.0 x 10$^6$ 293Vec-BaEV cell line (Biovec pharma, Vec-006) was cultured in a T75 flask at 37°C in a $CO_2$ 5% incubator for 24 hours the day before transfection. When the cell density reached 80%, 15 μg of the chimeric protein-encoding plasmid was transfected using lipofectamine 2000 (Invitrogen, 11668500). 48 hours after transfection, the culture medium containing the retrovirus was harvested, centrifuged at 500 x g and 4°C for 10 minutes, and passed through a 0.45 $\mu$m pore PVDF membrane filter (MILLIPORE, SE1M003M00) to remove cell debris. The culture medium containing retrovirus from which cell debris had been removed was mixed with Retro-X concentrator (Clontech, 631456) reagent in a ratio of 3:1 (virus culture medium: Retro-X concentrator reagent) for retrovirus concentration and reacted under 4°C refrigerated conditions. After 18 hours of reaction, the retrovirus pellet obtained by centrifugation for 45 minutes at 1,500 $\times$ g and 4°C was resuspended in Opti-MEM medium (Gibco, 11058021) and concentrated. The concentrated retrovirus was stored under -80°C conditions.

Example 2-3: Preparation of natural killer cells derived from human peripheral blood mononuclear cells (PBMCs)

Blood collected from different healthy donors was centrifuged at 400 x g for 30 minutes using a Ficoll-Hypaque concentration gradient and PBMCs were separated from the buffy coat. The separated PBMCs were counted using an ADAM-MC2 automatic cell counter (NanoEnTek), transferred to a fresh tube to obtain CD3(-) cells, and then centrifuged at 350 x g at 4°C for 10 minutes. After centrifugation, the supernatant was removed and 80 mL of CliniMACS buffer (20% human serum albumin, EDTA 2 mM) and 22 mL of CD3 magnetic beads (Miltenyi biotech, 130-050-101) were dispensed per 1 x $10^7$ cells to suspend the cell pellet, and incubated in the absence of light at 4°C for 15 minutes. After the reaction, 10 mL of CliniMACS buffer was dispensed for washing, centrifuged at 350 x g and at 4°C for 10 minutes, and then 0.5 mL of CliniMACS buffer was dispensed per 1 x $10^8$ cells to suspend the cell pellet.

3 mL of CliniMACS buffer was flowed into an LD column (Miltenyi Biotec, 130-042-901) and soaked, and the cell suspension was flowed into the column to obtain CD3(-) cells that passed through the column. 10 mL of CliniMACS buffer was added for washing and the cell pellet was centrifuged at 350 x g and 4°C for 10 minutes. The cell pellet was resuspended in CliniMACS buffer and the number of cells was measured using an automatic cell counter. Then, 80 mL of CliniMACS buffer and 22 mL of CD56 magnetic beads (Miltenyi biotech, 130-050-401) were dispensed per 1 x $10^7$ cells, the cell pellet was suspended, and the reaction was performed in the absence of light at 4°C for 15 minutes. For washing, 10 mL of CliniMACS buffer was dispensed and centrifuged at 350 x g and 4°C for 10 minutes, and 0.5 mL of CliniMACS buffer was dispensed per 1x$10^8$ cells to suspend the cell pellet.

3 mL of CliniMACS buffer was flowed onto an LS column (Miltenyi Biotec, 130-042-401) and soaked and the cell suspension was flowed. Then, the column was separated from the magnet stand, 5 mL of CliniMACS buffer was added, pressure is applied thereto with a piston, and CD3(-) CD56(+) natural killer cells were obtained in a fresh tube. The obtained cells were centrifuged at 350 x g and 4°C for 10 minutes. After centrifugation, the cell pellet was resuspended in CliniMACS buffer and the number of cells was measured using an automatic cell counter. The cells were suspended at 10 x $10^6$/mL to 20 x $10^6$/mL in CryoStor CS10 cryopreservative (Biolife solution, 210102). The suspended cells were dispensed into cryogenic vials (1 mL each), primarily frozen once using a cell freezing container (Corning, CLS432001), and then transferred to an LN2 tank for secondary freezing.

Example 2-4: Production of natural killer cells expressing chimeric proteins including IL-2 and IL-15

For the culture of the isolated natural killer cells of Example 2-3, 100 mL of CD335 (NKp46)-biotin and 100 mL of CD2-biotin included in the NK cell activation/expansion kit (Miltenyi Biotec, 130-094-483) were dispensed into a fresh tube, mixed, and mixed with 500 mL of anti-biotin MACSIBead. 300 mL of CliniMACS buffer was added to the same tube, and reacted for 2 hours at 4.0 rpm and 4°C using a microtube rotator (Thermo scientific, Hulamixer, 15920D). The frozen natural killer cells of Example 2-3 were thawed and the number of cells was measured. The previously prepared beads were washed with 1 mL of CliniMACS buffer, suspended in CN5-101 medium (CTS™ NK-Xpander™ Medium, 1X supplement (Gibco, A5019001), 5% human AB serum (Milan Analytica AG, #000084), and 50 nM GI-101 (GI-inovation, 8.2 mg/mL)) based on 5 mL of beads/1 x $10^6$ cells, seeded in 6-well plates, and cultured at 37°C in the presence of 5% $CO_2$ for 4 days.

To infect the retrovirus produced in Example 2-2, untreated 12-well plate was coated with 10 $\mu$g/mL of retronectin (Takara, T100B) according to the protocol provided by the manufacturer, 300 mL of retrovirus corresponding to 1 to 10 MOI was added to each well, and centrifuged at 2,000 x g and at 32°C for 2 hours, and the retrovirus was adsorbed onto the retronectin-coated plate. The supernatant was removed and then the plate was washed once with 1 mL of DPBS (WELGENE, LB001-02). On the 4th day from the start of culture, the number of natural killer cells was measured and suspended in CN5-101 medium at a cell density of 0.8 x $10^6$ cells/mL. 1 mL of the result was seeded onto each well plate coated with the prepared natural killer cells and 0.6 mL of CN5-101 medium was further seeded thereon. The result was centrifuged at 1,000 x g and 32°C for 15 minutes, and retrovirus and natural killer cells were linked to each other, cultured at 37°C in the presence of 5% $CO_2$ for 3 days, seeded at a cell density of 0.5 x $10^6$ cells/mL and then subcultured.

Specifically, on the third day of culture after transduction, the expression of the chimeric protein was detected using a flow cytometer, the same number of cells was suspended and cultured in CN5-101 medium containing GI-101 and CN5 medium not containing GI-101 (CTS™ NK-Xpander™ Medium, 1X supplement (Gibco, A5019001), 5% human AB serum (Milan Analytica AG, #000084)) at a cell density of 0.5 x $10^6$ cells/mL. Then, the number of cells was measured separately under the CN5-101 medium and CN5 medium conditions, and 5 mL of cells were sub-cultured at a cell density of 0.5 x $10^6$ cells/mL.

Example 2-5: Analysis of expression of chimeric proteins containing IL-2 and IL-15

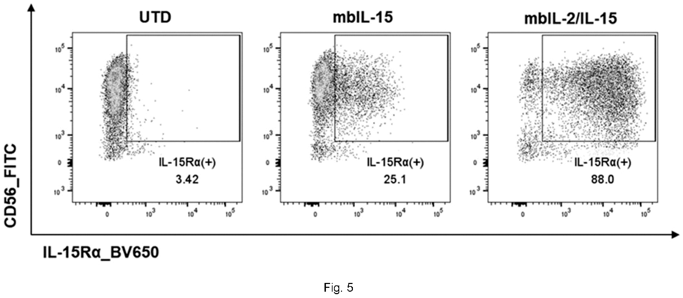

Retrovirus transduction was induced, natural killer cells were obtained on the third day and 0.2 x $10^6$ cells were seeded on a 96-well plate using an automatic cell counter. 250 µl of FACS buffer (DPBS containing 2% FBS) was added, followed by centrifugation at 350 x g for 5 minutes. After centrifugation, the supernatant was removed and the cell pellet was suspended in an FITC-labeled anti-CD56 antibody (Biolegend, 362546), a BV650-labeled anti-IL-15Rα antibody (BD, 747701), and a LIVE/DEAD Fixable violet dye sample (Invitrogen, L34955A), and reacted at 4°C for 30 minutes. Then, 150 µl of FACS buffer was added and centrifuged at 350 x g and 4°C for 5 minutes. The supernatant was removed, the cell pellet was resuspended in 200 to 250 µl of FACS buffer and seeded onto a FACS tube, and the expression of IL-15Rα constituting the chimeric protein was measured using a flow cytometer (FACSymphonyTM A3 Cell analyzer, BD).
As a result, as can be seen from FIG. 5, natural killer cells expressing the membrane-bound chimeric protein (mbIL-2/IL-15) containing IL-2 and IL-15 on the cell membrane surface showed a higher expression efficiency at 88% than the membrane-bound IL-15 protein (mbIL-15) at 25.1%.

Example 2-6: Comparison of *in-vitro* cell counting and cell viability

The cultured natural killer cells were cultured at the same cell density and in the same number for 19 days in CN5 medium and for 25 days in CN5-101 medium supplemented with GI-101 on the 3rd day after transduction. On days 0, 4, 7, 10, 13, 16, 19, 22, and 25, the cells attached to the bottom of the flask were removed with a scraper (SPL, 90020), pipetted and disaggregated to obtain samples necessary for cell counting. The cell density (x $10^6$ cells/mL), cell viability (%), and dead cell count of the obtained cells were measured using an automatic cell counter. The total cell count on days 0 and 4 of culture was calculated by multiplying the cell density of the live cells and the total volume (mL) of the cell culture medium, and then 5 mL of natural killer cells was suspended at a cell density of 0.5 x $10^6$ cells/mL and subcultured. Thus, the total cell count from day 7 was calculated as follows:

Predicted total cell count = live cell density (x $10^6$ cells/mL)/ inoculated cell density of the previous culture day (0.5 x $10^6$ cells/mL) x total cell count of the previous culture day (x $10^6$ cells)

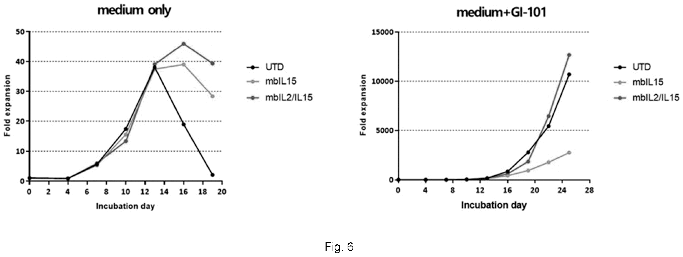

The cell fold expansion was calculated using the calculated total cell count and the predicted total cell count. The cell fold expansion was calculated as a multiple by dividing the total cell number on each culture day or the predicted total cell number based on the total cell number on day 0 of culture, and cell proliferation was stimulated in the presence of GI-101 (a cell proliferation-promoting protein) or culture was performed in the absent of GI-101, and the results are shown in FIG. 6.
As a result, as can be seen from FIG. 6, natural killer cells (mbIL-2/IL-15) expressing a membrane-bound chimeric protein containing IL-2 and IL-15 had superior proliferation and survival abilities in the presence or absence of GI-101 (a cell proliferation-promoting protein) stimulation compared to general natural killer cells (UTD) that did not undergo transformation and natural killer cells expressing membrane-bound IL-15 protein (mbIL-15).

**Example 3. Confirmation of anticancer efficacy and improvement of side effects of IL-2/IL-15 fusion proteins and natural killer cells expressing the same compared to control group**

Example 3-1: Confirmation of *in vitro* cell killing efficacy using various cancer cell lines

Target tumor cell lines (SKOV3, HCT116, A549) for *in vitro* cell killing activity evaluation were infected with lentivirus (Satorious, 4475) containing GFP and puromycin resistance gene, and cultured in R10 medium (10% fetal bovine serum, 1% penicillin-streptomycin) containing 0.5 µg/mL of puromycin, and only the transduced tumor cells were selected and cultured. The target tumor cell lines were suspended in R10 medium at a density of 3 x $10^4$ cells/mL (A549 and HCT116 cell lines) or 5 x $10^4$ cells/mL (SKOV3 cell line) one day before the *in vitro* cell killing activity evaluation, and 100 µl of the target tumor cell lines were seeded on each well of a 96-well plate (Corning, 3799) and cultured for 16 hours at 37°C in the presence of 5% $CO_2$.
The mbIL-2/IL-15 expressing natural killer cells produced in Example 2-4 were thawed, washed, and seeded onto a 96-well plate to which the target tumor cells prepared the previous day were attached at an E/T (effector-to-target) ratio of 10:1 or 3:1. The plates were imaged for 96 hours at 4-hour intervals for each well using an Incucyte®S3 device (Sartorius) installed inside a cell culture incubator. Then, the positive target tumor cells showing GFP fluorescence were counted and analyzed in the stored images.

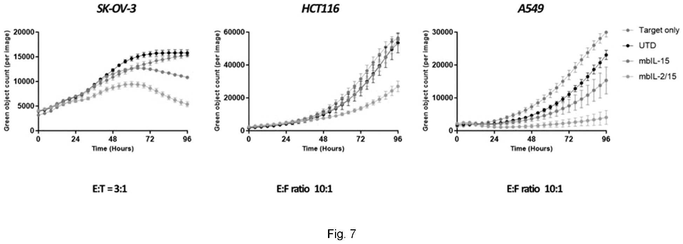

As a result, as can be seen from FIG. 7, natural killer cells expressing mbIL-2/IL-15 had high cell killing ability in three tumor cells compared to UTD and mbIL-15.

Industrial Applicability

The natural killer cells modified to express the chimeric protein including IL-2 and IL-15 proteins, variants thereof, or the fusion protein containing the same according to the present invention suppress side effects caused by excessive immune cell activity of IL-2, and prevent rapid exhaustion of natural killer cells, thereby maintaining an appropriate level of cell activity and cell survival ability for a long time. Therefore, based on the chimeric protein including IL-2 and IL-15 proteins with excellent innate immune function, the present invention provides a protein complex containing the chimeric protein and a fusion protein containing the chimeric protein, and natural killer cells modified to express the same.

Although specific configurations of the present invention have been described in detail, those skilled in the art will appreciate that this detailed description is provided as preferred embodiments for illustrative purposes, and should not be construed as limiting the scope of the present invention. Therefore, the substantial scope of the present invention is defined by the accompanying filed claims and equivalents thereto.

**Claims**

1. A chimeric protein or variant thereof by substituting an A-B loop domain, a helix B domain and a C-D loop domain of IL-2 with an A-B loop domain, a helix B domain and a C-D loop domain of IL-15, respectively.

2. The chimeric protein or variant thereof according to claim 1, wherein the chimeric protein or variant thereof comprises an amino acid sequence of SEQ ID NO: 33.

3. The chimeric protein or variant thereof according to claim 1, wherein the chimeric protein or the variant thereof comprises a substitution at least one amino acid position selected from the group consisting of amino acids at positions 73, 74, 76, 77, and 80 in the amino acid sequence of SEQ ID NO: 33.

4. The chimeric protein or variant thereof according to claim 1, wherein the variant comprises at least one amino acid substitution selected from the group consisting of D73E, L74F, S76D, S76E, S76N, S76K, S76L, N77D and V80L in the amino acid sequence of SEQ ID NO: 33.

5. The chimeric protein or variant thereof according to claim 1, wherein the variant comprises at least one amino acid substitution selected from the group consisting of D73E, L74F, S76D, N77D and V80L in the amino acid sequence of SEQ ID NO: 33.

6. The chimeric protein or variant thereof according to claim 1, wherein the variant comprises an amino acid sequence of SEQ ID NO: 35, 37, 39, 41, 43, 45, 47, 49, 51, 53, 55, 57, 59, 61, 63, 65, 67, 69, 71, 73 or 75.

7. A fusion protein comprising the chimeric protein or the variant thereof according to claim 1.

8. The fusion protein according to claim 7, further comprising an IL-15 receptor alpha protein or a fragment thereof.

9. The fusion protein according to claim 8, wherein the fragment of the IL-15 receptor alpha protein comprises a sushi domain of the IL-15 receptor alpha protein.

10. The fusion protein according to claim 7, further comprising an Fc domain.

11. A nucleic acid encoding the chimeric protein or the variant thereof according to claim 1, or a fusion protein comprising the same.

12. A vector comprising the nucleic acid according to claim 11.

13. A host cell introduced with the nucleic acid according to claim 11 or the vector according to claim 12.

14. A genetically modified cell expressing the chimeric protein or the variant thereof according to claim 1 or the fusion protein comprising the same.

15. The genetically modified cell according to claim 14, wherein the genetically modified cell is a natural killer cell.

16. The genetically modified cell according to claim 14, wherein the fusion protein comprises a membrane protein.

17. The genetically modified cell according to claim 16, wherein the membrane protein is selected from the group consisting of IL-15 receptor alpha (IL-15Rα), CD8α, CD4, CD3ε, CD3γ, CD3δ, CD3ζ, CD28, CD137, FcεRIγ, T-cell receptors, nicotinic acetylcholine receptors, GABA receptors, and fragments thereof.

18. The genetically modified cell according to claim 14, wherein the fusion protein has a structure of the following Formula (I) or Formula (II):

$$\text{N'-X-[L1]}_n\text{-Y-C'} \qquad \text{(Formula (I))}$$

$$\text{N'-Y-[L1]}_n\text{-X-C'} \qquad \text{(Formula (II))}$$

wherein X is a chimeric protein including IL-2 and IL-15 proteins or a variant thereof;
Y is an IL-15 receptor alpha (IL-15Rα) protein or a fragment thereof;
L1 is a linker; and
$n$ is an integer of 0 or greater.

19. A pharmaceutical composition for preventing or treating cancer, an infectious disease or an autoimmune disease comprising at least one of:

the chimeric protein or variant thereof according to claim 1;
the fusion protein according to claim 7; and
the genetically modified cell according to claim 14.

20. A method of producing the chimeric protein or variant thereof according to claim 1, or a fusion protein comprising the same,
the method comprising culturing the host cell according to claim 13 to produce a chimeric protein or a variant thereof, or a fusion protein comprising the same.

Fig. 1a

Fig. 1b

Fig. 1c

Fig. 1d

Fig. 1e

Fig. 1f

Fig. 2

Fig. 3

Fig. 4a

Fig. 4b

Fig. 5

Fig. 6

Fig. 7

## INTERNATIONAL SEARCH REPORT

| International application No. |
| --- |
| **PCT/KR2024/096098** |

| A. | CLASSIFICATION OF SUBJECT MATTER |
| --- | --- |

**C12N 5/0783**(2010.01)i; **C07K 14/55**(2006.01)i; **C07K 14/54**(2006.01)i; **C07K 14/715**(2006.01)i; **A61K 35/17**(2015.01)i; **A61K 39/00**(2006.01)i; **A61P 35/00**(2006.01)i

According to International Patent Classification (IPC) or to both national classification and IPC

| B. | FIELDS SEARCHED |
| --- | --- |

Minimum documentation searched (classification system followed by classification symbols)

C12N 5/0783(2010.01); A61K 35/17(2015.01); C07K 14/54(2006.01); C07K 14/715(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 인터루킨-2(interleukin-2), 인터루킨-15(interleukin-15), 재조합 단백질 (recombinant proteins), 변이체(mutant), 융합 단백질(fusion protein), 스시 도메인(sushi domain), 서열(sequence), 핵산 (nucleic acid), 벡터(vector), 숙주세포(host cell), 면역세포(immunocyte)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | KR 10-2022-0029477 A (GI CELL, INC.) 08 March 2022 (2022-03-08)<br>See abstract; and claims 1, 13-17, 20, 21 and 23. | 1-20 |
| A | JAKOBISIAK, M. et al. Interleukin 15 as a promising candidate for tumor immunotherapy. Cytokine & Growth Factor Reviews. 2011, vol. 22, pp. 99-108.<br>See pages 99-101. | 1-20 |
| A | MORTIER, E. et al. Soluble Interleukin-15 Receptor $\alpha$ (IL-15R$\alpha$)-sushi as a Selective and Potent Agonist of IL-15 Action through IL-15R$\beta$/$\gamma$: HYPERAGONIST IL-15·IL-15R$\alpha$ FUSION PROTEINS. THE JOURNAL OF BIOLOGICAL CHEMISTRY. 2006, vol. 281, no. 3, pp. 1612-1619.<br>See pages 1612-1613 and 1616. | 1-20 |
| A | CHIRIFU, M. et al. Crystal structure of the IL-15–IL-15R$\alpha$ complex, a cytokine-receptor unit presented in trans. NATURE IMMUNOLOGY. 2007, vol. 8, no. 9, pp. 1001-1007.<br>See abstract; and pages 1001 and 1003-1005. | 1-20 |

| ✓ Further documents are listed in the continuation of Box C. | ✓ See patent family annex. |
| --- | --- |

| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| --- | --- |

| Date of the actual completion of the international search | Date of mailing of the international search report |
| --- | --- |
| **06 December 2024** | **06 December 2024** |

| Name and mailing address of the ISA/KR | Authorized officer |
| --- | --- |
| **Korean Intellectual Property Office<br>Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| | International application No. |
|---|---|
| | **PCT/KR2024/096098** |

| C. | DOCUMENTS CONSIDERED TO BE RELEVANT | |
|---|---|---|
| Category* | Citation of document, with indication, where appropriate, of the relevant passages | Relevant to claim No. |
| A | US 10358477 B2 (JACQUES, Y. et al.) 23 July 2019 (2019-07-23)<br>See abstract; and claims 1-10. | 1-20 |
| A | WO 2021-262880 A2 (KADMON CORPORATION, LLC) 30 December 2021 (2021-12-30)<br>See entire document. | 1-20 |

Form PCT/ISA/210 (second sheet) (July 2022)

**INTERNATIONAL SEARCH REPORT**

International application No.

**PCT/KR2024/096098**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/KR2024/096098**

| Patent document cited in search report | | | Publication date (day/month/year) | Patent family member(s) | | | Publication date (day/month/year) |
|---|---|---|---|---|---|---|---|
| KR | 10-2022-0029477 | A | 08 March 2022 | CN | 116897163 | A | 17 October 2023 |
| | | | | EP | 4206219 | A1 | 05 July 2023 |
| | | | | JP | 2023-540296 | A | 22 September 2023 |
| | | | | US | 2023-0322881 | A1 | 12 October 2023 |
| | | | | WO | 2022-045849 | A1 | 03 March 2022 |
| US | 10358477 | B2 | 23 July 2019 | CN | 101360827 | A | 04 February 2009 |
| | | | | CN | 101360827 | B | 20 March 2013 |
| | | | | EP | 1777294 | A1 | 25 April 2007 |
| | | | | EP | 1934353 | A2 | 25 June 2008 |
| | | | | EP | 1934353 | B1 | 05 October 2011 |
| | | | | JP | 2009-512433 | A | 26 March 2009 |
| | | | | JP | 2013-226155 | A | 07 November 2013 |
| | | | | JP | 5394742 | B2 | 22 January 2014 |
| | | | | JP | 5886247 | B2 | 16 March 2016 |
| | | | | US | 12134639 | B2 | 05 November 2024 |
| | | | | US | 2020-0109184 | A1 | 09 April 2020 |
| | | | | WO | 2007-046006 | A2 | 26 April 2007 |
| | | | | WO | 2007-046006 | A3 | 04 October 2007 |
| WO | 2021-262880 | A2 | 30 December 2021 | CN | 116194471 | A | 30 May 2023 |
| | | | | EP | 4168124 | A2 | 26 April 2023 |
| | | | | JP | 2023-531920 | A | 26 July 2023 |
| | | | | KR | 10-2023-0027267 | A | 27 February 2023 |
| | | | | US | 2023-0416363 | A1 | 28 December 2023 |
| | | | | WO | 2021-262880 | A3 | 17 February 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5229109 A
- EP 307247 A
- WO 9108291 A

**Non-patent literature cited in the description**

- **SMITH**. *Science*, 1988, vol. 240, 1169-76
- **BAZAN**. *Science*, 1992, vol. 257, 410-413
- **MINAMI et al.** *Annu Rev Immunol*, 1993, vol. 11, 245-268
- **KRIEG et al.** *Proc Natl Acad Sci*, 2010, vol. 107, 11906-11
- **MAREK JAKOBISIAK et al.** *Cytokine & Growth Factor Reviews*, 2011, vol. 22, 99-108
- **FONTENOT et al.** *Nature Immunol*, 2005, vol. 6, 1142-51
- **H. ASAO**. *Encyclopedia of Endocrine Disease*, 2004, 60-63
- **WALDMANN**. *Nat Rev Immunol*, 2009, vol. 6, 595-601
- **OLEJNICZAK** ; **KASPRZAK**. *Med Sci Monit*, 2008, vol. 14, RA179-89
- **MALEK**. *Annu Rev Immunol*, 2008, vol. 26, 453-79
- **D'CRUZ** ; **KLEIN**. *Nature Immunol*, 2005, vol. 6, 1152-59
- **BRANDENBURG, S. et al.** *Eur J Immunol*, 2008, vol. 38 (6), 1643-53
- **FACCIABENE, A. et al.** *Cancer Res*, 2012, vol. 72 (9), 2162-71
- **ARENAS-RAMIREZ, N. et al.** *Sci Transl Med*, 2016, vol. 8 (367), 367-166
- **FELICES et al.** *JCI Insight*, 2018
- **FRUTOSO et al.** *Int J Mol Sci*, 2019, vol. 20 (18), 4514
- **MASARU IMAMURA et al.** *Blood*, 2014, vol. 124 (7), 1081-1088
- **FELICES et al.** *JCI Insight*, 2018, vol. 3 (3), e96219
- **CASSELL**. *Current Pharmaceutical Design*, 2002, vol. 8, 2171-2183
- **LOWE**. *Journal of Molecular Biology*, 2011, vol. 406, 160-175
- **HERMANSON, G.** Bioconjugate Techniques. Academic Press, 1996